(19) Europäisches Patentamt
European Patent Office
Office européen des brevets

(11) EP 2 896 698 A1

(12) **EUROPEAN PATENT APPLICATION**

(43) Date of publication:
**22.07.2015 Bulletin 2015/30**

(21) Application number: **14151610.4**

(22) Date of filing: **17.01.2014**

(51) Int Cl.:
***C12N 15/82*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **BASF Plant Science Company GmbH
67056 Ludwigshafen (DE)**
- **Crop Functional Genomics Center
Gwanak-Gu
Seoul 151-921 (KR)**

(72) Inventor: **The designation of the inventor has not yet been filed**

(74) Representative: **Blackwell, Ean David
BASF SE
Global Intellectual Property
Carl-Bosch-Strasse 38
67056 Ludwigshafen (DE)**

(54) **Plants having one or more enhanced yield-related traits and a method for making the same**

(57) The present invention relates generally to the field of molecular biology and concerns a method for enhancing various economically important yield-related traits in plants. More specifically, the present invention concerns a method for enhancing one or more yield-related traits in plants by modulating expression in a plant of a nucleic acid encoding a HvRAF-like (Hordeum vulgare "RAF-like protein family") polypeptide. The present invention also concerns plants having modulated expression of a nucleic acid encoding a HvRAF-like polypeptide, which plants have one or more enhanced yield-related traits relative to control plants. The invention also provides hitherto unknown HvRAF-like-encoding nucleic acids, and constructs comprising the same, useful in performing the methods of the invention.

EP 2 896 698 A1

SEQUENCE LISTING

<110> BASF Plant Science Company GmbH

<120> Plants having one or more enhanced yield-related traits and a method for making the same

<130> PF7664

<160> 36

<170> Biomax PatentTool according to PatentIN 3.1 format

<210> 1
<211> 1103
<212> DNA
<213> Hordeum vulgare

<400> 1
```
atgtgtggcg gcccatccta gcgcagctga tcccgccgtc ggcgggccgt ccgtcgaagc     60
aggcggcagc gggcggccgg gccccgccca cgagctccaa gaaggacggc gtgagcaaga    120
gccgccacag cagcacccca gatgccgacg acgacgtctt cgaggccgcc ttcgaggact    180
tcgatgacca cttcgacctg cgggcggagg aggacggcgg cgacgaccat gtcgtctttg    240
catccaagcc tgccttctct ccacgtccgg gtaggctata actctcatag tttcatctga    300
gtgtgcgatc atgaaacgta caaggttcac ggccgttggt tcttggtacc atggaagagg    360
aagactgatg agattccttt ttgcagccta cgacggtggc cgcgcggcgc atgcggcgag    420
caggaagaag cgcaccggcc acctccatgg catccggcag cggccgtggg gcaagtgggc    480
ggcggagatc cgcgacccgc acaagggcac ccgcgtctgg ctcggcacgt tcgacacggc    540
cgatgatgcc gcccgggcct acgacgtcgc cgcccgtcgc ctccgtggca gcaaggccaa    600
ggtcaacttc cccgacgcgg ccaggaccgg ggctcgcccg cgccgcgcca gccgtagaac    660
cgcgcagaaa ccgcaatgcc cccctgcgcg gacgacggcg tactctgcca ccgcagcagc    720
acgcgcacag ccggagcagg acgctatgat ggtcaaaccc gagctgatgg agtttttcaa    780
cgtggacgcc atcgtccacc tgaccactgc cgtcgccgcg ctaccgcctg tcacggcgag    840
caccttcgcc gacacgatgc cgagggtcga cgaggactct tctgtgggga gcggcggcgg    900
cgccatgctg ggttcgccg acgagcttgg gttcgatccg ttcatgatgt tccagctacc    960
ctgctcggac atgtacgaat ccgccgacag catcttcgcc ggagacgctg tcatcccgga   1020
tgccctcagc gtggacagtg gcatggacgc cgtcagcctc tggagcttcg acgagttccc   1080
catggacagc gccattttct gaa                                            1103
```

<210> 2
<211> 1512
<212> DNA
<213> Hordeum vulgare subsp. vulgare

<400> 2
```
gggaagacgt gagagaaaaa gcgcgagaca gaaagagaaa acaatgtgtg gcggcgccat     60
cctagcgcag ctgatcccgc cgtcggcggg ccgtccgtcg aagcaggcgg cagcgggcgg    120
ccgggcccgc ccacgagctc caagaagggc ggcgtgagca agaggcgcca caccagcact    180
atgccgacga cgacttcgag gccgccttcg aggacttcga tgacgacctc gacggcggcg    240
gcgaccatgt cgtttttgca tccaagcttg cgctctctcc gggtccaggt agggtaactc    300
tcatagtttc atctgagtgt gccatcgtga aacgtacaag gttcacgggc gttggttctt    360
ggtaccatgg aagactgatg agattccctt ttgcagccta cgacggttgc cgcgcggcgc    420
atgcggcgag caggaacaag cgcaccggcc acctccatgg catccggcag cgtccgtggg    480
gcaagtgggg ggcggagatc cgcgacccgc acaagggcac ccgcgtctgg ctcggcacgt    540
tcgacacggc cgatgatgcc gcccgggcct acgacgtcgc cgcccgacgc ctccgcggca    600
gcaaggccaa ggtcaacttc cccgaggcgg ccagggcagg ggctcgcccg cgccgcgcca    660
gccgtagaac cgcgcagaaa ccgcactgcc cccctgcgtg gacgacggcg tactctgcca    720
gcgtagcagc acgcaaacag ccggagcagg acgatatgat ggtcaagact gagctgatgg    780
agtttttcga cgtgggcgcc ttcgtcgacc tgaccactgc cgtcaccgcg ctaccgcctg    840
tcacggcgag aaccttcgcc gacacgatgc cgagggtcga cgaggactct tctgtgggga    900
gcggcggcgg cgccatgctg ggttcgccg acgagcttgg gttcgatccg ttcatgatgt    960
tccagctacc ctgctcggac atgtacgaat ccgccgacag tatcttcgcc ggagacgctg   1020
tcatcccgga tgccctcagc gtggacagtg gcatggacgc cgtcagcctc tggagcttcg   1080
```

acgagttccc catggacagc gccattttct gacgctttcc gtgtgatgca ctgcactctg 1140
ttggttgtaa gaatctccac ctggcctcta cgtagttcct tgtaaatgcc cgcgcacaga 1200
accttgctca gaccacattc tgtttcttgg ccaggaacga aggaaggatt gctgccgatg 1260
catgattgct tccttgatga acgcagattc gaaatgtatt ctactgtttg agtttcctgt 1320
tcgtcacaca ctgtaccaaa ctgtattgta ccctatcata atctgtactc ggtacctctc 1380
tgtactgctg gtaccaaact gtattgtact ctgtcatgat ctgtactagt ctttggtact 1440
gctggtcaat agtcctacga attgatcatg tcggcagtgc cgtctatgat tgtgcttgtg 1500
attgttaatc cc 1512

<210> 3
<211> 3958
<212> DNA
<213> Hordeum vulgare

<400> 3
ggttaggaga gtggttatag cccaagccag tcgcatccca tatttaatgc tctgtgtctc 60
atttctattg cttataattt tagtgggcaa caatatcact aatgtcccta ttagtggtga 120
ggtgtctaca atgaatattt ccaatcctaa aagactatga ctctggtgtt cagtgcccat 180
tgtgtgtgta tgtgagtaaa tggattagct gattaatgtt tgacacattt tatacacaag 240
gtcaatctac attggatcaa gaaaatgttg agaaagagta aaacccaagg cttatgagtg 300
ttgttgttgt tgaagcaccc catgcatgat tagagacaag gagaaagtgt gttatgaaga 360
agatgatttt ttttaaagga gatgcatgct tagagaaatt aaggttagct agccgactaa 420
tcctactaaa agtaatactt gattattgtc gtttcaatta aaaaaatacc ttgattattg 480
ctgttccggt tcgacgtcat tggtcaagta atcaatcagt gatatcaatt aggttaggcc 540
taactaatac aagtggtcgt ccatttcttt ctgggtctag gctcgatcat ttgcggagct 600
gagccaagtg gctcgcacaa gttcatgtat caggaaaaag ttgagctatt cacgcgccaa 660
attgtgtgtt cctccttgcc acgtagttgt gccgtgtcat tcggcagctg actgttgtgt 720
tcctggcttc ctgccacctg gcatcatgtg atgccacctc agttgttgag tcgtccaatg 780
taccggttcc ctgtcaaccg agaaaatcgg ctatttgcca cttttaatat tgggcttctc 840
aaaattgcca ctctcaagat tggtttcgta aaaatgtcat ccaacccatg tgtactttga 900
ttacaatgcc atttcctctt tttcaatgct ttccttttc ttttcccatt tcccaacatc 960
tgaagggacc aatataccc tagcctatgc acatgtacta gttttttgca tccaacttga 1020
aacaacgccg ttgagcgccc gccgtcggcc gagatcaccg catacagttc gccaagccct 1080
cctctggtcc tcccccatga tccatcagct cttctacctg cacattgtag ggctgctcct 1140
gcttgccgat tgcttggaag tgggctgccg attggctgga catcgcgtgt cacaggttgc 1200
ctggacgcca tgcgcccacg cgcctcttat cgacactctg cgtcatctat ctctaggtat 1260
cgcagtggaa tggaactcaa tccttctaca cagtattctt ctatctgtac cttcgaatat 1320
gccattactt gtgttaacct gcacatcaga gacaactagt agaagtatac aagggaaatt 1380
ttagaatatg taaattctat tctgcatggt tcatttagaa tgatgtcatg ttctcctaca 1440
acttgatgat ttgttctacc ttggtggtgt tatgtacatg tcattgatac cacattacat 1500
tcaggcaaac tgtagcgaat cttctgaata tatgttcaac aatatgaaaa tgagaagcaa 1560
cgtgttcatt tcattgtata acacacatac atgcagacgc cttgctttct tgttgggaga 1620
ggatacatct tcacaagcaa caacaacaac tacagaggat gtccactaca gctccaacaa 1680
agaagttgac tccaaaaaga aagctaaaaa ttgaaattgg atgaacgatc tctacgaaat 1740
tgatgctata gttatttgag gaagttgaat actcccttcg ttaataaact tgaacatcta 1800
ggttacctgc atgttcctgc ctcacctctc tagcatgtca cacgacgagc tgcgctagcg 1860
cacatgcacg gatgagctgc ggttgctcgc gggcatggcc ggcaagctat acgacggctc 1920
gtgcaaatgt ggacgagcac caacggcgag ctccgccgtc gcgcacaggc ttgggtgagc 1980
aacggtgggt ggtggcgaac tgcagttgct ggtgcaggcg ttggcaagct agggcggcga 2040
gctgcgatgc ctcgcactgg catcagcgag ctacggcgac gggctttggt ggagaacgaa 2100
acacctcaca gacccaacca atccacacag gcgtgagata aggttgaatt atccaggggt 2160
attttggtcc cttcaggtgc caggaaatgg aaaaagaaaa agaaaaccaa taaaaaagag 2220
aaaatggcat gttaatcaaa gtgcaatcaa actgagtggc acttttacgc agccaatctt 2280
aagagtggca attttgagaa gccgagtatt gaaaatggta aataaccaat tttctcctgc 2340
caaccacacg acacacacga tcgtcttggt cagctagctt gcgtttataa gtaggcgcag 2400
ctccgtctct cggtgaccaa cacaagacgt gagagaagaa agcgcgagac aggaagataa 2460
aacaatgtgt ggcggcgcca tcctagcgca gctgatcccg ccgtcggcgg gccgtccgtc 2520
gaagcaggcg gcagcgggcg gccgggcccc gcccacgagc tccaagaagg gcggcgtgag 2580
caagagccgc cacagcagca ccccagatgc cgacgacgac gtcttcgagg ccgccttcga 2640
ggacttcgat gaccacttcg acctgcgggc ggaggaggac ggcggcgacg accatgtcgt 2700
ctttgcatcc aagcctgcct tctctccacg tccgggtagg ctataactct catagtttca 2760
tctgagtgtg cgatcatgaa acgacaaggt tcacggccgt tggttcttgg taccatggaa 2820
gaggaagact gatgagattc ctttttgcag cctacgacgg tggccgcgcg gcgcatgcgg 2880

```
cgagcaggaa gaagcgcacc ggccacctcc atggcatccg gcagcggccg tggggcaagt     2940
gggcggcgga gatccgcgac ccgcacaagg gcacccgcgt ctggctcggc acgttcgaca     3000
cggccgatga tgccgcccgg gcctacgacg tcgccgcccg tcgcctccgt ggcagcaagg     3060
ccaaggtcaa cttccccgac gcggccagga ccggggctcg cccgcgccgc gccagccgta     3120
gaaccgcgca gaaaccgcaa tgcccccctg cgcggacgac ggcgtactct gccaccgcag     3180
cagcacgcgc acagccggag caggacgcta tgatggtcaa acccgagctg atggagtttt     3240
tcaacgtgga cgccatcgtc cacctgacca ctgccgtcgc cgcgctaccg cctgtcacgg     3300
cgagcacctt cgccgacacg atgccgaggg tcgacgagga ctcttctgtg gggagcggcg     3360
gcggcgccat gctggggttc gccgacgagc ttgggttcga tccgttcatg atgttccagc     3420
taccctgctc ggacatgtac gaatccgccg acagcatctt cgccggagac gctgtcatcc     3480
cggatgccct cagcgtggac agtggcatgg acgccgtcag cctctggagc ttcgacgagt     3540
tccccatgga cagcgccatt ttctgacgct ttccgtgtga tgcactgcac tctgttggtt     3600
gtaagaatct ccacctggcc tctacgtagt tccttgtaaa tgcccgcgca cagaaccttg     3660
ctcagaccag attctgtttc ttggccagga acgaaaggaa gggttgctgc cgatgcatga     3720
ttgcttcctc gatgaacgca gattcgaaat gtattctact gtttgagttt cttgttcgtc     3780
acacactgta ccaaactgta ttgtacccta tcataatttc tgctcggtac ctctctgtac     3840
tgctggtacc aaactgtatt gtactctgtc atgatctgta ctagtctttg gtactgctgg     3900
tcaatagtcc tacgaattga tcaaaaaaaa aaaaaaaaaa aaaaaaaaaa aaaaaaa        3958
```

```
<210> 4
<211> 792
<212> DNA
<213> Hordeum vulgare

<220>
<221> CDS
<222> (1)..(792)

<400> 4
atg aaa cgt aca agg ttc acg gcc gtt ggt tct tgg tac cat gga aga       48
Met Lys Arg Thr Arg Phe Thr Ala Val Gly Ser Trp Tyr His Gly Arg
1               5                   10                  15
gga aga ctg atg aga ttc ctt ttt gca gcc tac gac ggt ggc cgc gcg       96
Gly Arg Leu Met Arg Phe Leu Phe Ala Ala Tyr Asp Gly Gly Arg Ala
            20                  25                  30
gcg cat gcg gcg agc agg aag aag cgc acc ggc cac ctc cat ggc atc       144
Ala His Ala Ala Ser Arg Lys Lys Arg Thr Gly His Leu His Gly Ile
        35                  40                  45
cgg cag cgg ccg tgg ggc aag tgg gcg gcg gag atc cgc gac ccg cac       192
Arg Gln Arg Pro Trp Gly Lys Trp Ala Ala Glu Ile Arg Asp Pro His
    50                  55                  60
aag ggc acc cgc gtc tgg ctc ggc acg ttc gac acg gcc gat gat gcc       240
Lys Gly Thr Arg Val Trp Leu Gly Thr Phe Asp Thr Ala Asp Asp Ala
65                  70                  75                  80
gcc cgg gcc tac gac gtc gcc gcc cgt cgc ctc cgt ggc agc aag gcc       288
Ala Arg Ala Tyr Asp Val Ala Ala Arg Arg Leu Arg Gly Ser Lys Ala
                85                  90                  95
aag gtc aac ttc ccc gac gcg gcc agg acc ggg gct cgc ccg cgc cgc       336
Lys Val Asn Phe Pro Asp Ala Ala Arg Thr Gly Ala Arg Pro Arg Arg
            100                 105                 110
gcc agc cgt aga acc gcg cag aaa ccg caa tgc ccc cct gcg cgg acg       384
Ala Ser Arg Arg Thr Ala Gln Lys Pro Gln Cys Pro Pro Ala Arg Thr
        115                 120                 125
acg gcg tac tct gcc acc gca gca gca cgc gca cag ccg gag cag gac       432
Thr Ala Tyr Ser Ala Thr Ala Ala Ala Arg Ala Gln Pro Glu Gln Asp
    130                 135                 140
gct atg atg gtc aaa ccc gag ctg atg gag ttt ttc aac gtg gac gcc       480
Ala Met Met Val Lys Pro Glu Leu Met Glu Phe Phe Asn Val Asp Ala
145                 150                 155                 160
atc gtc cac ctg acc act gcc gtc gcc gcg cta ccg cct gtc acg gcg       528
Ile Val His Leu Thr Thr Ala Val Ala Ala Leu Pro Pro Val Thr Ala
                165                 170                 175
agc acc ttc gcc gac acg atg ccg agg gtc gac gag gac tct tct gtg       576
```

```
Ser Thr Phe Ala Asp Thr Met Pro Arg Val Asp Glu Asp Ser Ser Val
            180                 185                 190
ggg agc ggc ggc ggc gcc atg ctg ggg ttc gcc gac gag ctt ggg ttc       624
Gly Ser Gly Gly Gly Ala Met Leu Gly Phe Ala Asp Glu Leu Gly Phe
        195                 200                 205
gat ccg ttc atg atg ttc cag cta ccc tgc tcg gac atg tac gaa tcc       672
Asp Pro Phe Met Met Phe Gln Leu Pro Cys Ser Asp Met Tyr Glu Ser
    210                 215                 220
gcc gac agc atc ttc gcc gga gac gct gtc atc ccg gat gcc ctc agc       720
Ala Asp Ser Ile Phe Ala Gly Asp Ala Val Ile Pro Asp Ala Leu Ser
225                 230                 235                 240
gtg gac agt ggc atg gac gcc gtc agc ctc tgg agc ttc gac gag ttc       768
Val Asp Ser Gly Met Asp Ala Val Ser Leu Trp Ser Phe Asp Glu Phe
                245                 250                 255
ccc atg gac agc gcc att ttc tga                                       792
Pro Met Asp Ser Ala Ile Phe
            260

<210> 5
<211> 263
<212> PRT
<213> Hordeum vulgare

<400> 5
Met Lys Arg Thr Arg Phe Thr Ala Val Gly Ser Trp Tyr His Gly Arg
1               5                   10                  15
Gly Arg Leu Met Arg Phe Leu Phe Ala Ala Tyr Asp Gly Gly Arg Ala
            20                  25                  30
Ala His Ala Ala Ser Arg Lys Lys Arg Thr Gly His Leu His Gly Ile
        35                  40                  45
Arg Gln Arg Pro Trp Gly Lys Trp Ala Ala Glu Ile Arg Asp Pro His
    50                  55                  60
Lys Gly Thr Arg Val Trp Leu Gly Thr Phe Asp Thr Ala Asp Asp Ala
65                  70                  75                  80
Ala Arg Ala Tyr Asp Val Ala Ala Arg Arg Leu Arg Gly Ser Lys Ala
                85                  90                  95
Lys Val Asn Phe Pro Asp Ala Ala Arg Thr Gly Ala Arg Pro Arg Arg
            100                 105                 110
Ala Ser Arg Arg Thr Ala Gln Lys Pro Gln Cys Pro Pro Ala Arg Thr
        115                 120                 125
Thr Ala Tyr Ser Ala Thr Ala Ala Ala Arg Ala Gln Pro Glu Gln Asp
    130                 135                 140
Ala Met Met Val Lys Pro Glu Leu Met Glu Phe Phe Asn Val Asp Ala
145                 150                 155                 160
Ile Val His Leu Thr Thr Ala Val Ala Ala Leu Pro Pro Val Thr Ala
                165                 170                 175
Ser Thr Phe Ala Asp Thr Met Pro Arg Val Asp Glu Asp Ser Ser Val
            180                 185                 190
Gly Ser Gly Gly Gly Ala Met Leu Gly Phe Ala Asp Glu Leu Gly Phe
        195                 200                 205
Asp Pro Phe Met Met Phe Gln Leu Pro Cys Ser Asp Met Tyr Glu Ser
    210                 215                 220
Ala Asp Ser Ile Phe Ala Gly Asp Ala Val Ile Pro Asp Ala Leu Ser
225                 230                 235                 240
Val Asp Ser Gly Met Asp Ala Val Ser Leu Trp Ser Phe Asp Glu Phe
                245                 250                 255
Pro Met Asp Ser Ala Ile Phe
            260

<210> 6
<211> 987
<212> DNA
<213> Hordeum vulgare
```

<220>
<221> CDS
<222> (1)..(987)

<400> 6
```
atg tgt ggc ggc gcc atc cta gcg cag ctg atc ccg ccg tcg gcg ggc       48
Met Cys Gly Gly Ala Ile Leu Ala Gln Leu Ile Pro Pro Ser Ala Gly
 1               5                  10                  15
cgt ccg tcg aag cag gcg gca gcg ggc ggc cgg gcc ccg ccc acg agc       96
Arg Pro Ser Lys Gln Ala Ala Ala Gly Gly Arg Ala Pro Pro Thr Ser
            20                  25                  30
tcc aag aag ggc ggc gtg agc aag agc cgc cac agc agc acc cca gat      144
Ser Lys Lys Gly Gly Val Ser Lys Ser Arg His Ser Ser Thr Pro Asp
        35                  40                  45
gcc gac gac gac gtc ttc gag gcc gcc ttc gag gac ttc gat gac cac      192
Ala Asp Asp Asp Val Phe Glu Ala Ala Phe Glu Asp Phe Asp Asp His
    50                  55                  60
ttc gac ctg cgg gcg gag gag gac ggc ggc gac gac cat gtc gtc ttt      240
Phe Asp Leu Arg Ala Glu Glu Asp Gly Gly Asp Asp His Val Val Phe
65                  70                  75                  80
gca tcc aag cct gcc ttc tct cca cgt ccg gcc tac gac ggt ggc cgc      288
Ala Ser Lys Pro Ala Phe Ser Pro Arg Pro Ala Tyr Asp Gly Gly Arg
                85                  90                  95
gcg gcg cat gcg gcg agc agg aag aag cgc acc ggc cac ctc cat ggc      336
Ala Ala His Ala Ala Ser Arg Lys Lys Arg Thr Gly His Leu His Gly
            100                 105                 110
atc cgg cag cgg ccg tgg ggc aag tgg gcg gcg gag atc cgc gac ccg      384
Ile Arg Gln Arg Pro Trp Gly Lys Trp Ala Ala Glu Ile Arg Asp Pro
        115                 120                 125
cac aag ggc acc cgc gtc tgg ctc ggc acg ttc gac acg gcc gat gat      432
His Lys Gly Thr Arg Val Trp Leu Gly Thr Phe Asp Thr Ala Asp Asp
    130                 135                 140
gcc gcc cgg gcc tac gac gtc gcc gcc cgt cgc ctc cgt ggc agc aag      480
Ala Ala Arg Ala Tyr Asp Val Ala Ala Arg Arg Leu Arg Gly Ser Lys
145                 150                 155                 160
gcc aag gtc aac ttc ccc gac gcg gcc agg acc ggg gct cgc ccg cgc      528
Ala Lys Val Asn Phe Pro Asp Ala Ala Arg Thr Gly Ala Arg Pro Arg
                165                 170                 175
cgc gcc agc cgt aga acc gcg cag aaa ccg caa tgc ccc cct gcg cgg      576
Arg Ala Ser Arg Arg Thr Ala Gln Lys Pro Gln Cys Pro Pro Ala Arg
            180                 185                 190
acg acg gcg tac tct gcc acc gca gca gca cgc gca cag ccg gag cag      624
Thr Thr Ala Tyr Ser Ala Thr Ala Ala Ala Arg Ala Gln Pro Glu Gln
        195                 200                 205
gac gct atg atg gtc aaa ccc gag ctg atg gag ttt ttc aac gtg gac      672
Asp Ala Met Met Val Lys Pro Glu Leu Met Glu Phe Phe Asn Val Asp
    210                 215                 220
gcc atc gtc cac ctg acc act gcc gtc gcc gcg cta ccg cct gtc acg      720
Ala Ile Val His Leu Thr Thr Ala Val Ala Ala Leu Pro Pro Val Thr
225                 230                 235                 240
gcg agc acc ttc gcc gac acg atg ccg agg gtc gac gag gac tct tct      768
Ala Ser Thr Phe Ala Asp Thr Met Pro Arg Val Asp Glu Asp Ser Ser
                245                 250                 255
gtg ggg agc ggc ggc ggc gcc atg ctg ggg ttc gcc gac gag ctt ggg      816
Val Gly Ser Gly Gly Gly Ala Met Leu Gly Phe Ala Asp Glu Leu Gly
            260                 265                 270
ttc gat ccg ttc atg atg ttc ctg cta ccc tgc tcg gac atg tac gaa      864
Phe Asp Pro Phe Met Met Phe Leu Leu Pro Cys Ser Asp Met Tyr Glu
        275                 280                 285
tcc gcc gac agc atc ttc gcc gga gac gct gtc atc ccg gat gcc ctc      912
Ser Ala Asp Ser Ile Phe Ala Gly Asp Ala Val Ile Pro Asp Ala Leu
    290                 295                 300
```

```
agc gtg gac agt ggc atg gac gcc gtc agc ctc tgg agc ttc gac gag         960
Ser Val Asp Ser Gly Met Asp Ala Val Ser Leu Trp Ser Phe Asp Glu
305                 310                 315                 320
ttc ccc atg gac agc gcc att ttc tga                                     987
Phe Pro Met Asp Ser Ala Ile Phe
                325
```

<210> 7
<211> 328
<212> PRT
<213> Hordeum vulgare

<400> 7

```
Met Cys Gly Gly Ala Ile Leu Ala Gln Leu Ile Pro Pro Ser Ala Gly
1               5                   10                  15
Arg Pro Ser Lys Gln Ala Ala Ala Gly Gly Arg Ala Pro Pro Thr Ser
            20                  25                  30
Ser Lys Lys Gly Gly Val Ser Lys Ser Arg His Ser Ser Thr Pro Asp
        35                  40                  45
Ala Asp Asp Asp Val Phe Glu Ala Ala Phe Glu Asp Phe Asp Asp His
    50                  55                  60
Phe Asp Leu Arg Ala Glu Glu Asp Gly Gly Asp Asp His Val Val Phe
65                  70                  75                  80
Ala Ser Lys Pro Ala Phe Ser Pro Arg Pro Ala Tyr Asp Gly Gly Arg
                85                  90                  95
Ala Ala His Ala Ala Ser Arg Lys Lys Arg Thr Gly His Leu His Gly
            100                 105                 110
Ile Arg Gln Arg Pro Trp Gly Lys Trp Ala Ala Glu Ile Arg Asp Pro
        115                 120                 125
His Lys Gly Thr Arg Val Trp Leu Gly Thr Phe Asp Thr Ala Asp Asp
    130                 135                 140
Ala Ala Arg Ala Tyr Asp Val Ala Ala Arg Arg Leu Arg Gly Ser Lys
145                 150                 155                 160
Ala Lys Val Asn Phe Pro Asp Ala Ala Arg Thr Gly Ala Arg Pro Arg
                165                 170                 175
Arg Ala Ser Arg Arg Thr Ala Gln Lys Pro Gln Cys Pro Pro Ala Arg
            180                 185                 190
Thr Thr Ala Tyr Ser Ala Thr Ala Ala Ala Arg Ala Gln Pro Glu Gln
        195                 200                 205
Asp Ala Met Met Val Lys Pro Glu Leu Met Glu Phe Phe Asn Val Asp
    210                 215                 220
Ala Ile Val His Leu Thr Thr Ala Val Ala Ala Leu Pro Pro Val Thr
225                 230                 235                 240
Ala Ser Thr Phe Ala Asp Thr Met Pro Arg Val Asp Glu Asp Ser Ser
                245                 250                 255
Val Gly Ser Gly Gly Gly Ala Met Leu Gly Phe Ala Asp Glu Leu Gly
            260                 265                 270
Phe Asp Pro Phe Met Met Phe Leu Leu Pro Cys Ser Asp Met Tyr Glu
        275                 280                 285
Ser Ala Asp Ser Ile Phe Ala Gly Asp Ala Val Ile Pro Asp Ala Leu
    290                 295                 300
Ser Val Asp Ser Gly Met Asp Ala Val Ser Leu Trp Ser Phe Asp Glu
305                 310                 315                 320
Phe Pro Met Asp Ser Ala Ile Phe
                325
```

<210> 8
<211> 1008
<212> DNA
<213> Sorghum bicolor

<220>
<221> CDS

```
<222>  (1)..(1008)

<400>  8
atg tgc gga ggc gcc atc ctc gcg gaa ctg atc ccg ccg acg cgg cgc        48
Met Cys Gly Gly Ala Ile Leu Ala Glu Leu Ile Pro Pro Thr Arg Arg
1               5                   10                  15
gtg gcg ccg aag ccg gtg acc gag ggc cac ctc tgg ccg gcg agc tcc        96
Val Ala Pro Lys Pro Val Thr Glu Gly His Leu Trp Pro Ala Ser Ser
            20                  25                  30
aag aaa gcc ggc agc ggc agc aac aag agg cac cac cac gaa tac gcc       144
Lys Lys Ala Gly Ser Gly Ser Asn Lys Arg His His His Glu Tyr Ala
        35                  40                  45
gac gac gac gat ttt gag gcc gcc ttc gag gag ttc gac gac gac ttt       192
Asp Asp Asp Asp Phe Glu Ala Ala Phe Glu Glu Phe Asp Asp Asp Phe
    50                  55                  60
gac gtg cac gaa gac gac gag gac cac cct gac cac ttc gtg ttc tcg       240
Asp Val His Glu Asp Asp Glu Asp His Pro Asp His Phe Val Phe Ser
65                  70                  75                  80
tcc aaa tcc gct ttc tcc cca gcg cac gac ggc agg cgc gat gcg cga       288
Ser Lys Ser Ala Phe Ser Pro Ala His Asp Gly Arg Arg Asp Ala Arg
                85                  90                  95
gcc gcg gcg agc cag agg acg aag cgc ggc cgc cgc cac ttc cac ggc       336
Ala Ala Ala Ser Gln Arg Thr Lys Arg Gly Arg Arg His Phe His Gly
            100                 105                 110
atc cgg cag cgt ccc tgg ggc aag tgg gcg gcg gag atc cgc gac ccg       384
Ile Arg Gln Arg Pro Trp Gly Lys Trp Ala Ala Glu Ile Arg Asp Pro
        115                 120                 125
cac aag ggc acc cgc gtc tgg ctc ggc acc ttc aac acc gcc gag gac       432
His Lys Gly Thr Arg Val Trp Leu Gly Thr Phe Asn Thr Ala Glu Asp
    130                 135                 140
gcc gcc cgg gcc tac gac gtc gag gcg cgc cgc ctc cgc ggc agc aag       480
Ala Ala Arg Ala Tyr Asp Val Glu Ala Arg Arg Leu Arg Gly Ser Lys
145                 150                 155                 160
gcc aag gtc aac ttc ccc gca gcc agc gcg cgc cca cgc gca cgc cgc       528
Ala Lys Val Asn Phe Pro Ala Ala Ser Ala Arg Pro Arg Ala Arg Arg
                165                 170                 175
ggc aag ggc aac cca aga acc gcg ccg aaa cct gca gca cag gca cag       576
Gly Lys Gly Asn Pro Arg Thr Ala Pro Lys Pro Ala Ala Gln Ala Gln
            180                 185                 190
ccc gct ctg ctc ctt cga ggg gag aag ggt ctg gaa gga gtt gcc gtg       624
Pro Ala Leu Leu Leu Arg Gly Glu Lys Gly Leu Glu Gly Val Ala Val
        195                 200                 205
aag cct gaa act act atg gac atg ttc gac gtg ggc agc ttc ttc gac       672
Lys Pro Glu Thr Thr Met Asp Met Phe Asp Val Gly Ser Phe Phe Asp
    210                 215                 220
atg acc ttc ccc acc ttc cca gcc gcg ccg gcg gcg gcc atg gag agc       720
Met Thr Phe Pro Thr Phe Pro Ala Ala Pro Ala Ala Ala Met Glu Ser
225                 230                 235                 240
tcc ttc gct ggc agc agc gcc acg tcg gag acc cgg agc ccc ggg aag       768
Ser Phe Ala Gly Ser Ser Ala Thr Ser Glu Thr Arg Ser Pro Gly Lys
                245                 250                 255
aag ctg aga tac gac gac ggc gac tcg tcc gag ggg agc ggc tgt ggc       816
Lys Leu Arg Tyr Asp Asp Gly Asp Ser Ser Glu Gly Ser Gly Cys Gly
            260                 265                 270
tcc gcg ctg gag ctc gct gac gag ctg gcg ttc gat ccg ttt atg cta       864
Ser Ala Leu Glu Leu Ala Asp Glu Leu Ala Phe Asp Pro Phe Met Leu
        275                 280                 285
ctg cag atg ccc tac tct ggc ggg tac gat tcc ctg gac ggc ctt ttc       912
Leu Gln Met Pro Tyr Ser Gly Gly Tyr Asp Ser Leu Asp Gly Leu Phe
    290                 295                 300
gcc gca gag gcc gtg cag cag gac gtg aac aac gac atg aac ggc gtc       960
Ala Ala Glu Ala Val Gln Gln Asp Val Asn Asn Asp Met Asn Gly Val
305                 310                 315                 320
```

```
agc ctg tgg agc ttc gac gag ttc ccc gtc gac ggc tat gtt ttc           1005
Ser Leu Trp Ser Phe Asp Glu Phe Pro Val Asp Gly Tyr Val Phe
                325                 330                 335
tga                                                                   1008


<210> 9
<211> 335
<212> PRT
<213> Sorghum bicolor

<400> 9
Met Cys Gly Gly Ala Ile Leu Ala Glu Leu Ile Pro Pro Thr Arg Arg
1               5                   10                  15
Val Ala Pro Lys Pro Val Thr Glu Gly His Leu Trp Pro Ala Ser Ser
            20                  25                  30
Lys Lys Ala Gly Ser Gly Ser Asn Lys Arg His His His Glu Tyr Ala
        35                  40                  45
Asp Asp Asp Asp Phe Glu Ala Ala Phe Glu Glu Phe Asp Asp Asp Phe
    50                  55                  60
Asp Val His Glu Asp Asp Glu Asp His Pro Asp His Phe Val Phe Ser
65                  70                  75                  80
Ser Lys Ser Ala Phe Ser Pro Ala His Asp Gly Arg Arg Asp Ala Arg
                85                  90                  95
Ala Ala Ala Ser Gln Arg Thr Lys Arg Gly Arg Arg His Phe His Gly
            100                 105                 110
Ile Arg Gln Arg Pro Trp Gly Lys Trp Ala Ala Glu Ile Arg Asp Pro
        115                 120                 125
His Lys Gly Thr Arg Val Trp Leu Gly Thr Phe Asn Thr Ala Glu Asp
    130                 135                 140
Ala Ala Arg Ala Tyr Asp Val Glu Ala Arg Arg Leu Arg Gly Ser Lys
145                 150                 155                 160
Ala Lys Val Asn Phe Pro Ala Ala Ser Ala Arg Pro Arg Ala Arg Arg
                165                 170                 175
Gly Lys Gly Asn Pro Arg Thr Ala Pro Lys Pro Ala Ala Gln Ala Gln
            180                 185                 190
Pro Ala Leu Leu Leu Arg Gly Glu Lys Gly Leu Glu Gly Val Ala Val
        195                 200                 205
Lys Pro Glu Thr Thr Met Asp Met Phe Asp Val Gly Ser Phe Phe Asp
    210                 215                 220
Met Thr Phe Pro Thr Phe Pro Ala Ala Pro Ala Ala Ala Met Glu Ser
225                 230                 235                 240
Ser Phe Ala Gly Ser Ser Ala Thr Ser Glu Thr Arg Ser Pro Gly Lys
                245                 250                 255
Lys Leu Arg Tyr Asp Asp Gly Asp Ser Ser Glu Gly Ser Gly Cys Gly
            260                 265                 270
Ser Ala Leu Glu Leu Ala Asp Glu Leu Ala Phe Asp Pro Phe Met Leu
        275                 280                 285
Leu Gln Met Pro Tyr Ser Gly Gly Tyr Asp Ser Leu Asp Gly Leu Phe
    290                 295                 300
Ala Ala Glu Ala Val Gln Gln Asp Val Asn Asn Asp Met Asn Gly Val
305                 310                 315                 320
Ser Leu Trp Ser Phe Asp Glu Phe Pro Val Asp Gly Tyr Val Phe
                325                 330                 335

<210> 10
<211> 1005
<212> DNA
<213> Oryza sativa Japonica Group

<220>
<221> CDS
```

<222> (1)..(1005)

<400> 10

```
atg tgt gga ggc gcc atc ctc gcc gag ttc atc ccg gcg ccg tcg cgc      48
Met Cys Gly Gly Ala Ile Leu Ala Glu Phe Ile Pro Ala Pro Ser Arg
1               5                   10                  15
gcc gcg gcg gcg acc aag cgg gtg acc gcc agc cac ctg tgg ccg gcc      96
Ala Ala Ala Ala Thr Lys Arg Val Thr Ala Ser His Leu Trp Pro Ala
            20                  25                  30
ggc tcc aag aac gcc gcc cgc ggc aag agc aag agc aag agg cag cag      144
Gly Ser Lys Asn Ala Ala Arg Gly Lys Ser Lys Ser Lys Arg Gln Gln
        35                  40                  45
agg agc ttc gcc gac gtc gac gac ttc gag gcc gcc ttc gag cag ttc      192
Arg Ser Phe Ala Asp Val Asp Asp Phe Glu Ala Ala Phe Glu Gln Phe
    50                  55                  60
gac gat gac tcc gac ttc gac gac gcg gag gaa gaa gac gaa gga cac      240
Asp Asp Asp Ser Asp Phe Asp Asp Ala Glu Glu Glu Asp Glu Gly His
65                  70                  75                  80
ttc gtg ttc gcg tcc aaa tct cgt gtc gtc gcc ggg cac gac ggg cgc      288
Phe Val Phe Ala Ser Lys Ser Arg Val Val Ala Gly His Asp Gly Arg
                85                  90                  95
gcg gcg gcg agg gcg gcg agc aag aag aag cgg ggg cgg cac ttc cga      336
Ala Ala Ala Arg Ala Ala Ser Lys Lys Lys Arg Gly Arg His Phe Arg
            100                 105                 110
ggc atc cgg cag cgg cca tgg ggg aag tgg gcg gcg gag atc cgc gac      384
Gly Ile Arg Gln Arg Pro Trp Gly Lys Trp Ala Ala Glu Ile Arg Asp
        115                 120                 125
ccg cac aag ggc acg cgc gtc tgg ctc ggc acg ttc aac acc ccg gag      432
Pro His Lys Gly Thr Arg Val Trp Leu Gly Thr Phe Asn Thr Pro Glu
    130                 135                 140
gag gcc gca cgc gcc tac gac gtc gag gcg cgc cgc ctc cgc ggc agc      480
Glu Ala Ala Arg Ala Tyr Asp Val Glu Ala Arg Arg Leu Arg Gly Ser
145                 150                 155                 160
aag gcc aag gtc aac ttc ccc gcc acg ccc gcc gcc gcg cgc cca cgc      528
Lys Ala Lys Val Asn Phe Pro Ala Thr Pro Ala Ala Ala Arg Pro Arg
                165                 170                 175
cgc ggc aac acg aga gcc acc gcc gtg cca ccg ccg gcg aca gca ccc      576
Arg Gly Asn Thr Arg Ala Thr Ala Val Pro Pro Pro Ala Thr Ala Pro
            180                 185                 190
gcc gcc gcc ccg ccg cgc gga ctg aag cga gaa ttc tcg ccg cct gct      624
Ala Ala Ala Pro Pro Arg Gly Leu Lys Arg Glu Phe Ser Pro Pro Ala
        195                 200                 205
gag acc gcg cta cct ttc ttc acc aac ggc ttc gtc gac ctg acg acc      672
Glu Thr Ala Leu Pro Phe Phe Thr Asn Gly Phe Val Asp Leu Thr Thr
    210                 215                 220
gcc gcg gcg ccg cca ccg gcc atg atg atg acg agc tcc ttc acc gac      720
Ala Ala Ala Pro Pro Pro Ala Met Met Met Thr Ser Ser Phe Thr Asp
225                 230                 235                 240
agc gtc gcc acg tcg gag tcc ggc ggg agc ccc gcc aag aag gcg agg      768
Ser Val Ala Thr Ser Glu Ser Gly Gly Ser Pro Ala Lys Lys Ala Arg
                245                 250                 255
tcc gac gac gtc gac tcg tcc gag ggc agc gtc ggc ggc ggc agc gac      816
Ser Asp Asp Val Asp Ser Ser Glu Gly Ser Val Gly Gly Gly Ser Asp
            260                 265                 270
acg ctg ggt ttc acc gac gag ctg gag ttc gac ccg ttc atg ctg ttc      864
Thr Leu Gly Phe Thr Asp Glu Leu Glu Phe Asp Pro Phe Met Leu Phe
        275                 280                 285
cag ctc ccc tac tcc gac ggc tac gag tcc atc gac agc ctc ttc gcc      912
Gln Leu Pro Tyr Ser Asp Gly Tyr Glu Ser Ile Asp Ser Leu Phe Ala
    290                 295                 300
gcc ggc gac gcc aac agc gcg aac acc gac atg aac gcc ggc gtc aac      960
Ala Gly Asp Ala Asn Ser Ala Asn Thr Asp Met Asn Ala Gly Val Asn
305                 310                 315                 320
```

```
ctg tgg agc ttc gac gac ttc cca atc gac ggc gcc ctt ttc tga          1005
Leu Trp Ser Phe Asp Asp Phe Pro Ile Asp Gly Ala Leu Phe
                325                 330
```

<210> 11
<211> 334
<212> PRT
<213> Oryza sativa Japonica Group

<400> 11

```
Met Cys Gly Gly Ala Ile Leu Ala Glu Phe Ile Pro Ala Pro Ser Arg
1               5                   10                  15
Ala Ala Ala Ala Thr Lys Arg Val Thr Ala Ser His Leu Trp Pro Ala
            20                  25                  30
Gly Ser Lys Asn Ala Ala Arg Gly Lys Ser Lys Ser Lys Arg Gln Gln
        35                  40                  45
Arg Ser Phe Ala Asp Val Asp Asp Phe Glu Ala Ala Phe Glu Gln Phe
    50                  55                  60
Asp Asp Asp Ser Asp Phe Asp Asp Ala Glu Glu Glu Asp Glu Gly His
65                  70                  75                  80
Phe Val Phe Ala Ser Lys Ser Arg Val Val Ala Gly His Asp Gly Arg
                85                  90                  95
Ala Ala Ala Arg Ala Ala Ser Lys Lys Lys Arg Gly Arg His Phe Arg
            100                 105                 110
Gly Ile Arg Gln Arg Pro Trp Gly Lys Trp Ala Ala Glu Ile Arg Asp
        115                 120                 125
Pro His Lys Gly Thr Arg Val Trp Leu Gly Thr Phe Asn Thr Pro Glu
    130                 135                 140
Glu Ala Ala Arg Ala Tyr Asp Val Glu Ala Arg Arg Leu Arg Gly Ser
145                 150                 155                 160
Lys Ala Lys Val Asn Phe Pro Ala Thr Pro Ala Ala Ala Arg Pro Arg
                165                 170                 175
Arg Gly Asn Thr Arg Ala Thr Ala Val Pro Pro Pro Ala Thr Ala Pro
            180                 185                 190
Ala Ala Ala Pro Pro Arg Gly Leu Lys Arg Glu Phe Ser Pro Pro Ala
        195                 200                 205
Glu Thr Ala Leu Pro Phe Phe Thr Asn Gly Phe Val Asp Leu Thr Thr
    210                 215                 220
Ala Ala Ala Pro Pro Pro Ala Met Met Met Thr Ser Ser Phe Thr Asp
225                 230                 235                 240
Ser Val Ala Thr Ser Glu Ser Gly Gly Ser Pro Ala Lys Lys Ala Arg
                245                 250                 255
Ser Asp Asp Val Asp Ser Ser Glu Gly Ser Val Gly Gly Gly Ser Asp
            260                 265                 270
Thr Leu Gly Phe Thr Asp Glu Leu Glu Phe Asp Pro Phe Met Leu Phe
        275                 280                 285
Gln Leu Pro Tyr Ser Asp Gly Tyr Glu Ser Ile Asp Ser Leu Phe Ala
    290                 295                 300
Ala Gly Asp Ala Asn Ser Ala Asn Thr Asp Met Asn Ala Gly Val Asn
305                 310                 315                 320
Leu Trp Ser Phe Asp Asp Phe Pro Ile Asp Gly Ala Leu Phe
                325                 330
```

<210> 12
<211> 924
<212> DNA
<213> Brachypodium distachyon

<220>
<221> CDS
<222> (1)..(924)

<400> 12

```
atg tgc ggc ggc gcc atc cta gca gag ctg acc ccg ccg ccg gcg cgc        48
Met Cys Gly Gly Ala Ile Leu Ala Glu Leu Thr Pro Pro Pro Ala Arg
 1               5                  10                  15
ggt ggt ggt gcg tcg aag ccg gtc ggc gcc aag ggc caa gtc tgg ccg        96
Gly Gly Gly Ala Ser Lys Pro Val Gly Ala Lys Gly Gln Val Trp Pro
            20                  25                  30
gtg agc tcc aag aag gaa ggc ggc acg aac aag acg cgc cac ggc agc        144
Val Ser Ser Lys Lys Glu Gly Gly Thr Asn Lys Thr Arg His Gly Ser
        35                  40                  45
gtc gtc gac gac ttc gag gct gcc ttc gaa ggc ttc cac gac gac ttc        192
Val Val Asp Asp Phe Glu Ala Ala Phe Glu Gly Phe His Asp Asp Phe
    50                  55                  60
cct gtc ctg ttc gca tcc aag cct gcc ttc tcc cca gtc cat ggc gat        240
Pro Val Leu Phe Ala Ser Lys Pro Ala Phe Ser Pro Val His Gly Asp
65                  70                  75                  80
tcc ggg cgc gcc cgc gag gca cag gcg gcg gca agc tgc agg aag aag        288
Ser Gly Arg Ala Arg Glu Ala Gln Ala Ala Ala Ser Cys Arg Lys Lys
                85                  90                  95
aag cgc gtc cgg ggc ctc cac ggc atc cgg cag cgg ccg tgg ggc aag        336
Lys Arg Val Arg Gly Leu His Gly Ile Arg Gln Arg Pro Trp Gly Lys
            100                 105                 110
tgg gcg gcg gag atc cgc gac ccg cac aag ggc gcc cgc gtc tgg ctc        384
Trp Ala Ala Glu Ile Arg Asp Pro His Lys Gly Ala Arg Val Trp Leu
        115                 120                 125
ggc acg tac gac acc gcc gac gag gcc gcc cgg gcc tac gac gtc gcc        432
Gly Thr Tyr Asp Thr Ala Asp Glu Ala Ala Arg Ala Tyr Asp Val Ala
    130                 135                 140
gcc cgc cgc ctc cgc ggc agc aat gcc aag ctc aac ttc cct gcc gcg        480
Ala Arg Arg Leu Arg Gly Ser Asn Ala Lys Leu Asn Phe Pro Ala Ala
145                 150                 155                 160
cca ggc cgt gcg cgc cca cgc tgc gga acc gcg ccg aaa ccg cga cgc        528
Pro Gly Arg Ala Arg Pro Arg Cys Gly Thr Ala Pro Lys Pro Arg Arg
                165                 170                 175
ccc act act gcg cag acg gcg tgc ttc tct gct att act gcc gca gca        576
Pro Thr Thr Ala Gln Thr Ala Cys Phe Ser Ala Ile Thr Ala Ala Ala
            180                 185                 190
gca gca cgc gcc cag gag atc gag caa gag ctg atg cca atg cca tat        624
Ala Ala Arg Ala Gln Glu Ile Glu Gln Glu Leu Met Pro Met Pro Tyr
        195                 200                 205
ttc gac gtg gac gcg ttc ctc gac ctg gcc gcc tcc gtg gcc gag ctg        672
Phe Asp Val Asp Ala Phe Leu Asp Leu Ala Ala Ser Val Ala Glu Leu
    210                 215                 220
ccg ccg gtt ata atc ggg agc tcc ttc gcc gac agc gcc ggc ggt gcg        720
Pro Pro Val Ile Ile Gly Ser Ser Phe Ala Asp Ser Ala Gly Gly Ala
225                 230                 235                 240
gcc acg cgg ggg ttc gcc gac gag ctg gag ttc gac ccg ttc atg ctg        768
Ala Thr Arg Gly Phe Ala Asp Glu Leu Glu Phe Asp Pro Phe Met Leu
                245                 250                 255
tgt gag ctc ccc tgg tca gaa tcg gac aca tgc gaa tcc atc gaa gga        816
Cys Glu Leu Pro Trp Ser Glu Ser Asp Thr Cys Glu Ser Ile Glu Gly
            260                 265                 270
ctc ttc gcc ggg gac gct gtg cag gat gcc ggc ggc gtg aac act ggc        864
Leu Phe Ala Gly Asp Ala Val Gln Asp Ala Gly Gly Val Asn Thr Gly
        275                 280                 285
atg gac agc gtc agc ctc tgg agc ttc gac gag ttc ccc atg gac gcc        912
Met Asp Ser Val Ser Leu Trp Ser Phe Asp Glu Phe Pro Met Asp Ala
    290                 295                 300
gcc gct ttc tga                                                        924
Ala Ala Phe
305
```

<210> 13
<211> 307

<212> PRT
<213> Brachypodium distachyon

<400> 13
Met Cys Gly Gly Ala Ile Leu Ala Glu Leu Thr Pro Pro Pro Ala Arg
1 5 10 15
Gly Gly Gly Ala Ser Lys Pro Val Gly Ala Lys Gly Gln Val Trp Pro
20 25 30
Val Ser Ser Lys Lys Glu Gly Gly Thr Asn Lys Thr Arg His Gly Ser
35 40 45
Val Val Asp Asp Phe Glu Ala Ala Phe Glu Gly Phe His Asp Asp Phe
50 55 60
Pro Val Leu Phe Ala Ser Lys Pro Ala Phe Ser Pro Val His Gly Asp
65 70 75 80
Ser Gly Arg Ala Arg Glu Ala Gln Ala Ala Ala Ser Cys Arg Lys Lys
85 90 95
Lys Arg Val Arg Gly Leu His Gly Ile Arg Gln Arg Pro Trp Gly Lys
100 105 110
Trp Ala Ala Glu Ile Arg Asp Pro His Lys Gly Ala Arg Val Trp Leu
115 120 125
Gly Thr Tyr Asp Thr Ala Asp Glu Ala Ala Arg Ala Tyr Asp Val Ala
130 135 140
Ala Arg Arg Leu Arg Gly Ser Asn Ala Lys Leu Asn Phe Pro Ala Ala
145 150 155 160
Pro Gly Arg Ala Arg Pro Arg Cys Gly Thr Ala Pro Lys Pro Arg Arg
165 170 175
Pro Thr Thr Ala Gln Thr Ala Cys Phe Ser Ala Ile Thr Ala Ala Ala
180 185 190
Ala Ala Arg Ala Gln Glu Ile Glu Gln Glu Leu Met Pro Met Pro Tyr
195 200 205
Phe Asp Val Asp Ala Phe Leu Asp Leu Ala Ala Ser Val Ala Glu Leu
210 215 220
Pro Pro Val Ile Ile Gly Ser Ser Phe Ala Asp Ser Ala Gly Gly Ala
225 230 235 240
Ala Thr Arg Gly Phe Ala Asp Glu Leu Glu Phe Asp Pro Phe Met Leu
245 250 255
Cys Glu Leu Pro Trp Ser Glu Ser Asp Thr Cys Glu Ser Ile Glu Gly
260 265 270
Leu Phe Ala Gly Asp Ala Val Gln Asp Ala Gly Gly Val Asn Thr Gly
275 280 285
Met Asp Ser Val Ser Leu Trp Ser Phe Asp Glu Phe Pro Met Asp Ala
290 295 300
Ala Ala Phe
305

<210> 14
<211> 1023
<212> DNA
<213> Zea mays

<220>
<221> CDS
<222> (1)..(1023)

<400> 14
atg tgc gga ggc gcc atc ctc gcg gag ctg atc ccg ccg acg cgg cgc 48
Met Cys Gly Gly Ala Ile Leu Ala Glu Leu Ile Pro Pro Thr Arg Arg
1 5 10 15
gtg gcg tcg aag ccg gtg aca gaa ggc cac ctc tgg tcg gcg agc tcc 96
Val Ala Ser Lys Pro Val Thr Glu Gly His Leu Trp Ser Ala Ser Ser
20 25 30
aag aaa gcc ggc agc ggc agg gac aag agg cac cag cac gaa tac gcc 144
Lys Lys Ala Gly Ser Gly Arg Asp Lys Arg His Gln His Glu Tyr Ala

```
        35                  40                  45
gac gat gac ttc gag gcc gcc ttc gag gac ttc gac gac gac ttt gac        192
Asp Asp Asp Phe Glu Ala Ala Phe Glu Asp Phe Asp Asp Asp Phe Asp
    50                  55                  60
gtg cat gaa gac gac gag gac ggc cac ttc gta ttc tcg tcc aaa tcc        240
Val His Glu Asp Asp Glu Asp Gly His Phe Val Phe Ser Ser Lys Ser
65                  70                  75                  80
gcc ttg tcc cca gcc ctg cac gac ggg cgc gcg gcg agc cag aag aag        288
Ala Leu Ser Pro Ala Leu His Asp Gly Arg Ala Ala Ser Gln Lys Lys
                85                  90                  95
cag cgc ggg cgc cag ttc cgc ggc atc cgg cag cgg ccc tgg ggc aag        336
Gln Arg Gly Arg Gln Phe Arg Gly Ile Arg Gln Arg Pro Trp Gly Lys
            100                 105                 110
tgg gcg gcg gag atc cgc gac ccg cac aag ggc acc cgc gtc tgg ctc        384
Trp Ala Ala Glu Ile Arg Asp Pro His Lys Gly Thr Arg Val Trp Leu
        115                 120                 125
ggc acc ttc agc acc gcc gag gac gcc gcc cgg gcc tac gac gtg gag        432
Gly Thr Phe Ser Thr Ala Glu Asp Ala Ala Arg Ala Tyr Asp Val Glu
    130                 135                 140
gcg cgc cgc ctc cgc ggc agc aag gcc aag gtc aac ttc ccc gca gcc        480
Ala Arg Arg Leu Arg Gly Ser Lys Ala Lys Val Asn Phe Pro Ala Ala
145                 150                 155                 160
agc ggt cgc gct cgc ggt cgc gcg cgc cca cgc cgc ggc gac gac ggc        528
Ser Gly Arg Ala Arg Gly Arg Ala Arg Pro Arg Arg Gly Asp Asp Gly
                165                 170                 175
aac cca cga acc gcg ccg gaa acg cag cac cca gca cag ccc gct ctg        576
Asn Pro Arg Thr Ala Pro Glu Thr Gln His Pro Ala Gln Pro Ala Leu
            180                 185                 190
ctg cct cga gga gag aga gag acg cag agg aag gaa ggg atc gcc gcc        624
Leu Pro Arg Gly Glu Arg Glu Thr Gln Arg Lys Glu Gly Ile Ala Ala
        195                 200                 205
gtg aag cca gaa gct acg gag tcg ttc gac gtg ggc ggc ggt ctc ttc        672
Val Lys Pro Glu Ala Thr Glu Ser Phe Asp Val Gly Gly Gly Leu Phe
    210                 215                 220
ttc gac atg gcc ttc ccc acc ttc cca gcc tcg ccg ccg ccg cag gcc        720
Phe Asp Met Ala Phe Pro Thr Phe Pro Ala Ser Pro Pro Pro Gln Ala
225                 230                 235                 240
gtg gat acg tcc ttc gcc ggc agc acc gcc acg tcg gag acc ggg agc        768
Val Asp Thr Ser Phe Ala Gly Ser Thr Ala Thr Ser Glu Thr Gly Ser
                245                 250                 255
ccc gcg aag agg ccg aga tgc gac gaa gac tcg tcc gag ggc ggc agc        816
Pro Ala Lys Arg Pro Arg Cys Asp Glu Asp Ser Ser Glu Gly Gly Ser
            260                 265                 270
ggc tcc gcg ctg gag ctc gct gac gag ctg gcg ttc gac ccg ttt gtg        864
Gly Ser Ala Leu Glu Leu Ala Asp Glu Leu Ala Phe Asp Pro Phe Val
        275                 280                 285
ctg ctg cag atg ccc tac tcg ggt ggg tac gac gac gac tca ctg gac        912
Leu Leu Gln Met Pro Tyr Ser Gly Gly Tyr Asp Asp Asp Ser Leu Asp
    290                 295                 300
ggc ctt ttc gcc gca gat gag gcc gtg cag cag gac gtg ggc aac ggc        960
Gly Leu Phe Ala Ala Asp Glu Ala Val Gln Gln Asp Val Gly Asn Gly
305                 310                 315                 320
atg gac ggc gtc cgc ctg tgg agc ttc gac gag ttc ccc gcc gtc gac        1008
Met Asp Gly Val Arg Leu Trp Ser Phe Asp Glu Phe Pro Ala Val Asp
                325                 330                 335
ggt tct gtt ttc tga                                                    1023
Gly Ser Val Phe
            340
```

<210> 15
<211> 340
<212> PRT
<213> Zea mays

<400> 15
Met Cys Gly Gly Ala Ile Leu Ala Glu Leu Ile Pro Pro Thr Arg Arg
1 5 10 15
Val Ala Ser Lys Pro Val Thr Glu Gly His Leu Trp Ser Ala Ser Ser
20 25 30
Lys Lys Ala Gly Ser Gly Arg Asp Lys Arg His Gln His Glu Tyr Ala
35 40 45
Asp Asp Asp Phe Glu Ala Ala Phe Glu Asp Phe Asp Asp Asp Phe Asp
50 55 60
Val His Glu Asp Asp Glu Asp Gly His Phe Val Phe Ser Ser Lys Ser
65 70 75 80
Ala Leu Ser Pro Ala Leu His Asp Gly Arg Ala Ala Ser Gln Lys Lys
85 90 95
Gln Arg Gly Arg Gln Phe Arg Gly Ile Arg Gln Arg Pro Trp Gly Lys
100 105 110
Trp Ala Ala Glu Ile Arg Asp Pro His Lys Gly Thr Arg Val Trp Leu
115 120 125
Gly Thr Phe Ser Thr Ala Glu Asp Ala Ala Arg Ala Tyr Asp Val Glu
130 135 140
Ala Arg Arg Leu Arg Gly Ser Lys Ala Lys Val Asn Phe Pro Ala Ala
145 150 155 160
Ser Gly Arg Ala Arg Gly Arg Ala Arg Pro Arg Arg Gly Asp Asp Gly
165 170 175
Asn Pro Arg Thr Ala Pro Glu Thr Gln His Pro Ala Gln Pro Ala Leu
180 185 190
Leu Pro Arg Gly Glu Arg Glu Thr Gln Arg Lys Glu Gly Ile Ala Ala
195 200 205
Val Lys Pro Glu Ala Thr Glu Ser Phe Asp Val Gly Gly Gly Leu Phe
210 215 220
Phe Asp Met Ala Phe Pro Thr Phe Pro Ala Ser Pro Pro Pro Gln Ala
225 230 235 240
Val Asp Thr Ser Phe Ala Gly Ser Thr Ala Thr Ser Glu Thr Gly Ser
245 250 255
Pro Ala Lys Arg Pro Arg Cys Asp Glu Asp Ser Ser Glu Gly Gly Ser
260 265 270
Gly Ser Ala Leu Glu Leu Ala Asp Glu Leu Ala Phe Asp Pro Phe Val
275 280 285
Leu Leu Gln Met Pro Tyr Ser Gly Gly Tyr Asp Asp Asp Ser Leu Asp
290 295 300
Gly Leu Phe Ala Ala Asp Glu Ala Val Gln Gln Asp Val Gly Asn Gly
305 310 315 320
Met Asp Gly Val Arg Leu Trp Ser Phe Asp Glu Phe Pro Ala Val Asp
325 330 335
Gly Ser Val Phe
340

<210> 16
<211> 942
<212> DNA
<213> Hordeum vulgare var. distichum

<220>
<221> CDS
<222> (1)..(942)

<400> 16
atg tgc ggc ggc gcc atc cta gcg gag ctg atc tcg ccg tcg gcg ggc 48
Met Cys Gly Gly Ala Ile Leu Ala Glu Leu Ile Ser Pro Ser Ala Gly
1 5 10 15
cgt gcg ccg aag cag gcg cag gtg gca gca gcc ggg ccg gtc tcg gcc 96
Arg Ala Pro Lys Gln Ala Gln Val Ala Ala Ala Gly Pro Val Ser Ala
20 25 30

```
aac aag gac ggc aag agc aag ggg cac aag cac aac aag tac ggc agc        144
Asn Lys Asp Gly Lys Ser Lys Gly His Lys His Asn Lys Tyr Gly Ser
            35                  40                  45
gtt gcc gat gtc gac gac gac ctc cag gac ttc gac gac gac ctc gac        192
Val Ala Asp Val Asp Asp Asp Leu Gln Asp Phe Asp Asp Asp Leu Asp
        50                  55                  60
ctg cgg cag gcg gag gag gac gcc gac gac cac gtc gtt ttc gcg tgc        240
Leu Arg Gln Ala Glu Glu Asp Ala Asp Asp His Val Val Phe Ala Cys
65                  70                  75                  80
aag cct gcc ttc tct ccg ggt ccg gcc tac gac ggt ggc cgc gcg gca        288
Lys Pro Ala Phe Ser Pro Gly Pro Ala Tyr Asp Gly Gly Arg Ala Ala
                85                  90                  95
cag gcg gcg agc agg aag aag aag cgc gtc ctc cac ggc atc cgg cag        336
Gln Ala Ala Ser Arg Lys Lys Lys Arg Val Leu His Gly Ile Arg Gln
            100                 105                 110
cgt ccg tgg ggc aag tgg gcg gcg gag atc cgc gac ccg cac aag ggc        384
Arg Pro Trp Gly Lys Trp Ala Ala Glu Ile Arg Asp Pro His Lys Gly
        115                 120                 125
acc cgc gtc tgg ctc ggc acg ttc gac acg gcc gat gat gcc gcc cgg        432
Thr Arg Val Trp Leu Gly Thr Phe Asp Thr Ala Asp Asp Ala Ala Arg
    130                 135                 140
gcc tac gac gtc gcc gcc cgt cgc ctc cgc ggc agc aag gcc aag gtc        480
Ala Tyr Asp Val Ala Ala Arg Arg Leu Arg Gly Ser Lys Ala Lys Val
145                 150                 155                 160
aac ttc ccc gac gcg gcc agg gcc ggg gcg cgc ccg cgc cgc gcc agc        528
Asn Phe Pro Asp Ala Ala Arg Ala Gly Ala Arg Pro Arg Arg Ala Ser
                165                 170                 175
cgt aga acc gcg cag aaa ccg caa cgc ccc cct gcc tgg acg acg gcg        576
Arg Arg Thr Ala Gln Lys Pro Gln Arg Pro Pro Ala Trp Thr Thr Ala
            180                 185                 190
tac tct gcc aca gca gcc gca cac tca cag ccg gag cag gac gcc ttc        624
Tyr Ser Ala Thr Ala Ala Ala His Ser Gln Pro Glu Gln Asp Ala Phe
        195                 200                 205
gtc gac ctg acc act gcc gtc acc gcg cta ccg cct atc atg gaa agc        672
Val Asp Leu Thr Thr Ala Val Thr Ala Leu Pro Pro Ile Met Glu Ser
    210                 215                 220
tcc ttc gcc gac agc ggc tcg acg aag ccg atg ttc cac gag gac tct        720
Ser Phe Ala Asp Ser Gly Ser Thr Lys Pro Met Phe His Glu Asp Ser
225                 230                 235                 240
tca gcg ggg agc ggc ggc ggt gcc atg ccg ggg ttc acc gac gag ctt        768
Ser Ala Gly Ser Gly Gly Gly Ala Met Pro Gly Phe Thr Asp Glu Leu
                245                 250                 255
ggg ttc gat cca ttc atg ctg ttc cag ctc ccc tgc tcg gac acg tat        816
Gly Phe Asp Pro Phe Met Leu Phe Gln Leu Pro Cys Ser Asp Thr Tyr
            260                 265                 270
gaa tcc atc gac agc ctc ttc gcc gga gac gct gcc ctc cag gat gcc        864
Glu Ser Ile Asp Ser Leu Phe Ala Gly Asp Ala Ala Leu Gln Asp Ala
        275                 280                 285
cgc ggt gtg gac agc ggc atc gac ggc gtc agc ctc tgg agc ttc gac        912
Arg Gly Val Asp Ser Gly Ile Asp Gly Val Ser Leu Trp Ser Phe Asp
    290                 295                 300
gaa ttc ccc atg gac agc gcc att ttc tga                                942
Glu Phe Pro Met Asp Ser Ala Ile Phe
305                 310
```

<210> 17
<211> 313
<212> PRT
<213> Hordeum vulgare var. distichum

<400> 17

```
Met Cys Gly Gly Ala Ile Leu Ala Glu Leu Ile Ser Pro Ser Ala Gly
1               5                   10                  15
```

```
Arg Ala Pro Lys Gln Ala Gln Val Ala Ala Ala Gly Pro Val Ser Ala
                20                  25                  30
Asn Lys Asp Gly Lys Ser Lys Gly His Lys His Asn Lys Tyr Gly Ser
            35                  40                  45
Val Ala Asp Val Asp Asp Asp Leu Gln Asp Phe Asp Asp Asp Leu Asp
    50                  55                  60
Leu Arg Gln Ala Glu Glu Asp Ala Asp Asp His Val Val Phe Ala Cys
65                  70                  75                  80
Lys Pro Ala Phe Ser Pro Gly Pro Ala Tyr Asp Gly Gly Arg Ala Ala
                85                  90                  95
Gln Ala Ala Ser Arg Lys Lys Lys Arg Val Leu His Gly Ile Arg Gln
            100                 105                 110
Arg Pro Trp Gly Lys Trp Ala Ala Glu Ile Arg Asp Pro His Lys Gly
        115                 120                 125
Thr Arg Val Trp Leu Gly Thr Phe Asp Thr Ala Asp Asp Ala Ala Arg
    130                 135                 140
Ala Tyr Asp Val Ala Ala Arg Arg Leu Arg Gly Ser Lys Ala Lys Val
145                 150                 155                 160
Asn Phe Pro Asp Ala Ala Arg Ala Gly Ala Arg Pro Arg Arg Ala Ser
                165                 170                 175
Arg Arg Thr Ala Gln Lys Pro Gln Arg Pro Pro Ala Trp Thr Thr Ala
            180                 185                 190
Tyr Ser Ala Thr Ala Ala Ala His Ser Gln Pro Glu Gln Asp Ala Phe
        195                 200                 205
Val Asp Leu Thr Thr Ala Val Thr Ala Leu Pro Pro Ile Met Glu Ser
    210                 215                 220
Ser Phe Ala Asp Ser Gly Ser Thr Lys Pro Met Phe His Glu Asp Ser
225                 230                 235                 240
Ser Ala Gly Ser Gly Gly Gly Ala Met Pro Gly Phe Thr Asp Glu Leu
                245                 250                 255
Gly Phe Asp Pro Phe Met Leu Phe Gln Leu Pro Cys Ser Asp Thr Tyr
            260                 265                 270
Glu Ser Ile Asp Ser Leu Phe Ala Gly Asp Ala Ala Leu Gln Asp Ala
        275                 280                 285
Arg Gly Val Asp Ser Gly Ile Asp Gly Val Ser Leu Trp Ser Phe Asp
    290                 295                 300
Glu Phe Pro Met Asp Ser Ala Ile Phe
305                 310
```

<210> 18
<211> 987
<212> DNA
<213> Hordeum vulgare

<220>
<221> CDS
<222> (1)..(987)

<400> 18

```
atg tgt ggc ggc gcc atc cta gcg cag ctg atc ccg ccg tcg gcg ggc      48
Met Cys Gly Gly Ala Ile Leu Ala Gln Leu Ile Pro Pro Ser Ala Gly
  1               5                  10                  15
cgt ccg tcg aag cag gcg gca gcg ggc ggc cgg gcc ccg ccc acg agc      96
Arg Pro Ser Lys Gln Ala Ala Ala Gly Gly Arg Ala Pro Pro Thr Ser
                20                  25                  30
tcc aag aag ggc ggc gtg agc aag agc cgc cac agc agc acc cca gat     144
Ser Lys Lys Gly Gly Val Ser Lys Ser Arg His Ser Ser Thr Pro Asp
            35                  40                  45
gcc gac gac gac gtc ttc gag gcc gcc ttc gag gac ttc gat gac cac     192
Ala Asp Asp Asp Val Phe Glu Ala Ala Phe Glu Asp Phe Asp Asp His
    50                  55                  60
ttc gac ctg cgg gcg gag gag gac ggc ggc gac gac cat gtc gtc ttt     240
Phe Asp Leu Arg Ala Glu Glu Asp Gly Gly Asp Asp His Val Val Phe
```

```
65                  70                  75                  80
gca tcc aag cct gcc ttc tct cca cgt ccg gcc tac gac ggt ggc cgc         288
Ala Ser Lys Pro Ala Phe Ser Pro Arg Pro Ala Tyr Asp Gly Gly Arg
                85                  90                  95
gcg gcg cat gcg gcg agc agg aag aag cgc acc ggc cac ctc cat ggc         336
Ala Ala His Ala Ala Ser Arg Lys Lys Arg Thr Gly His Leu His Gly
            100                 105                 110
atc cgg cag cgg ccg tgg ggc aag tgg gcg gcg gag atc cgc gac ccg         384
Ile Arg Gln Arg Pro Trp Gly Lys Trp Ala Ala Glu Ile Arg Asp Pro
        115                 120                 125
cac aag ggc acc cgc gtc tgg ctc ggc acg ttc gac acg gcc gat gat         432
His Lys Gly Thr Arg Val Trp Leu Gly Thr Phe Asp Thr Ala Asp Asp
    130                 135                 140
gcc gcc cgg gcc tac gac gtc gcc gcc cgt cgc ctc cgt ggc agc aag         480
Ala Ala Arg Ala Tyr Asp Val Ala Ala Arg Arg Leu Arg Gly Ser Lys
145                 150                 155                 160
gcc aag gtc aac ttc ccc gac gcg gcc agg acc ggg gct cgc ccg cgc         528
Ala Lys Val Asn Phe Pro Asp Ala Ala Arg Thr Gly Ala Arg Pro Arg
                165                 170                 175
cgc gcc agc cgt aga acc gcg cag aaa ccg caa tgc ccc cct gcg cgg         576
Arg Ala Ser Arg Arg Thr Ala Gln Lys Pro Gln Cys Pro Pro Ala Arg
            180                 185                 190
acg acg gcg tac tct gcc acc gca gca gca cgc gca cag ccg gag cag         624
Thr Thr Ala Tyr Ser Ala Thr Ala Ala Ala Arg Ala Gln Pro Glu Gln
        195                 200                 205
gac gct atg atg gtc aaa ccc gag ctg atg gag ttt ttc aac gtg gac         672
Asp Ala Met Met Val Lys Pro Glu Leu Met Glu Phe Phe Asn Val Asp
    210                 215                 220
gcc atc gtc cac ctg acc act gcc gtc gcc gcg cta ccg cct gtc acg         720
Ala Ile Val His Leu Thr Thr Ala Val Ala Ala Leu Pro Pro Val Thr
225                 230                 235                 240
gcg agc acc ttc gcc gac acg atg ccg agg gtc gac gag gac tct tct         768
Ala Ser Thr Phe Ala Asp Thr Met Pro Arg Val Asp Glu Asp Ser Ser
                245                 250                 255
gtg ggg agc ggc ggc ggc gcc atg ctg ggg ttc gcc gac gag ctt ggg         816
Val Gly Ser Gly Gly Gly Ala Met Leu Gly Phe Ala Asp Glu Leu Gly
            260                 265                 270
ttc gat ccg ttc atg atg ttc cag cta ccc tgc tcg gac atg tac gaa         864
Phe Asp Pro Phe Met Met Phe Gln Leu Pro Cys Ser Asp Met Tyr Glu
        275                 280                 285
tcc gcc gac agc atc ttc gcc gga gac gct gtc atc ccg gat gcc ctc         912
Ser Ala Asp Ser Ile Phe Ala Gly Asp Ala Val Ile Pro Asp Ala Leu
    290                 295                 300
agc gtg gac agt ggc atg gac gcc gtc agc ctc tgg agc ttc gac gag         960
Ser Val Asp Ser Gly Met Asp Ala Val Ser Leu Trp Ser Phe Asp Glu
305                 310                 315                 320
ttc ccc atg gac agc gcc att ttc tga                                     987
Phe Pro Met Asp Ser Ala Ile Phe
                325
```

<210> 19
<211> 328
<212> PRT
<213> Hordeum vulgare

<400> 19

```
Met Cys Gly Gly Ala Ile Leu Ala Gln Leu Ile Pro Pro Ser Ala Gly
1               5                   10                  15
Arg Pro Ser Lys Gln Ala Ala Ala Gly Gly Arg Ala Pro Pro Thr Ser
            20                  25                  30
Ser Lys Lys Gly Gly Val Ser Lys Ser Arg His Ser Ser Thr Pro Asp
        35                  40                  45
Ala Asp Asp Asp Val Phe Glu Ala Ala Phe Glu Asp Phe Asp Asp His
```

```
    50                  55                  60
Phe Asp Leu Arg Ala Glu Glu Asp Gly Gly Asp Asp His Val Val Phe
65                  70                  75                  80
Ala Ser Lys Pro Ala Phe Ser Pro Arg Pro Ala Tyr Asp Gly Gly Arg
                85                  90                  95
Ala Ala His Ala Ala Ser Arg Lys Lys Arg Thr Gly His Leu His Gly
            100                 105                 110
Ile Arg Gln Arg Pro Trp Gly Lys Trp Ala Ala Glu Ile Arg Asp Pro
        115                 120                 125
His Lys Gly Thr Arg Val Trp Leu Gly Thr Phe Asp Thr Ala Asp Asp
    130                 135                 140
Ala Ala Arg Ala Tyr Asp Val Ala Ala Arg Arg Leu Arg Gly Ser Lys
145                 150                 155                 160
Ala Lys Val Asn Phe Pro Asp Ala Ala Arg Thr Gly Ala Arg Pro Arg
                165                 170                 175
Arg Ala Ser Arg Arg Thr Ala Gln Lys Pro Gln Cys Pro Pro Ala Arg
            180                 185                 190
Thr Thr Ala Tyr Ser Ala Thr Ala Ala Ala Arg Ala Gln Pro Glu Gln
        195                 200                 205
Asp Ala Met Met Val Lys Pro Glu Leu Met Glu Phe Phe Asn Val Asp
    210                 215                 220
Ala Ile Val His Leu Thr Thr Ala Val Ala Ala Leu Pro Pro Val Thr
225                 230                 235                 240
Ala Ser Thr Phe Ala Asp Thr Met Pro Arg Val Asp Glu Asp Ser Ser
                245                 250                 255
Val Gly Ser Gly Gly Gly Ala Met Leu Gly Phe Ala Asp Glu Leu Gly
            260                 265                 270
Phe Asp Pro Phe Met Met Phe Gln Leu Pro Cys Ser Asp Met Tyr Glu
        275                 280                 285
Ser Ala Asp Ser Ile Phe Ala Gly Asp Ala Val Ile Pro Asp Ala Leu
    290                 295                 300
Ser Val Asp Ser Gly Met Asp Ala Val Ser Leu Trp Ser Phe Asp Glu
305                 310                 315                 320
Phe Pro Met Asp Ser Ala Ile Phe
                325
```

<210> 20
<211> 987
<212> DNA
<213> Hordeum vulgare

<220>
<221> CDS
<222> (1)..(987)

<400> 20

```
atg tgt ggc ggc gcc atc cta gcg cag ctg atc ccg ccg tcg gcg ggc       48
Met Cys Gly Gly Ala Ile Leu Ala Gln Leu Ile Pro Pro Ser Ala Gly
  1               5                  10                  15
cgt ccg tcg aag cag gcg gca gcg ggc ggc cgg gcc gcg ccc acg agc       96
Arg Pro Ser Lys Gln Ala Ala Ala Gly Gly Arg Ala Ala Pro Thr Ser
             20                  25                  30
tcc aag aag ggc ggc gtg agc aag agc cgc cac agc agc acc cca gat      144
Ser Lys Lys Gly Gly Val Ser Lys Ser Arg His Ser Ser Thr Pro Asp
         35                  40                  45
gcc gac gac gac gtc tcg tct gcc gcc ttc gag gac ttc gat gac cac      192
Ala Asp Asp Asp Val Ser Ser Ala Ala Phe Glu Asp Phe Asp Asp His
     50                  55                  60
ttc gac ctg cgg gcg gag gag gac ggc ggc gac gac cat gtc gtc ttt      240
Phe Asp Leu Arg Ala Glu Glu Asp Gly Gly Asp Asp His Val Val Phe
 65                  70                  75                  80
gca tcc aag cct gcc ttc tct cca cgt ccg gcc tac gac ggt ggc cgc      288
Ala Ser Lys Pro Ala Phe Ser Pro Arg Pro Ala Tyr Asp Gly Gly Arg
```

```
                    85                      90                      95
gcg gcg cat gcg gcg agc agg aag aag cgc acc ggc cac ctc cat ggc        336
Ala Ala His Ala Ala Ser Arg Lys Lys Arg Thr Gly His Leu His Gly
                100                     105                     110
atc cgg cag cgg ccg tgg ggc aag tgg gcg gcg gag atc cgc gac ccg        384
Ile Arg Gln Arg Pro Trp Gly Lys Trp Ala Ala Glu Ile Arg Asp Pro
            115                     120                     125
cac aag ggc acc cgc gtc tgg ctc ggc acg ttc gac acg gcc gat gat        432
His Lys Gly Thr Arg Val Trp Leu Gly Thr Phe Asp Thr Ala Asp Asp
        130                     135                     140
gcc gcc cgg gcc tac gac gtc gcc gcc cgt cgc ctc cgt ggc agc aag        480
Ala Ala Arg Ala Tyr Asp Val Ala Ala Arg Arg Leu Arg Gly Ser Lys
145                     150                     155                     160
gcc aag gtc aac ttc ccc gac gcg gcc agg acc ggg gct cgc ccg cgc        528
Ala Lys Val Asn Phe Pro Asp Ala Ala Arg Thr Gly Ala Arg Pro Arg
                165                     170                     175
cgc gcc agc cgt aga acc gcg cag aaa ccg caa tgc ccc cct gcg cgg        576
Arg Ala Ser Arg Arg Thr Ala Gln Lys Pro Gln Cys Pro Pro Ala Arg
                180                     185                     190
acg acg gcg tac tct gcc acc gca gca gca cgc gca cag ccg gag cag        624
Thr Thr Ala Tyr Ser Ala Thr Ala Ala Ala Arg Ala Gln Pro Glu Gln
            195                     200                     205
gac gct atg atg gtc aaa ccc gag ctg atg gag ttt ttc aac gtg gac        672
Asp Ala Met Met Val Lys Pro Glu Leu Met Glu Phe Phe Asn Val Asp
        210                     215                     220
gcc atc gtc cac ctg acc agt gcc gtc gcc gcg cta ccg cct gtc acg        720
Ala Ile Val His Leu Thr Ser Ala Val Ala Ala Leu Pro Pro Val Thr
225                     230                     235                     240
gcg agc acc ttc gcc gac acg atg ccg agg gtc gac gag gac tct tct        768
Ala Ser Thr Phe Ala Asp Thr Met Pro Arg Val Asp Glu Asp Ser Ser
                    245                     250                     255
gtg ggg agc ggc ggc ggc gcc atg ctg ggg ttc gcc gac gag ctt ggg        816
Val Gly Ser Gly Gly Gly Ala Met Leu Gly Phe Ala Asp Glu Leu Gly
                260                     265                     270
ttc gat ccg ttc atg atg ttc ctg cta ccc tgc tcg gac atg tac gaa        864
Phe Asp Pro Phe Met Met Phe Leu Leu Pro Cys Ser Asp Met Tyr Glu
            275                     280                     285
tcc gcc gac agc atc ttc gcc gga gac gct gtc atc ccg gat gcc ctc        912
Ser Ala Asp Ser Ile Phe Ala Gly Asp Ala Val Ile Pro Asp Ala Leu
        290                     295                     300
agc gtg gac agt ggc atg gac gcc gtc agc ctc tgg agc gct gac gag        960
Ser Val Asp Ser Gly Met Asp Ala Val Ser Leu Trp Ser Ala Asp Glu
305                     310                     315                     320
ttc ccc atg gac agc gcc att ttc tga                                    987
Phe Pro Met Asp Ser Ala Ile Phe
                    325

<210> 21
<211> 328
<212> PRT
<213> Hordeum vulgare

<400> 21
Met Cys Gly Gly Ala Ile Leu Ala Gln Leu Ile Pro Pro Ser Ala Gly
1               5                   10                  15
Arg Pro Ser Lys Gln Ala Ala Ala Gly Gly Arg Ala Ala Pro Thr Ser
            20                  25                  30
Ser Lys Lys Gly Gly Val Ser Lys Ser Arg His Ser Ser Thr Pro Asp
        35                  40                  45
Ala Asp Asp Asp Val Ser Ser Ala Ala Phe Glu Asp Phe Asp Asp His
    50                  55                  60
Phe Asp Leu Arg Ala Glu Glu Asp Gly Gly Asp Asp His Val Val Phe
65                  70                  75                  80
```

```
Ala Ser Lys Pro Ala Phe Ser Pro Arg Pro Ala Tyr Asp Gly Gly Arg
                85                  90                  95
Ala Ala His Ala Ala Ser Arg Lys Lys Arg Thr Gly His Leu His Gly
            100                 105                 110
Ile Arg Gln Arg Pro Trp Gly Lys Trp Ala Ala Glu Ile Arg Asp Pro
        115                 120                 125
His Lys Gly Thr Arg Val Trp Leu Gly Thr Phe Asp Thr Ala Asp Asp
    130                 135                 140
Ala Ala Arg Ala Tyr Asp Val Ala Ala Arg Arg Leu Arg Gly Ser Lys
145                 150                 155                 160
Ala Lys Val Asn Phe Pro Asp Ala Ala Arg Thr Gly Ala Arg Pro Arg
                165                 170                 175
Arg Ala Ser Arg Arg Thr Ala Gln Lys Pro Gln Cys Pro Pro Ala Arg
            180                 185                 190
Thr Thr Ala Tyr Ser Ala Thr Ala Ala Ala Arg Ala Gln Pro Glu Gln
        195                 200                 205
Asp Ala Met Met Val Lys Pro Glu Leu Met Glu Phe Phe Asn Val Asp
    210                 215                 220
Ala Ile Val His Leu Thr Ser Ala Val Ala Ala Leu Pro Pro Val Thr
225                 230                 235                 240
Ala Ser Thr Phe Ala Asp Thr Met Pro Arg Val Asp Glu Asp Ser Ser
                245                 250                 255
Val Gly Ser Gly Gly Gly Ala Met Leu Gly Phe Ala Asp Glu Leu Gly
            260                 265                 270
Phe Asp Pro Phe Met Met Phe Leu Leu Pro Cys Ser Asp Met Tyr Glu
        275                 280                 285
Ser Ala Asp Ser Ile Phe Ala Gly Asp Ala Val Ile Pro Asp Ala Leu
    290                 295                 300
Ser Val Asp Ser Gly Met Asp Ala Val Ser Leu Trp Ser Ala Asp Glu
305                 310                 315                 320
Phe Pro Met Asp Ser Ala Ile Phe
                325

<210> 22
<211> 951
<212> DNA
<213> Triticum aestivum

<220>
<221> CDS
<222> (1)..(951)

<400> 22
atg tgc ggc ggc gcc atc cta gcg aag ctg atc ccg ccg acg ccg ccg       48
Met Cys Gly Gly Ala Ile Leu Ala Lys Leu Ile Pro Pro Thr Pro Pro
 1               5                  10                  15
tcg gcg ggc cgt gcc ccg aag cag gtg gcc gcg ggc ggg gtc tcg ccc       96
Ser Ala Gly Arg Ala Pro Lys Gln Val Ala Ala Gly Gly Val Ser Pro
            20                  25                  30
aag aag ggc ggc atg aac aag acg cac cac agc agc acc ccc gat gtc      144
Lys Lys Gly Gly Met Asn Lys Thr His His Ser Ser Thr Pro Asp Val
        35                  40                  45
gac gac ttc gag gcc gcc ttc gag gac ttc gat gac gac ttc cac ctg      192
Asp Asp Phe Glu Ala Ala Phe Glu Asp Phe Asp Asp Asp Phe His Leu
    50                  55                  60
cag gcg gag gag gac ggc gac gac cat gtc gtt ttt gca tcc aaa cct      240
Gln Ala Glu Glu Asp Gly Asp Asp His Val Val Phe Ala Ser Lys Pro
65                  70                  75                  80
gcc ttc tcc ccg gcc tac gat gat ggc cgc gcg gcg cag gcg gcg agc      288
Ala Phe Ser Pro Ala Tyr Asp Asp Gly Arg Ala Ala Gln Ala Ala Ser
                85                  90                  95
agg aag aag agc gtc cgc cgc ctc cac ggc atc cgt cag cgg ccg tgg      336
Arg Lys Lys Ser Val Arg Arg Leu His Gly Ile Arg Gln Arg Pro Trp
```

```
                100                 105                 110
ggc aag tgg gcg gcg gag atc cgc gac ccg cac aag ggc acc cgc gtc         384
Gly Lys Trp Ala Ala Glu Ile Arg Asp Pro His Lys Gly Thr Arg Val
            115                 120                 125
tgg ctc ggc acg ttc gac acg gcc gat gat gcc gcc cgg gcc tac gac         432
Trp Leu Gly Thr Phe Asp Thr Ala Asp Asp Ala Ala Arg Ala Tyr Asp
        130                 135                 140
gtc gcc gcc cac cgc ctc cgt ggc agc aag gcc aaa gtc aac ttc ccc         480
Val Ala Ala His Arg Leu Arg Gly Ser Lys Ala Lys Val Asn Phe Pro
145                 150                 155                 160
aac ggg acc agg gct ggg gcg cgc ctg caa cgt gcc agc cgg aga acc         528
Asn Gly Thr Arg Ala Gly Ala Arg Leu Gln Arg Ala Ser Arg Arg Thr
                165                 170                 175
gct tcg aaa cgg cag tgc ccc cct gcg cgg acg acg gcg tac tct gct         576
Ala Ser Lys Arg Gln Cys Pro Pro Ala Arg Thr Thr Ala Tyr Ser Ala
                180                 185                 190
gca cac gca cag aag gag cgg gac gct atg gtg gcc aag cct gag ctg         624
Ala His Ala Gln Lys Glu Arg Asp Ala Met Val Ala Lys Pro Glu Leu
            195                 200                 205
atg gag tct ttc gac atg gac gcc ttc gtc gac ctg acc act gct ttc         672
Met Glu Ser Phe Asp Met Asp Ala Phe Val Asp Leu Thr Thr Ala Phe
        210                 215                 220
acc acg cta ccg cct gtc atg gca agc tcc ttc gcc gac act ggc gcg         720
Thr Thr Leu Pro Pro Val Met Ala Ser Ser Phe Ala Asp Thr Gly Ala
225                 230                 235                 240
aag aag ccg atg gtc gat gag gat tcg tcg gat ggg agc ggc ggc gat         768
Lys Lys Pro Met Val Asp Glu Asp Ser Ser Asp Gly Ser Gly Gly Asp
                245                 250                 255
gcc atg ctg ggg ttc gat ccg ttc atg ctg ttc cag ctc ccc tgc tcg         816
Ala Met Leu Gly Phe Asp Pro Phe Met Leu Phe Gln Leu Pro Cys Ser
                260                 265                 270
gac acg tac gaa tcc atc gac agc ctc ttc gcc ggc gac gcc gtc atc         864
Asp Thr Tyr Glu Ser Ile Asp Ser Leu Phe Ala Gly Asp Ala Val Ile
            275                 280                 285
cag gat gcc ctc ggc gtg gac agt ggc atg gag ggc gtc agc ctc tgg         912
Gln Asp Ala Leu Gly Val Asp Ser Gly Met Glu Gly Val Ser Leu Trp
        290                 295                 300
agc ttc gag gag ttc ccc atg gac agc gcc att ttt tga                     951
Ser Phe Glu Glu Phe Pro Met Asp Ser Ala Ile Phe
305                 310                 315
```

<210> 23
<211> 316
<212> PRT
<213> Triticum aestivum

<400> 23

```
Met Cys Gly Gly Ala Ile Leu Ala Lys Leu Ile Pro Pro Thr Pro Pro
1               5                   10                  15
Ser Ala Gly Arg Ala Pro Lys Gln Val Ala Ala Gly Gly Val Ser Pro
            20                  25                  30
Lys Lys Gly Gly Met Asn Lys Thr His His Ser Ser Thr Pro Asp Val
        35                  40                  45
Asp Asp Phe Glu Ala Ala Phe Glu Asp Phe Asp Asp Asp Phe His Leu
    50                  55                  60
Gln Ala Glu Glu Asp Gly Asp Asp His Val Val Phe Ala Ser Lys Pro
65                  70                  75                  80
Ala Phe Ser Pro Ala Tyr Asp Asp Gly Arg Ala Ala Gln Ala Ala Ser
                85                  90                  95
Arg Lys Lys Ser Val Arg Arg Leu His Gly Ile Arg Gln Arg Pro Trp
            100                 105                 110
Gly Lys Trp Ala Ala Glu Ile Arg Asp Pro His Lys Gly Thr Arg Val
        115                 120                 125
```

```
Trp Leu Gly Thr Phe Asp Thr Ala Asp Asp Ala Ala Arg Ala Tyr Asp
    130                 135                 140
Val Ala Ala His Arg Leu Arg Gly Ser Lys Ala Lys Val Asn Phe Pro
145                 150                 155                 160
Asn Gly Thr Arg Ala Gly Ala Arg Leu Gln Arg Ala Ser Arg Arg Thr
                165                 170                 175
Ala Ser Lys Arg Gln Cys Pro Pro Ala Arg Thr Thr Ala Tyr Ser Ala
            180                 185                 190
Ala His Ala Gln Lys Glu Arg Asp Ala Met Val Ala Lys Pro Glu Leu
        195                 200                 205
Met Glu Ser Phe Asp Met Asp Ala Phe Val Asp Leu Thr Thr Ala Phe
    210                 215                 220
Thr Thr Leu Pro Pro Val Met Ala Ser Ser Phe Ala Asp Thr Gly Ala
225                 230                 235                 240
Lys Lys Pro Met Val Asp Glu Asp Ser Ser Asp Gly Ser Gly Gly Asp
                245                 250                 255
Ala Met Leu Gly Phe Asp Pro Phe Met Leu Phe Gln Leu Pro Cys Ser
            260                 265                 270
Asp Thr Tyr Glu Ser Ile Asp Ser Leu Phe Ala Gly Asp Ala Val Ile
        275                 280                 285
Gln Asp Ala Leu Gly Val Asp Ser Gly Met Glu Gly Val Ser Leu Trp
    290                 295                 300
Ser Phe Glu Glu Phe Pro Met Asp Ser Ala Ile Phe
305                 310                 315

<210> 24
<211> 1038
<212> DNA
<213> Zea mays

<220>
<221> CDS
<222> (1)..(1038)

<400> 24
atg tgc gga ggc gcc atc ctc gcg gag ctg atc ccg ccg acg cgg cgc      48
Met Cys Gly Gly Ala Ile Leu Ala Glu Leu Ile Pro Pro Thr Arg Arg
 1               5                  10                  15
gtg gcg tcg aag ccg gtg aca gaa ggc cac ctc tgg tcg gcg agc tcc      96
Val Ala Ser Lys Pro Val Thr Glu Gly His Leu Trp Ser Ala Ser Ser
                20                  25                  30
aag aaa gcc ggc agc ggc agg gac aag agg cac cag cac gaa tac gcc      144
Lys Lys Ala Gly Ser Gly Arg Asp Lys Arg His Gln His Glu Tyr Ala
            35                  40                  45
gac gat gac ttc gag gcc gcc ttc gag gac ttc gac gac gac ttt gac      192
Asp Asp Asp Phe Glu Ala Ala Phe Glu Asp Phe Asp Asp Asp Phe Asp
    50                  55                  60
gtg cat gaa gac gac gag gac ggc cac ctc gta ttc tcg tcc aaa tcc      240
Val His Glu Asp Asp Glu Asp Gly His Leu Val Phe Ser Ser Lys Ser
65                  70                  75                  80
gcc ttc tcc cca gcc ctg cac gac ggg cgc gcg gcg agc cag aag aag      288
Ala Phe Ser Pro Ala Leu His Asp Gly Arg Ala Ala Ser Gln Lys Lys
                85                  90                  95
cag cgc ggg cgc cag ttc cga ggc atc cgg cag cgg ccc tgg ggc aag      336
Gln Arg Gly Arg Gln Phe Arg Gly Ile Arg Gln Arg Pro Trp Gly Lys
            100                 105                 110
tgg gcg gcg gag atc cgc gac ccg cac aag ggc acc cgc gtc tgg ctc      384
Trp Ala Ala Glu Ile Arg Asp Pro His Lys Gly Thr Arg Val Trp Leu
        115                 120                 125
ggc acc ttc agc acc gcc gag gac gcc gcc cgg gcc tac gac gtg gag      432
Gly Thr Phe Ser Thr Ala Glu Asp Ala Ala Arg Ala Tyr Asp Val Glu
    130                 135                 140
gcg cgc cgc ctc cgc ggc agc aag gcc aag gtc aac ttc ccc gca gcc      480
```

```
Ala Arg Arg Leu Arg Gly Ser Lys Ala Lys Val Asn Phe Pro Ala Ala
145                 150                 155                 160
agc ggt cgc gct cgc gcg cgc cca cgc cgc ggc gac gac ggc aac cca      528
Ser Gly Arg Ala Arg Ala Arg Pro Arg Arg Gly Asp Asp Gly Asn Pro
                165                 170                 175
cga acc gcg ccg gaa ccg cag cac cct gct gct gct gct gct gct gca      576
Arg Thr Ala Pro Glu Pro Gln His Pro Ala Ala Ala Ala Ala Ala Ala
            180                 185                 190
gca cag ccc gct ctg ctg cct cga ggg gag aga gtc aga gag acg cag      624
Ala Gln Pro Ala Leu Leu Pro Arg Gly Glu Arg Val Arg Glu Thr Gln
        195                 200                 205
agg aag gaa ggg aac gcc gcc gtg aag cct gaa gct acg cag tcg ttc      672
Arg Lys Glu Gly Asn Ala Ala Val Lys Pro Glu Ala Thr Gln Ser Phe
    210                 215                 220
gac gtg ggc ggt ctc ttc ttc gac atg gcc ttc ccc acc ttc cca gcc      720
Asp Val Gly Gly Leu Phe Phe Asp Met Ala Phe Pro Thr Phe Pro Ala
225                 230                 235                 240
tcg ccg ccg ccg cag gcc gtg gag gcg tcc ttc acc ggc agc acc gcc      768
Ser Pro Pro Pro Gln Ala Val Glu Ala Ser Phe Thr Gly Ser Thr Ala
                245                 250                 255
acg tcg gag acc ggg agc ccc gcg aag agg cca aga tgc gac gac aac      816
Thr Ser Glu Thr Gly Ser Pro Ala Lys Arg Pro Arg Cys Asp Asp Asn
            260                 265                 270
tcg tcc gag ggc agc ggc ggc agc ggc tcc gcg ctg gag ctc gct gac      864
Ser Ser Glu Gly Ser Gly Gly Ser Gly Ser Ala Leu Glu Leu Ala Asp
        275                 280                 285
gag ctg gcg ttc gac ccg ttt atg ctg ctg cag atg ccc tat tct ggt      912
Glu Leu Ala Phe Asp Pro Phe Met Leu Leu Gln Met Pro Tyr Ser Gly
    290                 295                 300
ggg tac gac tca ctg gac ggc ctt ttc gcc gca gag gcc gtg cag cag      960
Gly Tyr Asp Ser Leu Asp Gly Leu Phe Ala Ala Glu Ala Val Gln Gln
305                 310                 315                 320
gac gtg ggc aac ggc atg gac ggc gtc agc ctg tgg agc ttc gac gag      1008
Asp Val Gly Asn Gly Met Asp Gly Val Ser Leu Trp Ser Phe Asp Glu
                325                 330                 335
ttc ccc gcc gtc gac ggt tgt gtt ttc tga                              1038
Phe Pro Ala Val Asp Gly Cys Val Phe
            340                 345

<210> 25
<211> 345
<212> PRT
<213> Zea mays

<400> 25
Met Cys Gly Gly Ala Ile Leu Ala Glu Leu Ile Pro Pro Thr Arg Arg
1               5                   10                  15
Val Ala Ser Lys Pro Val Thr Glu Gly His Leu Trp Ser Ala Ser Ser
            20                  25                  30
Lys Lys Ala Gly Ser Gly Arg Asp Lys Arg His Gln His Glu Tyr Ala
        35                  40                  45
Asp Asp Asp Phe Glu Ala Ala Phe Glu Asp Phe Asp Asp Asp Phe Asp
    50                  55                  60
Val His Glu Asp Asp Glu Asp Gly His Leu Val Phe Ser Ser Lys Ser
65                  70                  75                  80
Ala Phe Ser Pro Ala Leu His Asp Gly Arg Ala Ala Ser Gln Lys Lys
                85                  90                  95
Gln Arg Gly Arg Gln Phe Arg Gly Ile Arg Gln Arg Pro Trp Gly Lys
            100                 105                 110
Trp Ala Ala Glu Ile Arg Asp Pro His Lys Gly Thr Arg Val Trp Leu
        115                 120                 125
Gly Thr Phe Ser Thr Ala Glu Asp Ala Ala Arg Ala Tyr Asp Val Glu
    130                 135                 140
```

Ala Arg Arg Leu Arg Gly Ser Lys Ala Lys Val Asn Phe Pro Ala Ala
145 150 155 160
Ser Gly Arg Ala Arg Ala Arg Pro Arg Arg Gly Asp Asp Gly Asn Pro
165 170 175
Arg Thr Ala Pro Glu Pro Gln His Pro Ala Ala Ala Ala Ala Ala Ala
180 185 190
Ala Gln Pro Ala Leu Leu Pro Arg Gly Glu Arg Val Arg Glu Thr Gln
195 200 205
Arg Lys Glu Gly Asn Ala Ala Val Lys Pro Glu Ala Thr Gln Ser Phe
210 215 220
Asp Val Gly Gly Leu Phe Phe Asp Met Ala Phe Pro Thr Phe Pro Ala
225 230 235 240
Ser Pro Pro Pro Gln Ala Val Glu Ala Ser Phe Thr Gly Ser Thr Ala
245 250 255
Thr Ser Glu Thr Gly Ser Pro Ala Lys Arg Pro Arg Cys Asp Asp Asn
260 265 270
Ser Ser Glu Gly Ser Gly Gly Ser Gly Ser Ala Leu Glu Leu Ala Asp
275 280 285
Glu Leu Ala Phe Asp Pro Phe Met Leu Leu Gln Met Pro Tyr Ser Gly
290 295 300
Gly Tyr Asp Ser Leu Asp Gly Leu Phe Ala Ala Glu Ala Val Gln Gln
305 310 315 320
Asp Val Gly Asn Gly Met Asp Gly Val Ser Leu Trp Ser Phe Asp Glu
325 330 335
Phe Pro Ala Val Asp Gly Cys Val Phe
340 345

<210> 26
<211> 1023
<212> DNA
<213> Zea mays

<220>
<221> CDS
<222> (1)..(1023)

<400> 26
atg tgc gga ggc gcc atc ctc gcg gag ctg atc ccg ccg acg cgg cgc 48
Met Cys Gly Gly Ala Ile Leu Ala Glu Leu Ile Pro Pro Thr Arg Arg
1 5 10 15
gtg gcg tcg aag ccg gtg aca gaa ggc cac ctc tgg tcg gcg agc tcc 96
Val Ala Ser Lys Pro Val Thr Glu Gly His Leu Trp Ser Ala Ser Ser
20 25 30
aag aaa gcc ggc agc ggc agg gac aag agg cac cag cac gaa tac gcc 144
Lys Lys Ala Gly Ser Gly Arg Asp Lys Arg His Gln His Glu Tyr Ala
35 40 45
gac gat gac ttc gag gcc gcc ttc gag gac ttc gac gac gac ttt gac 192
Asp Asp Asp Phe Glu Ala Ala Phe Glu Asp Phe Asp Asp Asp Phe Asp
50 55 60
gtg cat gaa gac gac gag gac ggc cac ttc gta ttc tcg tcc aaa tcc 240
Val His Glu Asp Asp Glu Asp Gly His Phe Val Phe Ser Ser Lys Ser
65 70 75 80
gcc ttg tcc cca gcc ctg cac gac ggg cgc gcg gcg agc cag aag aag 288
Ala Leu Ser Pro Ala Leu His Asp Gly Arg Ala Ala Ser Gln Lys Lys
85 90 95
cag cgc ggg cgc cag ttc cgc ggc atc cgg cag cgg ccc tgg ggc aag 336
Gln Arg Gly Arg Gln Phe Arg Gly Ile Arg Gln Arg Pro Trp Gly Lys
100 105 110
tgg gcg gcg gag atc cgc gac ccg cac aag ggc acc cgc gtc tgg ctc 384
Trp Ala Ala Glu Ile Arg Asp Pro His Lys Gly Thr Arg Val Trp Leu
115 120 125
ggc acc ttc agc acc gcc gag gac gcc gcc cgg gcc tac gac gtg gag 432
Gly Thr Phe Ser Thr Ala Glu Asp Ala Ala Arg Ala Tyr Asp Val Glu

```
    130                 135                 140
gcg cgc cgc ctc cgc ggc agc aag gcc aag gtc aac ttc ccc gca gcc       480
Ala Arg Arg Leu Arg Gly Ser Lys Ala Lys Val Asn Phe Pro Ala Ala
145                 150                 155                 160
agc ggt cgc gct cgc ggt cgc gcg cgc cca cgc cgc ggc gac gac ggc       528
Ser Gly Arg Ala Arg Gly Arg Ala Arg Pro Arg Arg Gly Asp Asp Gly
                165                 170                 175
aac cca cga acc gcg ccg gaa acg cag cac cca gca cag ccc gct ctg       576
Asn Pro Arg Thr Ala Pro Glu Thr Gln His Pro Ala Gln Pro Ala Leu
            180                 185                 190
ctg cct cga gga gag aga gag acg cag agg aag gaa ggg atc gcc gcc       624
Leu Pro Arg Gly Glu Arg Glu Thr Gln Arg Lys Glu Gly Ile Ala Ala
        195                 200                 205
gtg aag cca gaa gct acg gag tcg ttc gac gtg ggc ggc ggt ctc ttc       672
Val Lys Pro Glu Ala Thr Glu Ser Phe Asp Val Gly Gly Gly Leu Phe
    210                 215                 220
ttc gac atg gcc ttc ccc acc ttc cca gcc tcg ccg ccg ccg cag gcc       720
Phe Asp Met Ala Phe Pro Thr Phe Pro Ala Ser Pro Pro Pro Gln Ala
225                 230                 235                 240
gtg gat acg tcc ttc gcc ggc agc acc gcc acg tcg gag acc ggg agc       768
Val Asp Thr Ser Phe Ala Gly Ser Thr Ala Thr Ser Glu Thr Gly Ser
                245                 250                 255
ccc gcg aag agg ccg aga tgc gac gaa gac tcg tcc gag ggc ggc agc       816
Pro Ala Lys Arg Pro Arg Cys Asp Glu Asp Ser Ser Glu Gly Gly Ser
            260                 265                 270
ggc tcc gcg ctg gag ctc gct gac gag ctg gcg ttc gac ccg ttt gtg       864
Gly Ser Ala Leu Glu Leu Ala Asp Glu Leu Ala Phe Asp Pro Phe Val
        275                 280                 285
ctg ctg cag atg ccc tac tcg ggt ggg tac gac gac gac tca ctg gac       912
Leu Leu Gln Met Pro Tyr Ser Gly Gly Tyr Asp Asp Asp Ser Leu Asp
    290                 295                 300
ggc ctt ttc tcc gca gat gag gcc gtg cag cag gac gtg ggc aac ggc       960
Gly Leu Phe Ser Ala Asp Glu Ala Val Gln Gln Asp Val Gly Asn Gly
305                 310                 315                 320
atg gac ggc gtc cgc ctg tgg agg ttt gac gag ttc ccc gcc gtc gac       1008
Met Asp Gly Val Arg Leu Trp Arg Phe Asp Glu Phe Pro Ala Val Asp
                325                 330                 335
ggt tct gtt ttt tga                                                   1023
Gly Ser Val Phe
            340
```

<210> 27
<211> 340
<212> PRT
<213> Zea mays

<400> 27

```
Met Cys Gly Gly Ala Ile Leu Ala Glu Leu Ile Pro Pro Thr Arg Arg
1               5                   10                  15
Val Ala Ser Lys Pro Val Thr Glu Gly His Leu Trp Ser Ala Ser Ser
            20                  25                  30
Lys Lys Ala Gly Ser Gly Arg Asp Lys Arg His Gln His Glu Tyr Ala
        35                  40                  45
Asp Asp Asp Phe Glu Ala Ala Phe Glu Asp Phe Asp Asp Asp Phe Asp
    50                  55                  60
Val His Glu Asp Asp Glu Asp Gly His Phe Val Phe Ser Ser Lys Ser
65                  70                  75                  80
Ala Leu Ser Pro Ala Leu His Asp Gly Arg Ala Ala Ser Gln Lys Lys
                85                  90                  95
Gln Arg Gly Arg Gln Phe Arg Gly Ile Arg Gln Arg Pro Trp Gly Lys
            100                 105                 110
Trp Ala Ala Glu Ile Arg Asp Pro His Lys Gly Thr Arg Val Trp Leu
        115                 120                 125
```

```
Gly Thr Phe Ser Thr Ala Glu Asp Ala Ala Arg Ala Tyr Asp Val Glu
    130                 135                 140
Ala Arg Arg Leu Arg Gly Ser Lys Ala Lys Val Asn Phe Pro Ala Ala
145                 150                 155                 160
Ser Gly Arg Ala Arg Gly Arg Ala Arg Pro Arg Arg Gly Asp Asp Gly
                165                 170                 175
Asn Pro Arg Thr Ala Pro Glu Thr Gln His Pro Ala Gln Pro Ala Leu
            180                 185                 190
Leu Pro Arg Gly Glu Arg Glu Thr Gln Arg Lys Glu Gly Ile Ala Ala
        195                 200                 205
Val Lys Pro Glu Ala Thr Glu Ser Phe Asp Val Gly Gly Gly Leu Phe
    210                 215                 220
Phe Asp Met Ala Phe Pro Thr Phe Pro Ala Ser Pro Pro Pro Gln Ala
225                 230                 235                 240
Val Asp Thr Ser Phe Ala Gly Ser Thr Ala Thr Ser Glu Thr Gly Ser
                245                 250                 255
Pro Ala Lys Arg Pro Arg Cys Asp Glu Asp Ser Ser Glu Gly Gly Ser
            260                 265                 270
Gly Ser Ala Leu Glu Leu Ala Asp Glu Leu Ala Phe Asp Pro Phe Val
        275                 280                 285
Leu Leu Gln Met Pro Tyr Ser Gly Gly Tyr Asp Asp Asp Ser Leu Asp
    290                 295                 300
Gly Leu Phe Ser Ala Asp Glu Ala Val Gln Gln Asp Val Gly Asn Gly
305                 310                 315                 320
Met Asp Gly Val Arg Leu Trp Arg Phe Asp Glu Phe Pro Ala Val Asp
                325                 330                 335
Gly Ser Val Phe
            340
```

<210> 28
<211> 53
<212> DNA
<213> Artificial sequence

<220>
<223> primer

<400> 28
ggggaccact ttgtacaaga aagctgggtt cagaaaatgg cgctgtccat ggg 53

<210> 29
<211> 55
<212> DNA
<213> Artificial sequence

<220>
<223> primer

<400> 29
ggggacaagt ttgtacaaaa aagcaggctt aaacaatgtg tggcggcgcc atcct 55

<210> 30
<211> 372
<212> PRT
<213> Artificial sequence

<220>
<223> consensus sequence

<220>
<221> Variant
<222> (9)..(9)

<223> Xaa in position 9 is any amino acid

<220>
<221> Variant
<222> (13)..(16)
<223> Xaa in position 13 to 16 is any amino acid

<220>
<221> Variant
<222> (17)..(22)
<223> Xaa in position 17 to 22 is any or no amino acid

<220>
<221> Variant
<222> (24)..(27)
<223> Xaa in position 24 to 27 is any amino acid

<220>
<221> Variant
<222> (29)..(30)
<223> Xaa in position 29 to 30 is any amino acid

<220>
<221> Variant
<222> (31)..(34)
<223> Xaa in position 31 to 34 is any or no amino acid

<220>
<221> Variant
<222> (36)..(36)
<223> Xaa in position 36 is any amino acid

<220>
<221> Variant
<222> (38)..(38)
<223> Xaa in position 38 is any amino acid

<220>
<221> Variant
<222> (40)..(40)
<223> Xaa in position 40 is any amino acid

<220>
<221> Variant
<222> (42)..(43)
<223> Xaa in position 42 to 43 is any amino acid

<220>
<221> Variant
<222> (45)..(55)
<223> Xaa in position 45 to 55 is any amino acid

<220>
<221> Variant
<222> (56)..(59)
<223> Xaa in position 56 to 59 is any or no amino acid

<220>
<221> Variant
<222> (64)..(64)
<223> Xaa in position 64 is any amino acid

<220>

<221> Variant
<222> (68)..(70)
<223> Xaa in position 68 to 70 is any amino acid

<220>
<221> Variant
<222> (71)..(80)
<223> Xaa in position 71 to 80 is any or no amino acid

<220>
<221> Variant
<222> (82)..(82)
<223> Xaa in position 82 is any amino acid

<220>
<221> Variant
<222> (85)..(85)
<223> Xaa in position 85 is any amino acid

<220>
<221> Variant
<222> (88)..(88)
<223> Xaa in position 88 is any amino acid

<220>
<221> Variant
<222> (90)..(90)
<223> Xaa in position 90 is any amino acid

<220>
<221> Variant
<222> (93)..(99)
<223> Xaa in position 93 to 99 is any amino acid

<220>
<221> Variant
<222> (100)..(104)
<223> Xaa in position 100 to 104 is any or no amino acid

<220>
<221> Variant
<222> (106)..(115)
<223> Xaa in position 106 to 115 is any amino acid

<220>
<221> Variant
<222> (116)..(119)
<223> Xaa in position 116 to 119 is any or no amino acid

<220>
<221> Variant
<222> (148)..(148)
<223> Xaa in position 148 is any amino acid

<220>
<221> Variant
<222> (151)..(151)
<223> Xaa in position 151 is any amino acid

<220>
<221> Variant
<222> (160)..(160)
<223> Xaa in position 160 is any amino acid

<220>
<221> Variant
<222> (175)..(175)
<223> Xaa in position 175 is any amino acid

<220>
<221> Variant
<222> (178)..(180)
<223> Xaa in position 178 to 180 is any amino acid

<220>
<221> Variant
<222> (183)..(184)
<223> Xaa in position 183 to 184 is any amino acid

<220>
<221> Variant
<222> (185)..(186)
<223> Xaa in position 185 to 186 is any or no amino acid

<220>
<221> Variant
<222> (190)..(227)
<223> Xaa in position 190 to 227 is any amino acid

<220>
<221> Variant
<222> (228)..(255)
<223> Xaa in position 228 to 255 is any or no amino acid

<220>
<221> Variant
<222> (257)..(258)
<223> Xaa in position 257 to 258 is any amino acid

<220>
<221> Variant
<222> (260)..(261)
<223> Xaa in position 260 to 261 is any amino acid

<220>
<221> Variant
<222> (264)..(268)
<223> Xaa in position 264 to 268 is any amino acid

<220>
<221> Variant
<222> (269)..(269)
<223> Xaa in position 269 is any or no amino acid

<220>
<221> Variant
<222> (272)..(273)
<223> Xaa in position 272 to 273 is any amino acid

<220>
<221> Variant
<222> (274)..(289)
<223> Xaa in position 274 to 289 is any or no amino acid

<220>
<221> Variant

<222> (291)..(291)
<223> Xaa in position 291 is any amino acid

<220>
<221> Variant
<222> (292)..(292)
<223> Xaa in position 292 is any or no amino acid

<220>
<221> Variant
<222> (296)..(296)
<223> Xaa in position 296 is any amino acid

<220>
<221> Variant
<222> (298)..(299)
<223> Xaa in position 298 to 299 is any amino acid

<220>
<221> Variant
<222> (300)..(301)
<223> Xaa in position 300 to 301 is any or no amino acid

<220>
<221> Variant
<222> (303)..(304)
<223> Xaa in position 303 to 304 is any amino acid

<220>
<221> Variant
<222> (305)..(305)
<223> Xaa in position 305 is any or no amino acid

<220>
<221> Variant
<222> (307)..(307)
<223> Xaa in position 307 is any amino acid

<220>
<221> Variant
<222> (308)..(309)
<223> Xaa in position 308 to 309 is any or no amino acid

<220>
<221> Variant
<222> (313)..(313)
<223> Xaa in position 313 is any amino acid

<220>
<221> Variant
<222> (319)..(322)
<223> Xaa in position 319 to 322 is any amino acid

<220>
<221> Variant
<222> (324)..(324)
<223> Xaa in position 324 is any amino acid

<220>
<221> Variant
<222> (326)..(329)
<223> Xaa in position 326 to 329 is any amino acid

<220>
<221> Variant
<222> (330)..(331)
<223> Xaa in position 330 to 331 is any or no amino acid

<220>
<221> Variant
<222> (333)..(333)
<223> Xaa in position 333 is any amino acid

<220>
<221> Variant
<222> (335)..(336)
<223> Xaa in position 335 to 336 is any amino acid

<220>
<221> Variant
<222> (339)..(348)
<223> Xaa in position 339 to 348 is any amino acid

<220>
<221> Variant
<222> (349)..(351)
<223> Xaa in position 349 to 351 is any or no amino acid

<220>
<221> Variant
<222> (354)..(355)
<223> Xaa in position 354 to 355 is any amino acid

<220>
<221> Variant
<222> (357)..(357)
<223> Xaa in position 357 is any amino acid

<220>
<221> Variant
<222> (366)..(366)
<223> Xaa in position 366 is any amino acid

<220>
<221> Variant
<222> (367)..(367)
<223> Xaa in position 367 is any or no amino acid

<220>
<221> Variant
<222> (369)..(371)
<223> Xaa in position 369 to 371 is any amino acid

<400> 30
```
Met Cys Gly Gly Ala Ile Leu Ala Xaa Leu Ile Pro Xaa Xaa Xaa Xaa
1               5                   10                  15
Xaa Xaa Xaa Xaa Xaa Xaa Lys Xaa Xaa Xaa Xaa Gly Xaa Xaa Xaa Xaa
            20                  25                  30
Xaa Xaa Ser Xaa Lys Xaa Gly Xaa Gly Xaa Xaa Lys Xaa Xaa Xaa Xaa
        35                  40                  45
Xaa Xaa Xaa Xaa Xaa Xaa Xaa Xaa Xaa Xaa Xaa Ala Ala Phe Glu Xaa
    50                  55                  60
Phe Asp Asp Xaa Xaa Xaa Xaa Xaa Xaa Xaa Xaa Xaa Xaa Xaa Xaa Xaa
65                  70                  75                  80
His Xaa Val Phe Xaa Ser Lys Xaa Ala Xaa Ser Pro Xaa Xaa Xaa Xaa
                85                  90                  95
```

```
Xaa Xaa Xaa Xaa Xaa Xaa Xaa Xaa Ala Xaa Xaa Xaa Xaa Xaa Xaa Xaa
            100                 105                 110
Xaa Xaa Xaa Xaa Xaa Xaa Xaa Gly Ile Arg Gln Arg Pro Trp Gly Lys
        115                 120                 125
Trp Ala Ala Glu Ile Arg Asp Pro His Lys Gly Thr Arg Val Trp Leu
    130                 135                 140
Gly Thr Phe Xaa Thr Ala Xaa Asp Ala Ala Arg Ala Tyr Asp Val Xaa
145                 150                 155                 160
Ala Arg Arg Leu Arg Gly Ser Lys Ala Lys Val Asn Phe Pro Xaa Ala
                165                 170                 175
Ala Xaa Xaa Xaa Ala Arg Xaa Xaa Xaa Xaa Pro Arg Arg Xaa Xaa Xaa
            180                 185                 190
Xaa Xaa Xaa Xaa Xaa Xaa Xaa Xaa Xaa Xaa Xaa Xaa Xaa Xaa Xaa Xaa
        195                 200                 205
Xaa Xaa Xaa Xaa Xaa Xaa Xaa Xaa Xaa Xaa Xaa Xaa Xaa Xaa Xaa Xaa
    210                 215                 220
Xaa Xaa Xaa Xaa Xaa Xaa Xaa Xaa Xaa Xaa Xaa Xaa Xaa Xaa Xaa Xaa
225                 230                 235                 240
Xaa Xaa Xaa Xaa Xaa Xaa Xaa Xaa Xaa Xaa Xaa Xaa Xaa Xaa Xaa Thr
                245                 250                 255
Xaa Xaa Ala Xaa Xaa Pro Pro Xaa Xaa Xaa Xaa Xaa Xaa Phe Ala Xaa
            260                 265                 270
Xaa Xaa Xaa Xaa Xaa Xaa Xaa Xaa Xaa Xaa Xaa Xaa Xaa Xaa Xaa Xaa
        275                 280                 285
Xaa Asp Xaa Xaa Asp Ser Ser Xaa Gly Xaa Xaa Xaa Xaa Gly Xaa Xaa
    290                 295                 300
Xaa Leu Xaa Xaa Xaa Asp Glu Leu Xaa Phe Asp Pro Phe Met Xaa Xaa
305                 310                 315                 320
Xaa Xaa Pro Xaa Ser Xaa Xaa Xaa Xaa Xaa Xaa Ser Xaa Asp Xaa Xaa
                325                 330                 335
Phe Ala Xaa Xaa Xaa Xaa Xaa Xaa Xaa Xaa Xaa Xaa Xaa Xaa Xaa Gly
            340                 345                 350
Met Xaa Xaa Val Xaa Leu Trp Ser Phe Asp Glu Phe Pro Xaa Xaa Asp
        355                 360                 365
Xaa Xaa Xaa Phe
    370
```

<210> 31
<211> 60
<212> PRT
<213> Artificial sequence

<220>
<223> protein pattern

<220>
<221> Variant
<222> (2)..(5)
<223> Xaa in position 2 to 5 is any or no amino acid

<220>
<221> Variant
<222> (33)..(33)
<223> Xaa in position 33 is Asp, Asn or Ser

<220>
<221> Variant
<222> (36)..(36)
<223> Xaa in position 36 is Asp or Glu

<220>
<221> Variant

<222> (45)..(45)
<223> Xaa in position 45 is Ala or Glu

<220>
<221> Variant
<222> (47)..(47)
<223> Xaa in position 47 is His or Arg

<220>
<221> Variant
<222> (60)..(60)
<223> Xaa in position 60 is Ala, Asp or Asn

<400> 31

```
Gly Xaa Xaa Xaa Xaa Ile Arg Gln Arg Pro Trp Gly Lys Trp Ala Ala
1               5                   10                  15
Glu Ile Arg Asp Pro His Lys Gly Thr Arg Val Trp Leu Gly Thr Phe
            20                  25                  30
Xaa Thr Ala Xaa Asp Ala Ala Arg Ala Tyr Asp Val Xaa Ala Xaa Arg
        35                  40                  45
Leu Arg Gly Ser Lys Ala Lys Val Asn Phe Pro Xaa
    50                  55                  60
```

<210> 32
<211> 30
<212> PRT
<213> Artificial sequence

<220>
<223> protein pattern

<220>
<221> Variant
<222> (2)..(2)
<223> Xaa in position 2 is any or no amino acid

<220>
<221> Variant
<222> (4)..(4)
<223> Xaa in position 4 is any or no amino acid

<220>
<221> Variant
<222> (6)..(9)
<223> Xaa in position 6 to 9 is any amino acid

<220>
<221> Variant
<222> (10)..(10)
<223> Xaa in position 10 is any or no amino acid

<220>
<221> Variant
<222> (14)..(14)
<223> Xaa in position 14 is Ala, Glu or Gly

<220>
<221> Variant
<222> (19)..(19)
<223> Xaa in position 19 is Met or Val

<220>

<221> Variant
<222> (20)..(20)
<223> Xaa in position 20 is Leu or Met

<220>
<221> Variant
<222> (21)..(22)
<223> Xaa in position 21 to 22 is any amino acid

<220>
<221> Variant
<222> (23)..(23)
<223> Xaa in position 23 is Leu or Met

<220>
<221> Variant
<222> (25)..(25)
<223> Xaa in position 25 is any amino acid

<220>
<221> Variant
<222> (27)..(27)
<223> Xaa in position 27 is Asp, Glu or Gly

<220>
<221> Variant
<222> (28)..(29)
<223> Xaa in position 28 to 29 is any amino acid

<220>
<221> Variant
<222> (30)..(30)
<223> Xaa in position 30 is Asp, Glu or Thr

<400> 32

```
Gly Xaa Gly Xaa Ala Xaa Xaa Xaa Xaa Xaa Asp Glu Leu Xaa Phe Asp
1               5                   10                  15
Pro Phe Xaa Xaa Xaa Xaa Xaa Pro Xaa Ser Xaa Xaa Xaa Xaa
            20                  25                  30
```

<210> 33
<211> 22
<212> PRT
<213> Artificial sequence

<220>
<223> protein pattern

<220>
<221> Variant
<222> (2)..(3)
<223> Xaa in position 2 to 3 is any amino acid

<220>
<221> Variant
<222> (4)..(5)
<223> Xaa in position 4 to 5 is any or no amino acid

<220>
<221> Variant
<222> (7)..(7)
<223> Xaa in position 7 is Asp, Gly or Asn

<220>
<221> Variant
<222> (8)..(8)
<223> Xaa in position 8 is Asn, Ser or Thr

<220>
<221> Variant
<222> (10)..(10)
<223> Xaa in position 10 is Ile or Met

<220>
<221> Variant
<222> (11)..(11)
<223> Xaa in position 11 is Asp or Glu

<220>
<221> Variant
<222> (12)..(12)
<223> Xaa in position 12 is Ala, Gly or Ser

<220>
<221> Variant
<222> (14)..(14)
<223> Xaa in position 14 is Arg or Ser

<220>
<221> Variant
<222> (17)..(17)
<223> Xaa in position 17 is Arg or Ser

<220>
<221> Variant
<222> (18)..(18)
<223> Xaa in position 18 is any amino acid

<220>
<221> Variant
<222> (19)..(19)
<223> Xaa in position 19 is Asp or Glu

<400> 33
```
Ala Xaa Xaa Xaa Xaa Val Xaa Xaa Gly Xaa Xaa Xaa Val Xaa Leu Trp
1               5                   10                  15
Xaa Xaa Xaa Glu Phe Pro
            20
```

<210> 34
<211> 8
<212> PRT
<213> Artificial sequence

<220>
<223> protein pattern

<220>
<221> Variant
<222> (2)..(2)
<223> Xaa in position 2 is Ala, Ile or Leu

<220>
<221> Variant

<222> (4)..(4)
<223> Xaa in position 4 is Gly or Ser

<220>
<221> Variant
<222> (5)..(5)
<223> Xaa in position 5 is Ile or Leu

<220>
<221> Variant
<222> (8)..(8)
<223> Xaa in position 8 is Ala or Gly

<400> 34
Ser Xaa Asp Xaa Xaa Phe Ala Xaa
1               5

<210> 35
<211> 2194
<212> DNA
<213> Oryza sativa

<400> 35
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag atttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctccttctc ccatctataa attcctcccc cctttcccc tctctatata 1020

ggaggcatcc aagccaagaa gagggagagc accaaggaca cgcgactagc agaagccgag 1080

cgaccgcctt ctcgatccat atcttccggt cgagttcttg gtcgatctct tccctcctcc 1140

acctcctcct cacagggtat gtgcctccct tcggttgttc ttggatttat tgttctaggt 1200

tgtgtagtac gggcgttgat gttaggaaag gggatctgta tctgtgatga ttcctgttct 1260

tggatttggg atagaggggt tcttgatgtt gcatgttatc ggttcggttt gattagtagt 1320

atggttttca atcgtctgga gagctctatg gaaatgaaat ggtttaggga tcggaatctt 1380

gcgattttgt gagtaccttt tgtttgaggt aaaatcagag caccggtgat tttgcttggt 1440

gtaataaagt acggttgttt ggtcctcgat tctggtagtg atgcttctcg atttgacgaa 1500

gctatccttt gtttattccc tattgaacaa aaataatcca actttgaaga cggtcccgtt 1560

gatgagattg aatgattgat tcttaagcct gtccaaaatt tcgcagctgg cttgtttaga 1620

tacagtagtc cccatcacga aattcatgga aacagttata atcctcagga acaggggatt 1680

ccctgttctt ccgatttgct ttagtcccag aattttttt cccaaatatc ttaaaaagtc 1740

actttctggt tcagttcaat gaattgattg ctacaaataa tgcttttata gcgttatcct 1800

agctgtagtt cagttaatag gtaatacccc tatagtttag tcaggagaag aacttatccg 1860

atttctgatc tccattttta attatatgaa atgaactgta gcataagcag tattcatttg 1920

gattattttt tttattagct ctcacccctt cattattctg agctgaaagt ctggcatgaa 1980

ctgtcctcaa ttttgttttc aaattcacat cgattatcta tgcattatcc tcttgtatct 2040

acctgtagaa gtttcttttt ggttattcct tgactgcttg attacagaaa gaaatttatg 2100

aagctgtaat cgggatagtt atactgcttg ttcttatgat tcatttcctt tgtgcagttc 2160

ttggtgtagc ttgccacttt caccagcaaa gttc 2194

<210> 36
<211> 1264
<212> DNA
<213> Oryza sativa

<400> 36
tcgacgctac tcaagtggtg ggaggccacc gcatgttcca acgaagcgcc aaagaaagcc 60

ttgcagactc taatgctatt agtcgcctag gatatttgga atgaaaggaa ccgcagagtt 120

tttcagcacc aagagcttcc ggtggctagt ctgatagcca aaattaagga ggatgccaaa 180

acatgggtct tggcgggcgc gaaacacctt gataggtggc ttacctttta acatgttcgg 240

gccaaaggcc ttgagacggt aaagttttct atttgcgctt gcgcatgtac aattttattc 300

ctctattcaa tgaaattggt ggctcactgg ttcattaaaa aaaaaagaat ctagcctgtt 360

cgggaagaag aggattttgt tcgtgagaga gagagagaga gagagagaga gagagagaga 420

gaaggaggag gaggattttc aggcttcgca ttgcccaacc tctgcttctg ttggcccaag 480

aagaatccca ggcgcccatg ggctggcagt ttaccacgga cctacctagc ctaccttagc 540

tatctaagcg ggccgaccta gtagccacgt gcctagtgta gattaaagtt gccgggccag 600

```
caggaagcca cgctgcaatg gcatcttccc ctgtccttcg cgtacgtgaa aacaaaccca       660
ggtaagctta gaatcttctt gcccgttgga ctgggacacc caccaatccc accatgcccc       720
gatattcctc cggtctcggt tcatgtgatg tcctctcttg tgtgatcacg gagcaagcat       780
tcttaaacgg caaaagaaaa tcaccaactt gctcacgcag tcacgctgca ccgcgcgaag       840
cgacgcccga taggccaaga tcgcgagata aaataacaac caatgatcat aaggaaacaa       900
gcccgcgatg tgtcgtgtgc agcaatcttg gtcatttgcg ggatcgagtg cttcacagct       960
aaccaaatat tcggccgatg atttaacaca ttatcagcgt agatgtacgt acgatttgtt      1020
aattaatcta cgagccttgc tagggcaggt gttctgccag ccaatccaga tcgccctcgt      1080
atgcacgctc acatgatggc agggcagggt tcacatgagc tctaacggtc gattaattaa      1140
tcccggggct cgactataaa tacctcccta atcccatgat caaaaccatc tcaagcagcc      1200
taatcatctc cagctgatca agagctctta attagctagc tagtgattag ctgcgcttgt      1260
gatc                                                                   1264
```

## Description

### Background

The present invention relates generally to the field of molecular biology and concerns a method for enhancing one or more yield-related traits in plants by modulating expression in a plant of a nucleic acid encoding a HvRAF-like (Hordeum vulgare "RAF-like protein family") polypeptide. The present invention also concerns plants having modulated expression of a nucleic acid encoding a HVRAF-like polypeptide, which plants have one or more enhanced yield-related traits relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta 218, 1-14, 2003). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

It has now been found that various yield-related traits may be improved in plants by modulating expression in a plant of a nucleic acid encoding a HvRAF-like (Hordeum vulgare "RAF-like protein family") polypeptide in a plant.

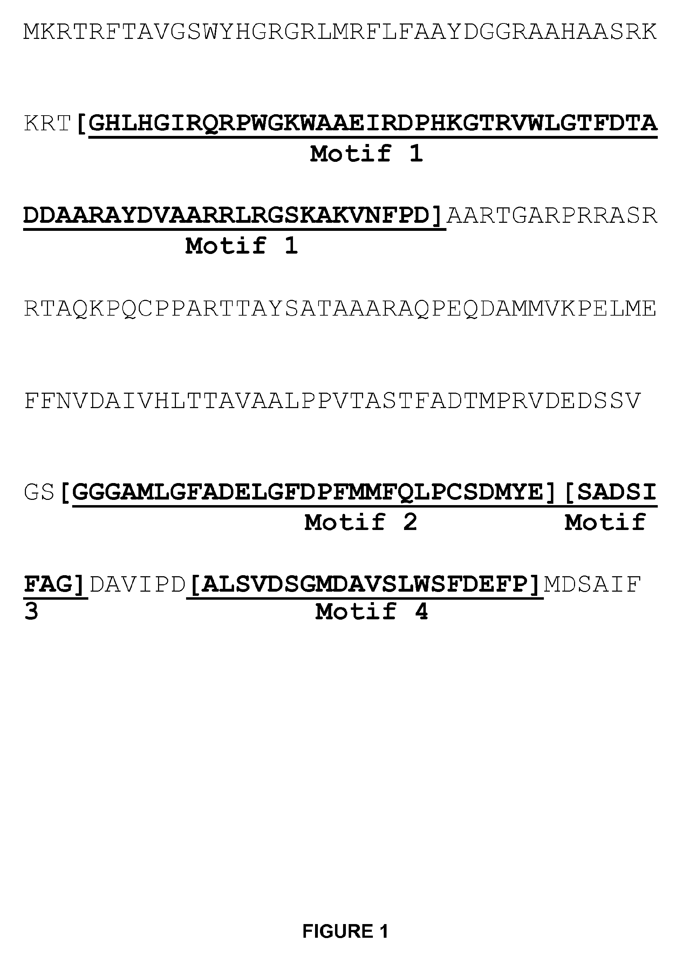

The HvRAF-like (Hordeum vulgare "RAF-like protein family") polypeptides are members of the AP2/ERF family of transcription factors. The open reading frame of the related gene GenBank protein ID: AAZ14086 encodes a polypeptide of 328 amino acids (predicted MW 35 kDa; pl 5.6), which contains a novel N-terminal MCGGAIL motif, a putative nuclear localization sequence (NLS; RKKR), an AP2/ERF domain that is highly conserved, and an acidic domain comprising a mammalian hemopexin domain signature-like sequence (Tolosano and Altrude 2002) at the C-terminus (Fig. 1) (Jung et al., 2007).

HvRAF-like polypeptides are translocated into the nucleus of cells, and are known to transactivate the lacZ reporter gene in yeast, and activate stress-responsive genes in 35S::HvRAF transgenic Arabidopsis (Jung et al., 2007).

In addition, ERF-type transcription factors have been shown to bind to the GCC box, an essential cis-acting element in ET-, MeJA-, and SA-responsive genes, and also CRT/DRE of several cold-, high salt-, and drought-inducible genes, thus regulating the expression of both biotic and abiotic stress-responsive genes (Ohme-Takagi and Shinshi 1995; Park et al. 2001; Lee et al. 2004; Yi et al. 2004).

**Definitions**

The following definitions will be used throughout the present application. The section captions and headings in this application are for convenience and reference purpose only and should not affect in any way the meaning or interpretation of this application. The technical terms and expressions used within the scope of this application are generally to be given the meaning commonly applied to them in the pertinent art of plant biology, molecular biology, bioinformatics and plant breeding. All of the following term definitions apply to the complete content of this application. The term "essentially", "about", "approximately" and the like in connection with an attribute or a value, particularly also define exactly the attribute or exactly the value, respectively. The term "about" in the context of a given numeric value or range relates in particular to a value or range that is within 20%, within 10%, or within 5% of the value or range given. As used herein, the term "comprising" also encompasses the term "consisting of".

<u>Peptide(s)/Protein(s)</u>

The terms "peptides", "oligopeptides", "polypeptide" and "protein" are used interchangeably herein and refer to amino acids in a polymeric form of any length, linked together by peptide bonds, unless mentioned herein otherwise.

<u>Polynucleotide(s)/Nucleic acid(s)/Nucleic acid sequence(s)/nucleotide sequence(s)</u>

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

<u>Homologue(s)</u>

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

"Homologues" of a gene encompass nucleic acid sequences with nucleotide substitutions, deletions and/or insertions relative to the unmodified gene in question and having similar biological and functional properties as the unmodified gene from which they are derived, or encoding polypeptides having substantially the same biological and functional activity as the polypeptide encoded by the unmodified nucleic acid sequence

Orthologues and paralogues are two different forms of homologues and encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

A "deletion" refers to removal of one or more amino acids from a protein.

An "insertion" refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

A "substitution" refers to replacement of amino acids of the protein with other amino acids having similar properties (such as similar hydrophobicity, hydrophilicity, antigenicity, propensity to form or break $\alpha$-helical structures or $\beta$-sheet structures). Amino acid substitutions are typically of single residues, but may be clustered depending upon functional constraints placed upon the polypeptide and may range from 1 to 10 amino acids. The amino acid substitutions are preferably conservative amino acid substitutions. Conservative substitution tables are well known in the art (see for example Creighton (1984) Proteins. W.H. Freeman and Company (Eds) and Table 1 below).

**Table 1:** Examples of conserved amino acid substitutions

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols (see Current Protocols in Molecular Biology, John Wiley & Sons, N.Y. (1989 and yearly updates)).

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

"Derivatives" of nucleic acids include nucleic acids which may, compared to the nucleotide sequence of the naturally-occurring form of the nucleic acid comprise deletions, alterations, or additions with non-naturally occurring nucleotides.

Functional fragments

The term "functional fragment" refers to any nucleic acid or protein which represents merely a part of the full length nucleic acid or full length protein, respectively, but still provides substantially the same function when overexpressed or repressed in a plant respectively, or still has the same biological activity of the full length nucleic acid or full length protein.

Domain, Motif/Consensus sequence/Signature

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)) & The Pfam protein families database: R.D. Finn, J. Mistry, J. Tate, P. Coggill, A. Heger, J.E. Pollington, O.L. Gavin, P. Gunesekaran, G. Ceric, K. Forslund, L. Holm, E.L. Sonnhammer, S.R. Eddy, A. Bateman Nucleic Acids Research (2010) Database Issue 38:211-222). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

Reciprocal BLAST

Typically, this involves a first BLAST involving BLASTing a query sequence (for example using any of the sequences listed in Table A of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived. The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Transit peptide

A "transit peptide" (transit signal, signal peptide, signal sequence) is a short (3-60 amino acids long) sequence that directs the transport of a protein, preferably to organelles within the cell or to certain subcellular locations or for the secretion of a protein.

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitrocellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The $T_m$ is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The $T_m$ may be calculated using the following equations, depending on the types of hybrids:

1) DNA-DNA hybrids (Meinkoth and Wahl, Anal. Biochem., 138: 267-284, 1984):

$$T_m = 81.5°C + 16.6 x \log_{10}[Na^+]^a + 0.41 x \%[G/C^b] - 500 x [L^c]^{-1} - 0.61 x \% \text{ formamide}$$

2) DNA-RNA or RNA-RNA hybrids:

$$T_m = 79.8°C + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m = 2 (l_n)$
For 20-35 nucleotides: $T_m = 22 + 1.46 (l_n)$

[a] or for other monovalent cation, but only accurate in the 0.01-0.4 M range.
[b] only accurate for %GC in the 30% to 75% range.

[c] L = length of duplex in base pairs.

[d] oligo, oligonucleotide; $I_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

<u>Splice variant</u>

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

<u>Allelic variant</u>

"Alleles" or "allelic variants" are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

<u>Endogenous</u>

Reference herein to an "endogenous" nucleic acid and / or protein refers to the nucleic acid and / or protein in question as found in a plant in its natural form (i.e., without there being any human intervention like recombinant DNA engineering), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Exogenous

The term "exogenous" (in contrast to "endogenous") nucleic acid or gene refers to a nucleic acid that has been introduced in a plant by means of recombinant DNA technology. An "exogenous" nucleic acid can either not occur in the plant in its natural form, be different from the nucleic acid in question as found in the plant in its natural form, or can be identical to a nucleic acid found in the plant in its natural form, but not integrated within its natural genetic environment.

Gene shuffling/Directed evolution

"Gene shuffling" or "directed evolution" consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Construct

Artificial DNA (such as but, not limited to plasmids or viral DNA) capable of replication in a host cell and used for introduction of a DNA sequence of interest into a host cell or host organism. Host cells of the invention may be any cell selected from bacterial cells, such as Escherichia coli or Agrobacterium species cells, yeast cells, fungal, algal or cyanobacterial cells or plant cells. The skilled artisan is well aware of the genetic elements that must be present on the genetic construct in order to successfully transform, select and propagate host cells containing the sequence of interest. The sequence(s) of interest is/are operably linked to one or more control sequences (at least to a promoter) as described herein. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

Operably linked

The term "operably linked" or "functionally linked" is used interchangeably and, as used herein, refers to a functional linkage between the promoter sequence and the gene of interest, such that the promoter sequence is able to direct transcription of the gene of interest.

The term "functional linkage" or "functionally linked" with respect to regulatory elements, is to be understood as meaning, for example, the sequential arrangement of a regulatory element (e.g. a promoter) with a nucleic acid sequence to be expressed and, if appropriate, further regulatory elements (such as e.g., a terminator, NEENA or a RENA) in such a way that each of the regulatory elements can fulfil its intended function to allow, modify, facilitate or otherwise influence expression of said nucleic acid sequence. As a synonym the wording "operable linkage" or "operably linked" may be used. The expression may result, depending on the arrangement of the nucleic acid sequences, in sense or antisense RNA. To this end, direct linkage in the chemical sense is not necessarily required. Genetic control sequences such as, for example, enhancer sequences, can also exert their function on the target sequence from positions which are further away, or indeed from other DNA molecules. Preferred arrangements are those in which the nucleic acid sequence to be expressed is recombinantly positioned behind the sequence acting as promoter, so that the two sequences are linked covalently to each other. The distance between the promoter sequence and the recombinant nucleic acid sequence to be expressed is preferably less than 200 base pairs, especially preferably less than 100 base pairs, very especially preferably less than 50 base pairs. In a preferred embodiment, the nucleic acid sequence to be transcribed is located behind the promoter in such a way that the transcription start is identical with the desired beginning of the chimeric RNA of the invention. Functional linkage, and an expression construct, can be generated by means of customary recombination and cloning techniques as described (e.g., in Maniatis T, Fritsch EF and Sambrook J (1989) Molecular Cloning: A Laboratory Manual, 2nd Ed., Cold Spring Harbor Laboratory, Cold Spring Harbor (NY); Silhavy et al. (1984) Experiments with Gene Fusions, Cold Spring Harbor Laboratory, Cold Spring Harbor (NY); Ausubel et al. (1987) Current Protocols in Molecular Biology, Greene Publishing Assoc. and Wiley Interscience; Gelvin et al. (Eds) (1990) Plant Molecular Biology Manual; Kluwer Academic Publisher, Dordrecht, The Netherlands). However, further sequences, which, for example, act as a linker with specific cleavage sites for restriction enzymes, or as a signal peptide, may also be positioned between the two sequences. The insertion of sequences may also lead to the expression of fusion proteins. Preferably, the expression construct, consisting of a linkage of a regulatory region for example a promoter and nucleic acid sequence to be expressed, can exist in a vector-integrated form and be inserted into a plant genome, for example by transformation.

Constitutive promoter

A "constitutive promoter" refers to a promoter that is transcriptionally active during most, but not necessarily all, phases of growth and development and under most environmental conditions, in at least one cell, tissue or organ.

Table 2a below gives examples of constitutive promoters.

Table 2a: Examples of constitutive promoters

| Gene Source | Reference |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A "ubiquitous promoter" is active in substantially all tissues or cells of an organism.

Developmentally-regulated promoter

A "developmentally-regulated promoter" is active during certain developmental stages or in parts of the plant that undergo developmental changes.

Inducible promoter

An "inducible promoter" has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An "organ-specific" or "tissue-specific promoter" is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

Table 2b: Examples of root-specific promoters

| Gene Source | Reference |
|---|---|
| RCc3 | Plant Mol Biol. 1995 Jan;27(2):237-48 |
| Arabidopsis PHT1 | Koyama et al. J Biosci Bioeng. 2005 Jan;99(1):38-42.; Mudge et al. (2002, Plant J. 31:341) |
| Medicago phosphate transporter | Xiao et al., 2006, Plant Biol (Stuttg). 2006 Jul;8(4):439-49 |
| Arabidopsis Pyk10 | Nitz et al. (2001) Plant Sci 161(2): 337-346 |
| root-expressible genes | Tingey et al., EMBO J. 6: 1, 1987. |
| tobacco auxin-inducible gene | Van der Zaal et al., Plant Mol. Biol. 16, 983, 1991. |
| β-tubulin | Oppenheimer, et al., Gene 63: 87, 1988. |
| tobacco root-specific genes | Conkling, et al., Plant Physiol. 93: 1203, 1990. |
| B. napus G1-3b gene | United States Patent No. 5, 401, 836 |
| SbPRP1 | Suzuki et al., Plant Mol. Biol. 21: 109-119, 1993. |
| LRX1 | Baumberger et al. 2001, Genes & Dev. 15:1128 |
| BTG-26 Brassica napus | US 20050044585 |
| LeAMT1 (tomato) | Lauter et al. (1996, PNAS 3:8139) |
| The LeNRT1-1 (tomato) | Lauter et al. (1996, PNAS 3:8139) |
| class I patatin gene (potato) | Liu et al., Plant Mol. Biol. 17 (6): 1139-1154 |
| KDC1 (Daucus carota) | Downey et al. (2000, J. Biol. Chem. 275:39420) |
| TobRB7 gene | W Song (1997) PhD Thesis, North Carolina State University, Raleigh, NC USA |
| OsRAB5a (rice) | Wang et al. 2002, Plant Sci. 163:273 |
| ALF5 (Arabidopsis) | Diener et al. (2001, Plant Cell 13:1625) |
| NRT2;1Np (N. plumbaginifolia) | Quesada et al. (1997, Plant Mol. Biol. 34:265) |

A "seed-specific promoter" is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters (endosperm/aleurone/embryo specific) are shown in Table 2c to Table 2f below. Further examples of seed-specific promoters are given in Qing Qu and Takaiwa (Plant Biotechnol. J. 2, 113-125, 2004), which disclosure is incorporated by reference herein as if fully set forth.

**Table 2c:** Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |

(continued)

| Gene source | Reference |
|---|---|
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat α, β, γ-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice α-globulin Glb-1 | Wu et al, Plant Cell Physiology 39(8) 885-889, 1998 |
| rice OSH1 | Sato et al, Proc. Natl. Acad. Sci. USA, 93: 8117-8122, |
| | 1996 |
| rice α-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum α-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | unpublished |
| PRO0147, trypsin inhibitor ITR1 (barley) | unpublished |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 2d:** examples of endosperm-specific promoters

| Gene source | Reference |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 2e:** Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO015 | WO 2004/070039 |
| PRO0175 | WO 2004/070039 |
| PRO005 | WO 2004/070039 |
| PRO0095 | WO 2004/070039 |

**Table 2f:** Examples of aleurone-specific promoters:

| Gene source | Reference |
|---|---|
| $\alpha$-amylase (Amy32b) | Lanahan et al, Plant Cell 4:203-211, 1992; Skriver et al, Proc Natl Acad Sci USA 88:7266-7270, 1991 |
| cathepsin $\beta$-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A "green tissue-specific promoter" as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g below.

**Table 2g:** Examples of green tissue-specific promoters

| Gene | Expression | Reference |
|---|---|---|
| Maize Orthophosphate dikinase | Leaf specific | Fukavama et al., Plant Physiol. 2001 Nov;127(3):1136-46 |
| Maize Phosphoenolpyruvate carboxylase | Leaf specific | Kausch et al., Plant Mol Biol. 2001 Jan;45(1):1-15 |
| Rice Phosphoenolpyruvate carboxylase | Leaf specific | Lin et al., 2004 DNA Seq. 2004 Aug;15(4):269-76 |
| Rice small subunit Rubisco | Leaf specific | Nomura et al., Plant Mol Biol. 2000 Sep;44(1):99-106 |
| rice beta expansin EXBP9 | Shoot specific | WO 2004/070039 |
| Pigeonpea small subunit Rubisco | Leaf specific | Panguluri et al., Indian J Exp Biol. 2005 Apr;43(4):369-72 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of green meristem-specific promoters which may be used to perform the methods of the invention are shown in Table 2h below.

**Table 2h:** Examples of meristem-specific promoters

| Gene source | Expression pattern | Reference |
|---|---|---|
| rice OSH1 | Shoot apical meristem, from embryo globular stage to seedling stage | Sato et al. (1996) Proc. Natl. Acad. Sci. USA, 93: 8117-8122 |
| Rice metallothionein | Meristem specific | BAD87835.1 |
| WAK1 & WAK2 | Shoot and root apical meristems, and in expanding leaves and sepals | Wagner & Kohorn (2001) Plant Cell 13(2): 303-318 |

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the IoxP sequences. If the marker gene is integrated between the IoxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not present in, or originating from, the genome of said plant, or are present in the genome of said plant but not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

It shall further be noted that in the context of the present invention, the term "isolated nucleic acid" or "isolated polypeptide" may in some instances be considered as a synonym for a "recombinant nucleic acid" or a "recombinant polypeptide", respectively and refers to a nucleic acid or polypeptide that is not located in its natural genetic environment and/or that has been modified by recombinant methods. An isolated nucleic acid sequence or isolated nucleic acid molecule is one that is not in its native surrounding or its native nucleic acid neighbourhood, yet it is physically and functionally connected to other nucleic acid sequences or nucleic acid molecules and is found as part of a nucleic acid construct, vector sequence or chromosome.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. For the purposes of this invention, the original unmodulated expression may also be absence of any expression. The term "modulating the activity" or the term "modulating expression" with respect to the proteins or nucleic acids used in the methods of the invention shall mean any change of the expression which leads to enhanced yield-related traits in the plants. The expression can increase from zero (absence of, or immeasurable expression) to a certain amount, or can decrease from a certain amount to immeasurable small amounts or zero.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level. For the purposes of this invention, the original wild-type expression level might also be zero, i.e. absence of expression or immeasurable expression. Reference herein to "increased expression" is taken to mean an increase in gene expression and/or, as far as referring to polypeptides, increased polypeptide levels and/or increased polypeptide activity, relative to control plants. The increase in expression is in increasing order of preference at least 10%, 20%, 30%, 40% or 50%, 60%, 70%, 80%, 85%, 90%, or 95%, 96%, 97%, 98%, 99% or even more compared to that of control plants.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

To obtain increased expression or overexpression of a polypeptide most commonly the nucleic acid encoding this polypeptide is overexpressed in sense orientation with a polyadenylation signal. Introns or other enhancing elements may be used in addition to a promoter suitable for driving expression with the intended expression pattern.

Decreased expression

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing, preferably by recombinant methods, and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an $\alpha$-anomeric nucleic acid sequence. An $\alpha$-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g.,

hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation(s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

<u>Transformation</u>

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art. Alternatively, a plant cell that cannot be regenerated into a plant may be chosen as host cell, i.e. the resulting transformed plant cell does not have the capacity to regenerate into a (whole) plant.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for Agrobacterium-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis (Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:1-9; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

The genetically modified plant cells can be regenerated via all methods with which the skilled worker is familiar. Suitable methods can be found in the abovementioned publications by S.D. Kung and R. Wu, Potrykus or Höfgen and Willmitzer. Alternatively, the genetically modified plant cells are non-regenerable into a whole plant.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

Throughout this application a plant, plant part, seed or plant cell transformed with - or interchangeably transformed by - a construct or transformed with or by a nucleic acid is to be understood as meaning a plant, plant part, seed or plant cell that carries said construct or said nucleic acid as a transgene due the result of an introduction of this construct or this nucleic acid by biotechnological means. The plant, plant part, seed or plant cell therefore comprises this recombinant construct or this recombinant nucleic acid.

Throughout this application a plant, plant part, seed or plant cell transformed with - or interchangeably transformed by - a construct or transformed with or by a nucleic acid is to be understood as meaning a plant, plant part, seed or plant cell that carries said construct or said nucleic acid as a transgene due the result of an introduction of this construct or this nucleic acid by biotechnological means. The plant, plant part, seed or plant cell therefore comprises this recombinant construct or this recombinant nucleic acid.

## T-DNA activation tagging

"T-DNA activation" tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

"Homologous recombination" allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8), and approaches exist that are generally applicable regardless of the target organism (Miller et al, Nature Biotechnol. 25, 778-785, 2007).

Yield related Trait(s)

A "Yield related trait" is a trait or feature which is related to plant yield. Yield-related traits may comprise one or more of the following non-limitative list of features: early flowering time, yield, biomass, seed yield, early vigour, greenness index, growth rate, agronomic traits, such as e.g. tolerance to submergence (which leads to yield in rice), Water Use Efficiency (WUE), Nitrogen Use Efficiency (NUE), etc.

Reference herein to "enhanced yield-related trait" is taken to mean an increase relative to control plants in a yield-related trait, for instance in early vigour and/or in biomass, of a whole plant or of one or more parts of a plant, which may include (i) aboveground parts, preferably aboveground harvestable parts, and/or (ii) parts below ground, preferably harvestable parts below ground.

In particular, such harvestable parts are roots such as taproots, stems, beets, tubers, leaves, flowers or seeds.

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters.

The terms "yield" of a plant and "plant yield" are used interchangeably herein and are meant to refer to vegetative biomass such as root and/or shoot biomass, to reproductive organs, and/or to propagules such as seeds of that plant.

Flowers in maize are unisexual; male inflorescences (tassels) originate from the apical stem and female inflorescences (ears) arise from axillary bud apices. The female inflorescence produces pairs of spikelets on the surface of a central axis (cob). Each of the female spikelets encloses two fertile florets, one of them will usually mature into a maize kernel once fertilized. Hence a yield increase in maize may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate, which is the number of filled florets (i.e. florets containing seed) divided by the total number of florets and multiplied by 100), among others.

Inflorescences in rice plants are named panicles. The panicle bears spikelets, which are the basic units of the panicles, and which consist of a pedicel and a floret. The floret is borne on the pedicel and includes a flower that is covered by two protective glumes: a larger glume (the lemma) and a shorter glume (the palea). Hence, taking rice as an example, a yield increase may manifest itself as an increase in one or more of the following: number of plants per square meter, number of panicles per plant, panicle length, number of spikelets per panicle, number of flowers (or florets) per panicle; an increase in the seed filling rate which is the number of filled florets (i.e. florets containing seeds) divided by the total number of florets and multiplied by 100; an increase in thousand kernel weight, among others.

Early flowering time

Plants having an "early flowering time" as used herein are plants which start to flower earlier than control plants. Hence this term refers to plants that show an earlier start of flowering. Flowering time of plants can be assessed by counting the number of days ("time to flower") between sowing and the emergence of a first inflorescence. The "flowering time" of a plant can for instance be determined using the method as described in WO 2007/093444.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increased growth rate

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a mature seed up to the stage where the plant has produced mature seeds, similar to the starting material. This life cycle may be influenced by factors such as speed of germination, early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Stress resistance

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35%, 30% or 25%, more preferably less than 20% or 15% in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Abiotic stresses or environmental stresses may be due to drought or excess water, anaerobic stress, salt stress, chemical toxicity, oxidative stress and hot, cold or freezing temperatures.

"Biotic stresses" are typically those stresses caused by pathogens, such as bacteria, viruses, fungi, plants, nematodes and insects, or other animals, which may result in negative effects on plant growth.

The "abiotic stress" may be an osmotic stress caused by a water stress, e.g. due to drought, salt stress, or freezing stress. Abiotic stress may also be an oxidative stress or a cold stress. "Freezing stress" is intended to refer to stress due to freezing temperatures, i.e. temperatures at which available water molecules freeze and turn into ice. "Cold stress", also called "chilling stress", is intended to refer to cold temperatures, e.g. temperatures below 10°, or preferably below 5°C, but at which water molecules do not freeze. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 97%, 95%, 92%, 90%, 87%, 85%, 83%, 80%, 77% or 75% of the average production of such plant in a given environment. Average production may be calculated on harvest and/or season basis. Persons skilled in the art are aware of average yield productions of a crop.

In particular, the methods of the present invention may be performed under non-stress conditions. In an example, the methods of the present invention may be performed under non-stress conditions such as mild drought to give plants having increased yield relative to control plants.

In another embodiment, the methods of the present invention may be performed under stress conditions, preferably under abiotic stress conditions.
In an example, the methods of the present invention may be performed under stress conditions such as drought to give plants having increased yield relative to control plants.
In another example, the methods of the present invention may be performed under stress conditions such as nutrient deficiency to give plants having increased yield relative to control plants.
Nutrient deficiency may result from a lack of nutrients such as nitrogen, phosphates and other phosphorous-containing compounds, potassium, calcium, magnesium, manganese, iron and boron, amongst others.
In yet another example, the methods of the present invention may be performed under stress conditions such as salt stress to give plants having increased yield relative to control plants. The term salt stress is not restricted to common salt (NaCl), but may be any one or more of: NaCl, KCl, LiCl, $MgCl_2$, $CaCl_2$, amongst others.
In yet another example, the methods of the present invention may be performed under stress conditions such as cold stress or freezing stress to give plants having increased yield relative to control plants.

Increase/Improve/Enhance

The terms "increase", "improve" or "enhance" in the context of a yield-related trait are interchangeable and shall mean in the sense of the application at least a 3%, 4%, 5%, 6%, 7%, 8%, 9% or 10%, preferably at least 15% or 20%, more preferably 25%, 30%, 35% or 40% increase in the yield-related trait (such as more yield and/or growth) in comparison to control plants as defined herein.

Seed yield

Increased seed yield may manifest itself as one or more of the following:

a) an increase in seed biomass (total seed weight) which may be on an individual seed basis and/or per plant and/or per square meter;
b) increased number of flowers per plant;
c) increased number of seeds;
d) increased seed filling rate (which is expressed as the ratio between the number of filled florets divided by the total number of florets);

e) increased harvest index, which is expressed as a ratio of the yield of harvestable parts, such as seeds, divided by the biomass of aboveground plant parts; and

f) increased thousand kernel weight (TKW), which is extrapolated from the number of seeds counted and their total weight. An increased TKW may result from an increased seed size and/or seed weight, and may also result from an increase in embryo and/or endosperm size.

The terms "filled florets" and "filled seeds" may be considered synonyms.

An increase in seed yield may also be manifested as an increase in seed size and/or seed volume. Furthermore, an increase in seed yield may also manifest itself as an increase in seed area and/or seed length and/or seed width and/or seed perimeter.

<u>Greenness Index</u>

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

<u>Biomass</u>

The term "biomass" as used herein is intended to refer to the total weight of a plant or plant part. Total weight can be measured as dry weight, fresh weight or wet weight. Within the definition of biomass, a distinction may be made between the biomass of one or more parts of a plant, which may include any one or more of the following:

- aboveground parts such as but not limited to shoot biomass, seed biomass, leaf biomass, etc.;
- aboveground harvestable parts such as but not limited to shoot biomass, seed biomass, leaf biomass, stem biomass, setts etc.;
- parts below ground, such as but not limited to root biomass, tubers, bulbs, etc.;
- harvestable parts below ground, such as but not limited to root biomass, tubers, bulbs, etc.;
- harvestable parts partially below ground such as but not limited to beets and other hypocotyl areas of a plant, rhizomes, stolons or creeping rootstalks;
- vegetative biomass such as root biomass, shoot biomass, etc.;
- reproductive organs; and
- propagules such as seed.

In a preferred embodiment throughout this application any reference to "root" as biomass or harvestable parts or as organ of increased sugar content is to be understood as a reference to harvestable parts partly inserted in or in physical contact with the ground such as but not limited to beets and other hypocotyl areas of a plant, rhizomes, stolons or creeping rootstalks, but not including leaves, as well as harvestable parts belowground, such as but not limited to root, taproot, tubers or bulbs.

<u>Marker assisted breeding</u>

Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

<u>Use as probes in (gene mapping)</u>

Use of nucleic acids encoding the protein of interest for genetically and physically mapping the genes requires only a nucleic acid sequence of at least 15 nucleotides in length. These nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the nucleic acids encoding the protein of interest. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid encoding the protein of interest in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

<u>Plant</u>

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Tripsacum dactyloides, Triticosecale rimpaui, Triticum* spp. *(e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes (or null control plants) are individuals missing the transgene by segregation. Further, control plants are grown under equal growing conditions to the growing conditions of the plants of the invention, i.e. in the vicinity of, and simultaneously with, the plants of the invention. A "control plant" as used herein refers not only to whole plants, but also to plant parts, including seeds and seed parts.

Propagation material / Propagule

"Propagation material" or "propagule" is any kind of organ, tissue, or cell of a plant capable of developing into a complete plant. "Propagation material" can be based on vegetative reproduction (also known as vegetative propagation, vegetative multiplication, or vegetative cloning) or sexual reproduction. Propagation material can therefore be seeds or parts of the non-reproductive organs, like stem or leave. In particular, with respect to poaceae, suitable propagation material can also be sections of the stem, i.e., stem cuttings (like setts).

Stalk

A "stalk" is the stem of a plant belonging the Poaceae, and is also known as the "millable cane". In the context of poaceae "stalk", "stem", "shoot", or "tiller' are used interchangeably.

Sett

A "sett" is a section of the stem of a plant from the Poaceae, which is suitable to be used as propagation material. Synonymous expressions to "sett" are "seed-cane", "stem cutting", "section of the stalk", and "seed piece".

**Detailed description of the invention**

The present invention shows that modulating expression in a plant of a nucleic acid encoding a HvRAF-like polypeptide provides plants having enhanced yield-related traits relative to control plants.

According to a first embodiment, the present invention provides a method for enhancing one or more yield-related traits in plants relative to control plants, comprising modulating expression in a plant of a nucleic acid encoding a HvRAF-like polypeptide and optionally selecting for plants having one or more enhanced yield-related traits. According to another embodiment, the present invention provides a method for producing plants having one or more enhanced yield-related traits relative to control plants, wherein said method comprises the steps of modulating expression in said plant of a nucleic acid encoding a HvRAF-like polypeptide as described herein and optionally selecting for plants having one or more enhanced yield-related traits.

A preferred method for modulating (preferably, increasing) expression of a nucleic acid encoding a HvRAF-like polypeptide is by introducing and expressing in a plant a nucleic acid encoding a HvRAF-like polypeptide.

Any reference hereinafter to a "protein useful in the methods of the invention" is taken to mean a HvRAF-like polypeptide as defined herein. Any reference hereinafter to a "nucleic acid useful in the methods of the invention" is taken to mean a nucleic acid capable of encoding such a HvRAF-like polypeptide. In one embodiment any reference to a protein or nucleic acid "useful in the methods of the invention" is to be understood to mean proteins or nucleic acids "useful in the methods, constructs, plants, harvestable parts and products of the invention". The nucleic acid to be introduced into a plant (and therefore useful in performing the methods of the invention) is any nucleic acid encoding the type of protein which will now be described, hereafter also named *"HvRAF-like* nucleic acid" or *"HvRAF-like* gene".

A "HvRAF-like polypeptide" as defined herein refers to any polypeptide preferably comprising an AP2 domain corresponding to PFAM accession number PFAM PF00847, and preferably an interpro accession no. ScanRegExp PS00024 C-terminal HEMOPEXIN domain.

Preferably, an HvRAF-like polypeptide useful in the present invention comprises the consensus sequence: M-C-G-G-A-I-L-A-x(1)-L-I-P-x(4,10)-K-x(4)-G-x(2,6)-S-x(1)-K-x(1)-G-x(1)-G-x(2)-K-x(11,15)-A-A-F-E-x(1)-F-D-D-x(3,13)-H-x(1)-V-F-x(1)-S-K-x(1)-A-x(1)-S-P-x(7,12)-A-x(10,14)-G-I-R-Q-R-P-W-G-K-W-A-A-E-I-R-D-P-H-K-G-T-R-V-W-L-G-T-F-x(1)-T-A-x(1)-D-A-A-R-A-Y-D-V-x(1)-A-R-R-L-R-G-S-K-A-K-V-N-F-P-x(1)-A-A-x(3)-A-R-x(2,4)-P-R-R-x(38,66)-T-x(2)-A-x(2)-P-P-x(5,6)-F-A-x(2,18)-D-x(1,2)-D-S-S-x(1)-G-x(2,4)-G-x(2,3)-L-x(1,3)-D-E-L-x(1)-F-D-P-F-M-x(4)-P-x(1)-S-x(4,6)-S-x(1)-D-x(2)-F-A-x(10,13)-G-M-x(2)-V-x(1)-L-W-S-F-D-E-F-P-x(1,2)-D-x(3)-F.

According one embodiment, there is provided a method for improving yield-related traits as provided herein in plants relative to control plants, comprising modulating expression in a plant of a nucleic acid encoding a HvRAF-like polypeptide as defined herein.

In one embodiment the nucleic acid sequence employed in the methods, constructs, plants, harvestable parts and products of the invention is a nucleic acid molecule selected from the group consisting of:

(i) a nucleic acid represented by SEQ ID NO: 1 or SEQ ID NO: 4;
(ii) the complement of a nucleic acid represented by SEQ ID NO: 1 or SEQ ID NO: 4;
(iii) a nucleic acid encoding a HvRAF-like polypeptide having in increasing order of preference at least 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 5 and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs given in SEQ ID NO: 31 to SEQ ID NO: 34, and further preferably conferring one or more enhanced yield-related traits relative to control plants; and
(iv) a nucleic acid molecule which hybridizes with a nucleic acid molecule of (i) to (iii) under high stringency hybridization conditions and preferably confers one or more enhanced yield-related traits relative to control plants;

Or encode a polypeptide selected from the group consisting of:

(i) an amino acid sequence represented by SEQ ID NO: 5;
(ii) an amino acid sequence having, in increasing order of preference, at least 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 5, and additionally or alternatively comprising one or more motifs having in increasing order of preference at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% or more sequence identity to any one or more of the motifs given in SEQ ID NO: 31 to SEQ ID NO: 34, and further preferably conferring one or more enhanced yield-related traits relative to control plants; and
(iii) derivatives of any of the amino acid sequences given in (i) or (ii) above.

Preferably the polypeptide comprises one or more motifs and/ or domains as defined elsewhere herein.

Motifs 1 to 4 were derived using the MEME algorithm (Bailey and Elkan, Proceedings of the Second International Conference on Intelligent Systems for Molecular Biology, pp. 28-36, AAAI Press, Menlo Park, California, 1994). At each position within a MEME motif, the residues are shown that are present in the query set of sequences with a frequency higher than 0.2. Residues within square brackets represent alternatives.

In one embodiment, the HvRAF-like polypeptide as used herein comprises at least one or more of the motifs 1, 2, 3 or 4:

Motif 1 (SEQ ID NO: 31): G-x(0,4)-I-R-Q-R-P-W-G-K-W-A-A-E-I-R-D-P-H-K-G-T-R-V-W-L-G-T-F-[DNS]-T-A-[DE]-D-A-A-R-A-Y-D-V-[AE]-A-[HR]-R-L-R-G-S-K-A-K-V-N-F-P-[ADN];
Motif 2 (SEQ ID NO: 32): G-x(0,1)-G-x(0,1)-A-x(4,5)-D-E-L-[AEG]-F-D-P-F-[MV]-[LM]-x(2)-[LM]-P-x-S-[DEG]-x(2)-[DET];
Motif 3 (SEQ ID NO: 33): A-x(2,4)-V-[DGN]-[NST]-G-[IM]-[DE]-[AGS]-V-[RS]-L-W-[RS]-x-[DE]-E-F-P;
Motif 4 (SEQ ID NO: 34): S-[AIL]-D-[GS]-[IL]-F-A-[AG].

In still another embodiment, the HvRAF-like polypeptide comprises in increasing order of preference, at least 2, at least 3, or all 4 motifs as defined above.

Preferably, the HvRAF-like polypeptide comprises Motifs 1 and 2, Motifs 1 and 3, motifs 1 and 4, motifs 2 and 3, motifs 2 and 4, motifs 3 and 4, motifs 1, 2 and 3, motifs 2, 3 and 4, motifs 1, 2 and 4, motifs 1, 3 and 4 or motifs 1, 2, 3 and 4.

Additionally or alternatively, the HvRAF-like protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71 %, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid sequence represented by SEQ ID NO: 5, provided that the homologous protein comprises any one or more of the conserved motifs as outlined above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). In one embodiment the sequence identity level is determined by comparison of the polypeptide sequences over the entire length of the sequence of SEQ ID NO: 5. Alternatively the sequence identity is determined by comparison of a nucleic acid sequence to the sequence encoding the mature protein in SEQ ID NO: 1, or SEQ ID NO: 4

In another embodiment, the sequence identity level is determined by comparison of one or more conserved domains or motifs in SEQ ID NO: 5 with corresponding conserved domains or motifs in other HvRAF-like polypeptides. Compared to overall sequence identity, the sequence identity will generally be higher when only conserved domains or motifs are considered. Preferably the motifs in a HvRAF-like polypeptide have, in increasing order of preference, at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to any one or more of the motifs represented by SEQ ID NO: 31 to SEQ ID NO: 34 (Motifs 1 to 4). In other words, in another embodiment a method for enhancing one or more yield-related traits in plants is provided wherein said HvRAF-like polypeptide comprises a conserved domain (or motif) with at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the conserved domain starting with amino acid 43 up to amino acid 102 in SEQ ID NO:5.

In another embodiment, a method for enhancing one or more yield-related traits in plants is provided wherein said HvRAF-like polypeptide comprises a conserved motif with at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the conserved motif starting with amino acid 195 up to amino acid 223 in SEQ ID NO:5.

In a further embodiment a method for enhancing one or more yield-related traits in plants is provided wherein said HvRAF-like polypeptide comprises a conserved motif with at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the conserved motif starting with amino acid 238 up to amino acid 257 in SEQ ID NO:5.

In a further embodiment a method for enhancing one or more yield-related traits in plants is provided wherein said HvRAF-like polypeptide comprises a conserved motif with at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the conserved motif starting with amino acid 224 up to amino acid 231 in SEQ ID NO:5.

The terms "domain", "signature" and "motif" are defined in the "definitions" section herein.

Furthermore, HvRAF-like polypeptides (at least in their native form) typically are transcription factors, which translocate into the nucleus, and appear to be activators of stress-responsive genes. Tools and techniques for measuring transcription factor activity are well known in the art. Further details are provided in Example 13. In addition, nucleic acids encoding HvRAF-like polypeptides, when expressed in rice according to the methods of the present invention as outlined in Examples 7 and 9, give plants having increased yield related traits, in particular increases in biomass, seed yield and early vigour. Another function of the nucleic acid sequences encoding HvRAF-like polypeptides is to confer information for synthesis of the HvRAF-like protein that increases yield or yield related traits as described herein, when such a nucleic acid sequence of the invention is transcribed and translated in a living plant cell.

The present invention is illustrated by transforming plants with the nucleic acid sequence represented by SEQ ID NO: 1 or SEQ ID NO: 4, encoding the polypeptide sequence of SEQ ID NO: 5. However, performance of the invention is not restricted to these sequences; the methods of the invention may advantageously be performed using any HvRAF-like-encoding nucleic acid or HvRAF-like polypeptide as defined herein. The term "HvRAF-like" or "HvRAF-like polypeptide" as used herein also intends to include homologues as defined hereunder of SEO ID NO: 5.

Examples of nucleic acids encoding HvRAF-like polypeptides are given in Table A of the Examples section herein. Such nucleic acids are useful in performing the methods of the invention. The amino acid sequences given in Table A of the Examples section are example sequences of orthologues and paralogues of the HvRAF-like polypeptide represented by SEQ ID NO: 5, the terms "orthologues" and "paralogues" being as defined herein. Further orthologues and paralogues may readily be identified by performing a so-called reciprocal blast search as described in the definitions section; where the query sequence is SEQ ID NO: 1, SEQ ID NO: 4 or SEQ ID NO: 5, the second BLAST (back-BLAST) would be against rice sequences.

With respect to the sequences of the invention, a nucleic acid or a polypeptide sequence of plant origin has the characteristic of a codon usage optimised for expression in plants, and of the use of amino acids and regulatory sites common in plants, respectively. The plant of origin may be any plant, but preferably those plants as described in the previous paragraph.

The invention also provides hitherto unknown HvRAF-like-encoding nucleic acids and HvRAF-like polypeptides useful for conferring one or more enhanced yield-related traits in plants relative to control plants.

According to a further embodiment of the present invention, there is therefore provided an isolated nucleic acid molecule selected from the group consisting of:

(i) a nucleic acid represented by any one of SEQ ID NO: 1 or SEQ ID NO: 4;
(ii) the complement of a nucleic acid represented by any one of SEQ ID NO: 1 or SEQ ID NO: 4;
(iii) a nucleic acid encoding the polypeptide as represented by SEQ ID NO: 5, preferably as a result of the degeneracy of the genetic code, said isolated nucleic acid can be derived from a polypeptide sequence as represented by SEQ ID NO: 5 and further preferably confers one or more enhanced yield-related traits relative to control plants;
(iv) a nucleic acid having, in increasing order of preference at least 30 %, 31 %, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with any of the nucleic acid sequences of table A and further preferably conferring one or more enhanced yield-related traits relative to control plants;
(v) a nucleic acid molecule which hybridizes with a nucleic acid molecule of (i) to (iv) under stringent hybridization conditions and preferably confers one or more enhanced yield-related traits relative to control plants; and
(vi) a nucleic acid encoding a HvRAF-like polypeptide having, in increasing order of preference, at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 5 and any of the other amino acid sequences in Table A and preferably conferring one or more enhanced yield-related traits relative to control plants.

According to a further embodiment of the present invention, there is also provided an isolated polypeptide selected from the group consisting of:

(i) an amino acid sequence represented by SEQ ID NO: 5;
(ii) an amino acid sequence having, in increasing order of preference, at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 5 and any of the other amino acid sequences in Table A and preferably conferring one or more enhanced yield-related traits relative to control plants; and
(iii) derivatives of any of the amino acid sequences given in (i) or (ii) above.

Nucleic acid variants may also be useful in practising the methods of the invention. Examples of such variants include nucleic acids encoding homologues and derivatives of any one of the amino acid sequences given in Table A of the Examples section, the terms "homologue" and "derivative" being as defined herein. Also useful in the methods, constructs, plants, harvestable parts and products of the invention are nucleic acids encoding homologues and derivatives of orthologues or paralogues of any one of the amino acid sequences given in Table A of the Examples section. Homologues and derivatives useful in the methods of the present invention have substantially the same biological and functional activity as the unmodified protein from which they are derived. Further variants useful in practising the methods of the invention are variants in which codon usage is optimised or in which miRNA target sites are removed.

Further nucleic acid variants useful in practising the methods of the invention include portions of nucleic acids encoding HvRAF-like polypeptides, nucleic acids hybridising to nucleic acids encoding HvRAF-like polypeptides, splice variants of nucleic acids encoding HvRAF-like polypeptides, allelic variants of nucleic acids encoding HvRAF-like polypeptides and variants of nucleic acids encoding HvRAF-like polypeptides obtained by gene shuffling. The terms hybridising sequence, splice variant, allelic variant and gene shuffling are as described herein.

Nucleic acids encoding HvRAF-like polypeptides need not be full-length nucleic acids, since performance of the methods of the invention does not rely on the use of full-length nucleic acid sequences. According to the present invention, there is provided a method for enhancing one or more yield-related traits in plants, comprising introducing, preferably by recombinant methods, and expressing in a plant a portion of any one of the nucleic acid sequences given in Table A of the Examples section, or a portion of a nucleic acid encoding an orthologue, paralogue or homologue of any of the amino acid sequences given in Table A of the Examples section.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods, constructs, plants, harvestable parts and products of the invention, encode a HvRAF-like polypeptide as defined herein or at least part thereof, and have substantially the same biological activity as the amino acid sequences given in Table A of the Examples section. Preferably, the portion is a portion of any one of the nucleic acids given in Table A of the Examples section, or is a portion of a nucleic acid encoding an orthologue or paralogue of any one of the amino acid sequences given in Table A of the Examples section. Preferably the portion is at least 500, 550, 600, 650, 700, 750, 800, 850, 900, 950, 1000, 1050 consecutive nucleotides in length, the consecutive nucleotides being of any one of the nucleic acid sequences given in Table A of the Examples section, or of a nucleic acid encoding an orthologue or paralogue of any one of the amino acid sequences given in Table A of the Examples section. Most preferably the portion is a portion of the nucleic acid of SEQ ID NO: 1 or of SEQ ID NO: 4. Preferably, the portion encodes a fragment of an amino acid sequence which comprises one or more of motifs 1, 2, 3 and 4, and/or has transcription factor biological activity, and/or has at least 30% sequence identity to SEO ID NO: 5.

Another nucleic acid variant useful in the methods, constructs, plants, harvestable parts and products of the invention is a nucleic acid capable of hybridising, under reduced stringency conditions, preferably under stringent conditions, with a nucleic acid encoding a HvRAF-like polypeptide as defined herein, or with a portion as defined herein. According to the present invention, there is provided a method for enhancing one or more yield-related traits in plants, comprising introducing, preferably by recombinant methods, and expressing in a plant a nucleic acid capable of hybridizing to the complement of a nucleic acid encoding any one of the proteins given in Table A of the Examples section, or to the complement of a nucleic acid encoding an orthologue, paralogue or homologue of any one of the proteins given in Table A.

Hybridising sequences useful in the methods, constructs, plants, harvestable parts and products of the invention encode a HvRAF-like polypeptide as defined herein, having substantially the same biological activity as the amino acid sequences given in Table A of the Examples section. Preferably, the hybridising sequence is capable of hybridising to the complement of a nucleic acid encoding any one of the proteins given in Table A of the Examples section, or to a portion of any of these sequences, a portion being as defined herein, or the hybridising sequence is capable of hybridising to the complement of a nucleic acid encoding an orthologue or paralogue of any one of the amino acid sequences given in Table A of the Examples section. Most preferably, the hybridising sequence is capable of hybridising to the complement of a nucleic acid encoding the polypeptide as represented by SEQ ID NO: 5 or to a portion thereof. In one embodiment, the hybridization conditions are of medium stringency, preferably of high stringency, as defined herein.

Preferably, the hybridising sequence encodes a polypeptide with an amino acid sequence which comprises one or more of motifs 1, 2, 3 and 4, and/or has transcription factor biological activity, and/or has at least 30% sequence identity to SEQ ID NO: 5.

In another embodiment, there is provided a method for enhancing one or more yield-related traits in plants, comprising introducing, preferably by recombinant methods, and expressing in a plant a splice variant of a nucleic acid encoding any one of the proteins given in Table A of the Examples section, or a splice variant of a nucleic acid encoding an orthologue, paralogue or homologue of any of the amino acid sequences given in Table A of the Examples section.

Preferred splice variants are splice variants of a nucleic acid represented by SEQ ID NO: 1 or SEQ ID NO: 4, or a splice variant of a nucleic acid encoding an orthologue or paralogue of SEQ ID NO: 5. Preferably, the amino acid sequence encoded by the splice variant comprises one or more of motifs 1, 2, 3 and 4, and/or has transcription factor biological activity, and/or has at least 30% sequence identity to SEQ ID NO: 5.

In yet another embodiment, there is provided a method for enhancing one or more yield-related traits in plants, comprising introducing, preferably by recombinant methods, and expressing in a plant an allelic variant of a nucleic acid encoding any one of the proteins given in Table A of the Examples section, or comprising introducing, preferably by recombinant methods, and expressing in a plant an allelic variant of a nucleic acid encoding an orthologue, paralogue or homologue of any of the amino acid sequences given in Table A of the Examples section.

The polypeptides encoded by allelic variants useful in the methods of the present invention have substantially the same biological activity as the HvRAF-like polypeptide of SEQ ID NO: 5 and any of the amino acid sequences depicted in Table A of the Examples section. Allelic variants exist in nature, and encompassed within the methods of the present invention is the use of these natural alleles. Preferably, the allelic variant is an allelic variant of SEQ ID NO: 1, SEQ ID NO: 4 or an allelic variant of a nucleic acid encoding an orthologue or paralogue of SEQ ID NO: 5. Preferably, the amino acid sequence encoded by the allelic variant comprises one or more of motifs 1, 2, 3 and 4, and/or has transcription factor biological activity, and/or has at least 30% sequence identity to SEQ ID NO: 5.

In yet another embodiment, there is provided a method for enhancing one or more yield-related traits in plants, comprising introducing, preferably by recombinant methods, and expressing in a plant a variant of a nucleic acid encoding any one of the proteins given in Table A of the Examples section, or comprising introducing, preferably by recombinant methods, and expressing in a plant a variant of a nucleic acid encoding an orthologue, paralogue or homologue of any of the amino acid sequences given in Table A of the Examples section, which variant nucleic acid is obtained by gene shuffling.

Preferably, the amino acid sequence encoded by the variant nucleic acid obtained by gene shuffling comprises one or more of motifs 1, 2, 3 and 4, and/or has transcription factor biological activity, and/or has at least 30% sequence identity to SEQ ID NO: 5.

Furthermore, nucleic acid variants may also be obtained by site-directed mutagenesis. Several methods are available to achieve site-directed mutagenesis, the most common being PCR based methods (Current Protocols in Molecular Biology. Wiley Eds.). HvRAF-like polypeptides differing from the sequence of SEQ ID NO: 5 by one or several amino acids (substitution(s), insertion(s) and/or deletion(s) as defined herein) may equally be useful to increase the yield of plants in the methods and constructs and plants of the invention.

Nucleic acids encoding HvRAF-like polypeptides may be derived from any natural or artificial source. The nucleic acid may be modified from its native form in composition and/or genomic environment through deliberate human manipulation. Preferably the HvRAF-like polypeptide-encoding nucleic acid is from a plant, further preferably from a monocotyledonous plant, more preferably from the family Poaceae, most preferably the nucleic acid is from *Hordeum vulgare.*

In another embodiment the present invention extends to recombinant chromosomal DNA comprising a nucleic acid sequence useful in the methods of the invention, wherein said nucleic acid is present in the chromosomal DNA as a result of recombinant methods, but is not in its natural genetic environment. In a further embodiment the recombinant chromosomal DNA of the invention is comprised in a plant cell. DNA comprised within a cell, particularly a cell with cell walls like a plant cell, is better protected from degradation than a bare nucleic acid sequence. The same holds true for a DNA construct comprised in a host cell, for example a plant cell.

Performance of the methods of the invention gives plants having one or more enhanced yield-related traits. In particular performance of the methods of the invention gives plants having increased early vigour and/or increased yield, especially increased biomass and/or increased seed yield relative to control plants. The terms "early vigour" "yield" and "seed yield" are described in more detail in the "definitions" section herein.

The present invention thus provides a method for increasing yield-related traits, especiallybiomass, seed yield and/or early vigour of plants, relative to control plants, which method comprises modulating expression in a plant of a nucleic acid encoding a HvRAF-like polypeptide as defined herein.

According to a preferred feature of the present invention, performance of the methods of the invention gives plants having an increased growth rate relative to control plants. Therefore, according to the present invention, there is provided a method for increasing the growth rate of plants, which method comprises modulating expression in a plant of a nucleic acid encoding a HvRAF-like polypeptide as defined herein.

Performance of the methods of the invention results in plants having increased seed yield relative to the seed yield of control plants, and/or increased aboveground biomass, in particular stem biomass relative to the aboveground biomass, and in particular stem biomass of control plants, and/or increased root biomass relative to the root biomass of control plants and/or increased beet biomass relative to the beet biomass of control plants. Moreover, it is particularly contemplated that the sugar content (in particular the sucrose content) in the above ground parts, particularly stem (in particular of sugar cane plants) and/or in the belowground parts, in particular in roots including taproots and tubers and/or in beets (in particular in sugar beets) is increased relative to the sugar content (in particular the sucrose content) in corresponding part(s) of the control plant.

Performance of the methods of the invention gives plants grown under non-stress conditions or under mild drought conditions, and/or under field conditions increased yield-related traits relative to control plants grown under comparable conditions. Therefore, according to the present invention, there is provided a method for increasing yield-related traits in plants grown under non-stress conditions or under mild drought conditions, which method comprises modulating expression in a plant of a nucleic acid encoding a HvRAF-like polypeptide.

Performance of the methods of the invention gives plants grown under conditions of drought, increased yield-related traits relative to control plants grown under comparable conditions. Therefore, according to the present invention, there is provided a method for increasing yield-related traits in plants grown under conditions of drought which method comprises modulating expression in a plant of a nucleic acid encoding a HvRAF-like polypeptide.

Performance of the methods of the invention gives plants grown under conditions of nutrient deficiency, particularly under conditions of nitrogen deficiency, increased yield-related traits relative to control plants grown under comparable conditions. Therefore, according to the present invention, there is provided a method for increasing yield-related traits in plants grown under conditions of nutrient deficiency, which method comprises modulating expression in a plant of a nucleic acid encoding a HvRAF-like polypeptide.

Performance of the methods of the invention gives plants grown under conditions of salt stress, increased yield-related traits relative to control plants grown under comparable conditions. Therefore, according to the present invention, there is provided a method for increasing yield-related traits in plants grown under conditions of salt stress, which method comprises modulating expression in a plant of a nucleic acid encoding a HvRAF-like polypeptide.

The invention also provides genetic constructs and vectors to facilitate introduction and/or expression in plants of nucleic acids encoding HvRAF-like polypeptides. The gene constructs may be inserted into vectors, which may be commercially available, suitable for transforming into plants or host cells and suitable for expression of the gene of interest in the transformed cells. The invention also provides use of a gene construct as defined herein in the methods of the invention.

More specifically, the present invention provides a construct comprising:

(a) an isolated nucleic acid encoding a HvRAF-like polypeptide as defined above;
(b) one or more control sequences capable of driving expression of the nucleic acid sequence of (a); and optionally
(c) a transcription termination sequence.

Preferably, the nucleic acid encoding a HvRAF-like polypeptide is as defined above. The term "control sequence" and "termination sequence" are as defined herein.

In particular the genetic construct of the invention is a plant expression construct, i.e. a genetic construct that allows for the expression of the nucleic acid encoding a HvRAF-like polypeptide in a plant, plant cell or plant tissue after the construct has been introduced into this plant, plant cell or plant tissue, preferably by recombinant means. The plant expression construct may for example comprise said nucleic acid encoding a HvRAF-like polypeptide in functional linkage to a promoter and optionally other control sequences controlling the expression of said nucleic acid in one or more plant cells, wherein the promoter and optional the other control sequences are not natively found in functional linkage to said nucleic acid. In a preferred embodiment the control sequence(s) including the promoter result in overexpression of said nucleic acid when the construct of the invention has been introduced into a plant, plant cell or plant tissue.

The genetic construct of the invention may be comprised in a host cell - for example a plant cell - seed, agricultural product or plant. Plants or host cells are transformed with a genetic construct such as a vector or an expression cassette comprising any of the nucleic acids described above. Thus the invention furthermore provides plants or host cells transformed with a construct as described above. In particular, the invention provides plants transformed with a construct as described above, which plants have increased yield-related traits as described herein.

In one embodiment the genetic construct of the invention confers increased yield or yield related traits to a plant when it has been introduced into said plant, which plant expresses the nucleic acid encoding the HvRAF-like polypeptide comprised in the genetic construct and preferably resulting in increased abundance of the HvRAF-like polypeptide. In another embodiment the genetic construct of the invention confers increased yield or yield related traits to a plant comprising plant cells in which the construct has been introduced, which plant cells express the *HvRAF-like* nucleic acid comprised in the genetic construct.

The promoter in such a genetic construct may be a promoter not native to the nucleic acid described above, i.e. a promoter different from the promoter regulating the expression of the *HvRAF-like* nucleic acid in its native surrounding. In a particular embodiment the nucleic acid encoding the HvRAF-like polypeptide useful in the methods, constructs, plants, harvestable parts and products of the invention is in functional linkage to a promoter resulting in the expression of the *HvRAF-like* nucleic acid in

- aboveground biomass preferably the leaves and shoot, more preferably the stem, of monocot plants, preferably Poaceae plants, more preferably Saccharum species plants, and/or
- leaves, belowground biomass and/or root biomass, preferably tubers, taproots and/or beet organs, more preferably taproot and beet organs of dicot plants, more preferably Solanaceae and/or Beta species plants.

The expression cassette or the genetic construct of the invention may be comprised in a host cell, plant cell, seed, agricultural product or plant.

The skilled artisan is well aware of the genetic elements that must be present on the genetic construct in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence, but preferably the promoter is of plant origin. A constitutive promoter is particularly useful in the methods. See the "Definitions" section herein for definitions of the various promoter types. Also useful in the methods of the invention is a root-specific promoter.

The constitutive promoter is preferably a ubiquitous constitutive promoter of medium strength. More preferably it is a plant derived promoter, e.g. a promoter of plant chromosomal origin, such as a GOS2 promoter or a promoter of substantially the same strength and having substantially the same expression pattern (a functionally equivalent promoter), more preferably the promoter is the promoter GOS2 promoter from rice. Further preferably the constitutive promoter is represented by a nucleic acid sequence substantially similar to SEQ ID NO: 35, most preferably the constitutive promoter is as represented by SEQ ID NO: 35. See the "Definitions" section herein for further examples of constitutive promoters.

According to another preferred embodiment of the invention, the nucleic acid encoding a HvRAF-like polypeptide is operably linked to a root-specific promoter. The root-specific promoter is preferably an RCc3 promoter (Plant Mol Biol. 1995 Jan;27(2):237-48) or a promoter of substantially the same strength and having substantially the same expression pattern (a functionally equivalent promoter), more preferably the RCc3 promoter is from rice, further preferably the RCc3 promoter is represented by a nucleic acid sequence substantially similar to SEQ ID NO: 36, most preferably the promoter is as represented by SEQ ID NO: 36. Examples of other root-specific promoters which may also be used to perform the methods of the invention are shown in Table 2b in the "Definitions" section.

It should be clear that the applicability of the present invention is not restricted to the HvRAF-like polypeptide-encoding nucleic acid represented by SEQ ID NO: 1 and/or 4, nor is the applicability of the invention restricted to the rice GOS2 promoter when expression of a HvRAF-like polypeptide-encoding nucleic acid is driven by a constitutive promoter.

Yet another embodiment relates to genetic constructs useful in the methods, constructs, plants, harvestable parts and products of the invention wherein the genetic construct comprises the *HvRAF-like* nucleic acid of the invention functionally linked a promoter as disclosed herein above and further functionally linked to one or more of

1) nucleic acid expression enhancing nucleic acids (NEENAs):

a) as disclosed in the international patent application published as WO2011/023537 in table 1 on page 27 to page 28 and/or SEQ ID NO: 1 to 19 and/or as defined in items i) to vi) of claim 1 of said international application which NEENAs are herewith incorporated by reference; and/or

b) as disclosed in the international patent application published as WO2011/023539 in table 1 on page 27 and/or SEQ ID NO: 1 to 19 and/or as defined in items i) to vi) of claim 1 of said international application which NEENAs are herewith incorporated by reference; and/or

c) as contained in or disclosed in:

i) the European priority application filed on 05 July 2011 as EP 11172672.5 in table 1 on page 27 and/or SEQ ID NO: 1 to 14937, preferably SEQ ID NO: 1 to 5, 14936 or 14937, and/or as defined in items i) to v) of claim 1 of said European priority application which NEENAs are herewith incorporated by reference; and/or

ii) the European priority application filed on 06 July 2011 as EP 11172825.9 in table 1 on page 27 and/or SEQ ID NO: 1 to 65560, preferably SEQ ID NO: 1 to 3, and/or as defined in items i) to v) of claim 1 of said European priority application which NEENAs are herewith incorporated by reference;

and/or

d) equivalents having substantially the same enhancing effect;

and/or

2) functionally linked to one or more Reliability Enhancing Nucleic Acid (RENA) molecule

a) as contained in or disclosed in the European priority application filed on 15 September 2011 as EP 11181420.8 in table 1 on page 26 and/or SEQ ID NO: 1 to 16 or 94 to 116666, preferably SEQ ID NO: 1 to 16, and/or as defined in point i) to v) of item a) of claim 1 of said European priority application which RENA molecule(s) are herewith incorporated by reference; or

b) equivalents having substantially the same enhancing effect.

A preferred embodiment of the invention relates to a nucleic acid molecule useful in the methods, constructs, plants, harvestable parts and products of the invention and encoding a HvRAF-like polypeptide of the invention under the control of a promoter as described herein above, wherein the NEENA, RENA and/or the promoter is heterologous to the *HvRAF-like* nucleic acid molecule of the invention.

Optionally, one or more terminator sequences may be used in the construct introduced into a plant. Those skilled in the art will be aware of terminator sequences that may be suitable for use in performing the invention. Preferably, the construct comprises an expression cassette comprising a GOS2 promoter, operably linked to the nucleic acid encoding the HvRAF-like polypeptide. More preferably, the construct furthermore comprises a zein terminator (t-zein) linked to the 3' end of the HvRAF-like coding sequence. Furthermore, one or more sequences encoding selectable markers may be present on the construct introduced into a plant.

According to a preferred feature of the invention, the modulated expression is increased expression. Methods for increasing expression of nucleic acids or genes, or gene products, are well documented in the art and examples are provided in the definitions section.

As mentioned above, a preferred method for modulating expression of a nucleic acid encoding a HvRAF-like polypeptide is by introducing, preferably by recombinant methods, and expressing in a plant a nucleic acid encoding a HvRAF-like polypeptide; however the effects of performing the method, i.e. enhancing one or more yield-related traits may also be achieved using other well-known techniques, including but not limited to T-DNA activation tagging, TILLING, homologous recombination. A description of these techniques is provided in the definitions section.

The invention also provides a method for the production of transgenic plants having one or more enhanced yield-related traits relative to control plants, comprising introduction and expression in a plant of any nucleic acid encoding a HvRAF-like polypeptide as defined herein.

More specifically, the present invention provides a method for the production of transgenic plants having one or more enhanced yield-related traits, particularly increased seed yield, which method comprises:

(i) introducing and expressing in a plant or plant cell a recombinant HvRAF-like polypeptide-encoding nucleic acid or a genetic construct comprising a HvRAF-like polypeptide-encoding nucleic acid; and
(ii) cultivating the plant cell under conditions promoting plant growth and development.

Preferably, the introduction of the HvRAF-like polypeptide-encoding nucleic acid is by recombinant methods.

The nucleic acid of (i) may be any of the nucleic acids capable of encoding a HvRAF-like polypeptide as defined herein.

Cultivating the plant cell under conditions promoting plant growth and development, may or may not include regeneration and/or growth to maturity. Accordingly, in a particular embodiment of the invention, the plant cell transformed by the method according to the invention is regenerable into a transformed plant. In another particular embodiment, the plant cell transformed by the method according to the invention is not regenerable into a transformed plant, i.e. cells that are not capable to regenerate into a plant using cell culture techniques known in the art. While plants cells generally have the characteristic of totipotency, some plant cells cannot be used to regenerate or propagate intact plants from said cells. In one embodiment of the invention the plant cells of the invention are such cells. In another embodiment the plant cells of the invention are plant cells that do not sustain themselves in an autotrophic way. One example are plant cells that do not sustain themselves through photosynthesis by synthesizing carbohydrate and protein from such inorganic substances as water, carbon dioxide and mineral salt.

The nucleic acid may be introduced directly into a plant cell or into the plant itself (including introduction into a tissue, organ or any other part of a plant). According to a preferred feature of the present invention, the nucleic acid is preferably introduced into a plant or plant cell by transformation. The term "transformation" is described in more detail in the "definitions" section herein.

In one embodiment the method for the production of a transgenic sugarcane plant, a transgenic part thereof, or a transgenic plant cell thereof, having one or more enhanced yield-related traits relative to control plants, comprises the step of harvesting setts from the transgenic plant and planting the setts and growing the setts to plants, wherein the setts comprises the exogenous nucleic acid encoding the HvRAF-like polypeptide and the promoter sequence operably linked thereto.

In one embodiment the present invention extends to any plant cell or plant produced by any of the methods described herein, and to all plant parts and propagules thereof.

The present invention encompasses plants or parts thereof (including seeds) obtainable by the methods according to the present invention. The plants or plant parts or plant cells comprise a nucleic acid transgene encoding a HvRAF-like polypeptide as defined above, preferably in a genetic construct such as an expression cassette. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

In a further embodiment the invention extends to seeds recombinantly comprising the expression cassettes of the invention, the genetic constructs of the invention, or the nucleic acids encoding the HvRAF-like and/or the HvRAF-like polypeptides as described above.

The invention also includes host cells containing an isolated nucleic acid encoding a HvRAF-like polypeptide as defined above. In one embodiment host cells according to the invention are plant cells, yeasts, bacteria or fungi. Host plants for the nucleic acids, construct, expression cassette or the vector used in the method according to the invention are, in principle, advantageously all plants which are capable of synthesizing the polypeptides used in the inventive method. In a particular embodiment the plant cells of the invention overexpress the nucleic acid molecule of the invention.

The methods of the invention are advantageously applicable to any plant, in particular to any plant as defined herein. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to an embodiment of the present invention, the plant is a crop plant. Examples of crop plants include but are not limited to chicory, carrot, cassava, trefoil, soybean, beet, sugar beet, sunflower, canola, alfalfa, rapeseed, linseed, cotton, tomato, potato and tobacco. According to another embodiment of the present invention, the plant is a monocotyledonous plant. Examples of monocotyledonous plants include sugarcane. According to another embodiment of the present invention, the plant is a cereal. Examples of cereals include rice, maize, wheat, barley, millet, rye, triticale, sorghum, emmer, spelt, einkorn, teff, milo and oats. In a particular embodiment the plants of the invention, or used in the methods of the invention, are selected from the group consisting of maize, wheat, rice, soybean, cotton, oilseed rape including canola, sugarcane, sugar beet and alfalfa. Advantageously the methods of the invention are more efficient than the known methods, because the plants of the invention have increased yield and/or tolerance to an environmental stress compared to control plants used in comparable methods.

The invention also extends to harvestable parts of a plant such as, but not limited to seeds, leaves, fruits, flowers, stems, roots, rhizomes, tubers and bulbs, which harvestable parts comprise a recombinant nucleic acid encoding a HvRAF-like polypeptide. In particular, such harvestable parts are roots such as taproots, rhizomes, fruits, stems, beets, tubers, bulbs, leaves, flowers and / or seeds. In one embodiment harvestable parts are stem cuttings (like setts of sugar cane).

The invention furthermore relates to products derived or produced, preferably directly derived or directly produced, from a harvestable part of such a plant, such as dry pellets, pressed stems, meal or powders, oil, fat and fatty acids, carbohydrates, sap, juice or proteins. Preferred carbohydrates are starch, cellulose or sugars, preferably sucrose. Also preferred products are residual dry fibers, e.g., of the stem (like bagasse from sugar cane after cane juice removal), molasse, or filtercake, preferably from sugar cane. In one embodiment the product comprises a recombinant nucleic acid encoding a HvRAF-like polypeptide and/or a recombinant HvRAF-like polypeptide for example as an indicator of the particular quality of the product.

The invention also includes methods for manufacturing a product comprising a) growing the plants of the invention and b) producing said product from or by the plants of the invention or parts thereof, including stem, root, beet and/or seeds. In a further embodiment the methods comprise the steps of a) growing the plants of the invention, b) removing the harvestable parts as described herein from the plants and c) producing said product from, or with the harvestable parts of plants according to the invention. In one embodiment, the product is produced from the stem of the transgenic plant.

In a further embodiment the products produced by the manufacturing methods of the invention are plant products such as, but not limited to, a foodstuff, feedstuff, a food supplement, feed supplement, fiber, cosmetic or pharmaceutical. In another embodiment the methods for production are used to make agricultural products such as, but not limited to, plant extracts, proteins, amino acids, carbohydrates, fats, oils, polymers, vitamins, and the like.

In yet another embodiment the polynucleotides or the polypeptides or the constructs of the invention are comprised in an agricultural product. In a particular embodiment the nucleic acid sequences and protein sequences of the invention may be used as product markers, for example where an agricultural product was produced by the methods of the invention. Such a marker can be used to identify a product to have been produced by an advantageous process resulting not only in a greater efficiency of the process but also improved quality of the product due to increased quality of the plant material and harvestable parts used in the process. Such markers can be detected by a variety of methods known in the art, for example but not limited to PCR based methods for nucleic acid detection or antibody based methods for protein detection.

The present invention also encompasses use of nucleic acids encoding HvRAF-like polypeptides as described herein and use of these HvRAF-like polypeptides in enhancing any of the aforementioned yield-related traits in plants. For example, nucleic acids encoding HvRAF-like polypeptide described herein, or the HvRAF-like polypeptides themselves, may find use in breeding programmes in which a DNA marker is identified which may be genetically linked to a HvRAF-like polypeptide-encoding gene. The nucleic acids/genes, or the HvRAF-like polypeptides themselves may be used to define a molecular marker. This DNA or protein marker may then be used in breeding programmes to select plants having one or more enhanced yield-related traits as defined herein in the methods of the invention. Furthermore, allelic variants of a HvRAF-like polypeptide-encoding nucleic acid/gene may find use in marker-assisted breeding programmes. Nucleic acids encoding HvRAF-like polypeptides may also be used as probes for genetically and physically mapping the genes that they are a part of, and as markers for traits linked to those genes. Such information may be useful in plant breeding in order to develop lines with desired phenotypes.

Moreover, the present invention relates to the following specific embodiments, wherein the expression "as defined in item X" is meant to direct the artisan to apply the definition as disclosed in item X. For example, "a nucleic acid as defined in item 1" has to be understood such that the definition of the nucleic acid as in item 1 is to be applied to the nucleic acid. In consequence the term "as defined in item" may be replaced with the corresponding definition of that item:

**Items:**

1. A method for enhancing one or more yield-related traits in plants relative to control plants, comprising modulating expression in a plant of a nucleic acid encoding a HvRAF-like polypeptide, wherein said HvRAF-like polypeptide comprises having in increasing order of preference at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more sequence identity to any one or more of the motifs 1 to 4 as provided in SEQ ID NO: 31 to SEQ ID NO: 34.
2. Method according to item 1, wherein said modulated expression is effected by introducing and expressing in a plant said nucleic acid encoding said HvRAF-like polypeptide.
3. Method according to item 1 or 2, wherein said one or more enhanced yield-related traits comprise increased yield and/or early vigour relative to control plants, and preferably comprise increased biomass and/or increased seed yield relative to control plants.
4. Method according to any one of items 1 to 3, wherein said one or more enhanced yield-related traits are obtained under non-stress conditions.
5. Method according to any one of items 1 to 3, wherein said one or more enhanced yield-related traits are obtained under conditions of drought stress, salt stress or nitrogen deficiency.
6. Method according to any of items 1 to 5, wherein said HvRAF-like polypeptide comprises one or more of the following motifs:

   (i) Motif 1 represented by SEQ ID NO: 31,
   (ii) Motif 2 represented by SEQ ID NO: 32,
   (iii) Motif 3 represented by SEQ ID NO: 33,
   (iv) Motif 4 represented by SEQ ID NO: 34.

7. Method according to any one of items 1 to 6, wherein said nucleic acid encoding an HvRAF-like polypeptide is of plant origin, preferably from a dicotyledonous plant, further preferably from the family Poaceae, more preferably from the genus *Hordeum,* most preferably from *Hordeum vulgare.*
8. Method according to any one of items 1 to 7, wherein said nucleic acid encoding a HvRAF-like polypeptide encodes any one of the polypeptides listed in Table A or is a portion of such a nucleic acid, or a nucleic acid capable of hybridising with such a nucleic acid.
9. Method according to any one of items 1 to 8, wherein said nucleic acid sequence encodes an orthologue or paralogue of any of the polypeptides given in Table A.
10. Method according to any one of items 1 to 9, wherein said nucleic acid encodes the polypeptide represented by SEQ ID NO: 5.
11. Method according to any one of items 1 to 10, wherein said nucleic acid is operably linked to a constitutive promoter of plant origin, preferably to a medium strength constitutive promoter of plant origin, more preferably to a GOS2 promoter, most preferably to a GOS2 promoter from rice.
12. Plant, or part thereof, or plant cell, obtainable by a method according to any one of items 1 to 11, wherein said plant, plant part or plant cell comprises a recombinant nucleic acid encoding a HvRAF-like polypeptide as defined in any of items 1 and 6 to 10.
13. Construct comprising:

   (i) nucleic acid encoding an HvRAF-like polypeptide as defined in any of items 1 and 6 to 10;
   (ii) one or more control sequences capable of driving expression of the nucleic acid sequence of (i); and optionally
   (iii) a transcription termination sequence.

14. Construct according to item 13, wherein one of said control sequences is a constitutive promoter of plant origin, preferably to a medium strength constitutive promoter of plant origin, more preferably to a GOS2 promoter, most preferably to a GOS2 promoter from rice.

15. Use of a construct according to item 13 or 14 in a method for making plants having one or more enhanced yield-related traits, preferably increased yield and/or early vigour relative to control plants, and more preferably increased seed yield and/or increased biomass relative to control plants.

16. Plant, plant part or plant cell transformed with a construct according to item 13 or 14.

17. Method for the production of a transgenic plant having one or more enhanced yield-related traits relative to control plants, preferably increased yield and/or early vigour relative to control plants, and more preferably increased seed yield and/or increased biomass relative to control plants, comprising:

(i) introducing and expressing in a plant cell or plant a nucleic acid encoding an HvRAF-like polypeptide as defined in any of items 1 and 6 to 10; and
(ii) cultivating said plant cell or plant under conditions promoting plant growth and development.

18. Transgenic plant having one or more enhanced yield-related traits relative to control plants, preferably increased yield and/or early vigour relative to control plants, and more preferably increased seed yield and/or increased biomass, resulting from modulated expression of a nucleic acid encoding an HvRAF-like polypeptide as defined in any of items 1 and 6 to 10 or a transgenic plant cell derived from said transgenic plant.

19. Transgenic plant according to item 12, 16 or 18, or a transgenic plant cell derived therefrom, wherein said plant is a crop plant, such as beet, sugarbeet or alfalfa; or a monocotyledonous plant such as sugarcane; or a cereal, such as rice, maize, wheat, barley, millet, rye, triticale, sorghum, emmer, spelt, einkorn, teff, milo or oats.

20. Harvestable parts of a plant according to item 19, wherein said harvestable parts are preferably shoot and/or rootbiomass and/or seeds.

21. Products derived from a plant according to item 19 and/or from harvestable parts of a plant according to item 20.

22. Use of a nucleic acid encoding an HvRAF-like polypeptide as defined in any of items 1 and 6 to 10 for enhancing one or more yield-related traits in plants relative to control plants, preferably for increasing yield and/or early vigour , and more preferably for increasing seed yield and/or for increasing biomass in plants relative to control plants.

23. A method for manufacturing a product, comprising the steps of growing the plants according to item 12, 16, 19 or 20 and producing said product from or by said plants; or parts thereof, including seeds.

24. An isolated nucleic acid molecule selected from the group consisting of:

(i) a nucleic acid represented by SEQ ID NO: 1 or SEQ ID NO: 4;
(ii) the complement of a nucleic acid represented by SEQ ID NO: 1 or SEQ ID NO: 4;
(iii) a nucleic acid encoding a HvRAF-like polypeptide having in increasing order of preference at least 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 5 and additionally or alternatively comprising one or more motifs having in increasing order of preference at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% or more sequence identity to any one or more of the motifs 1 to 4 as provided in SEQ ID NO: 31 to SEQ ID NO: 34, and further preferably conferring one or more enhanced yield-related traits relative to control plants; and
(iv) a nucleic acid molecule which hybridizes with a nucleic acid molecule of (i) to (iii) under high stringency hybridization conditions and preferably confers one or more enhanced yield-related traits relative to control plants.

25. An isolated polypeptide encoded by the isolated nucleotide according to item 24.

26. An expression cassette comprising the isolated nucleic acid molecule according to item 24 and a promoter which is operably linked to said isolated nucleic acid molecule, and, optionally, a transcription termination sequence.

27. An expression cassette according to item 26, wherein said promoter is heterologous with respect to said nucleic acid molecule.

28. An expression vector comprising the expression cassette of item 26 or 27, in particular a T-DNA vector.

29. A host cell comprising the isolated nucleic acid molecule of item 24, the expression cassette of items 26 or 27, or the vector of item 28.

30. A host cell according to item 29, wherein said host cell is an Agrobacterium cell or a plant cell.

31. A plant comprising the isolated nucleic acid molecule of item 24, the expression cassette of items 26 or 27, or the vector of item 28.

32. Method according to any one of items 1 to 11, wherein said polypeptide is encoded by a nucleic acid molecule comprising a nucleic acid molecule selected from the group consisting of:

(i) a nucleic acid represented by SEQ ID NO: 1 or SEQ ID NO: 4;
(ii) the complement of a nucleic acid represented by SEQ ID NO: 1 or SEQ ID NO: 4;
(iii) a nucleic acid encoding the polypeptide as represented by SEQ ID NO: 5, and further preferably confers one or more enhanced yield-related traits relative to control plants;
(iv) a nucleic acid having, in increasing order of preference at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with SEQ ID NO: 1 or SEQ ID NO: 4, and further preferably conferring one or more enhanced yield-related traits relative to control plants,
(v) a nucleic acid molecule which hybridizes to the complement of a nucleic acid molecule of (i) to (iv) under stringent hybridization conditions and preferably confers one or more enhanced yield-related traits relative to control plants,
(vi) a nucleic acid encoding said polypeptide having, in increasing order of preference, at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 5 and preferably conferring one or more enhanced yield-related traits relative to control plants; or
(vii) a nucleic acid comprising any combination(s) of features of (i) to (vi) above.

## Description of figures

The present invention will now be described with reference to the following figures in which:

**Figure 1** represents the domain structure of SEQ ID NO: 5 with conserved motifs.
**Figure 2** represents a multiple alignment of various HvRAF-like polypeptides. The asterisks indicate identical amino acids among the various protein sequences, colons represent highly conserved amino acid substitutions, and the dots represent less conserved amino acid substitution; on other positions there is no sequence conservation. These alignments can be used for defining further motifs or signature sequences, when using conserved amino acids.
**Figure 3** shows the MATGAT table of Example 3.

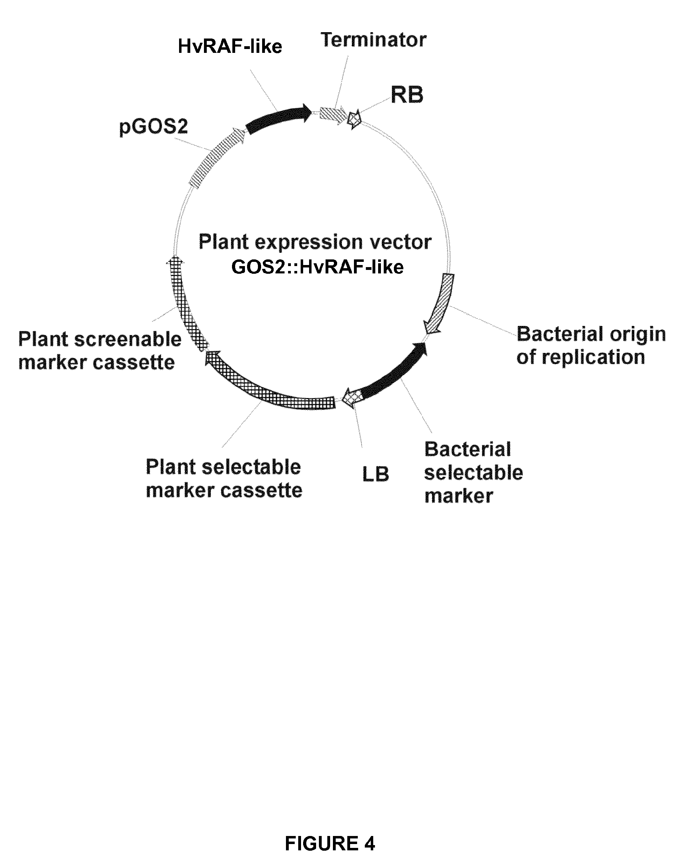

**Figure 4** represents the binary vector used for increased expression in Oryza sativa of a HvRAF-like-encoding nucleic acid under the control of a rice GOS2 promoter (pGOS2).

## Examples

The present invention will now be described with reference to the following examples, which are by way of illustration only. The following examples are not intended to limit the scope of the invention. Unless otherwise indicated, the present invention employs conventional techniques and methods of plant biology, molecular biology, bioinformatics and plant breedings.
DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

Example 1: Identification of sequences related to SEQ ID NO: 1, SEQ ID NO: 4 and SEQ ID NO: 5

Sequences (full length cDNA, ESTs or genomic) related to SEQ ID NO: 1, SEQ ID NO: 4 and SEQ ID NO: 5 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of SEQ ID NO: 1 was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A provides a list of nucleic acid sequences related to SEQ ID NO: 1, SEQ ID NO: 4 and SEQ ID NO: 5.

**Table A:** Examples of HvRAF-like nucleic acids and polypeptides:

| Plant Source | Nucleic acid SEQ ID NO: | Protein SEQ ID NO: |
|---|---|---|
| *Hordeum vulgare* | 1 | na |
| *Hordeum vulgare subsp. vulgare* | 2 | na |
| *Hordeum vulgare* | 3 | na |
| *Hordeum vulgare* | 4 | 5 |
| *Hordeum vulgare* | 6 | 7 |
| *Sorghum bicolor* | 8 | 9 |
| *Oryza sativa Japonica Group* | 10 | 11 |
| *Brachypodium distachyon* | 12 | 13 |
| *Zea mays* | 14 | 15 |
| *Hordeum vulgare var. distichum* | 16 | 17 |
| *Hordeum vulgare* | 18 | 19 |
| *Hordeum vulgare* | 20 | 21 |
| *Triticum aestivum* | 22 | 23 |
| *Zea mays* | 24 | 25 |
| *Zea mays* | 26 | 27 |

Sequences have been tentatively assembled and publicly disclosed by research institutions, such as The Institute for Genomic Research (TIGR; beginning with TA). For instance, the Eukaryotic Gene Orthologs (EGO) database may be used to identify such related sequences, either by keyword search or by using the BLAST algorithm with the nucleic acid sequence or polypeptide sequence of interest. Special nucleic acid sequence databases have been created for particular organisms, e.g. for certain prokaryotic organisms, such as by the Joint Genome Institute. Furthermore, access to proprietary databases, has allowed the identification of novel nucleic acid and polypeptide sequences.

Example 2: Alignment of HvRAF-like polypeptide sequences

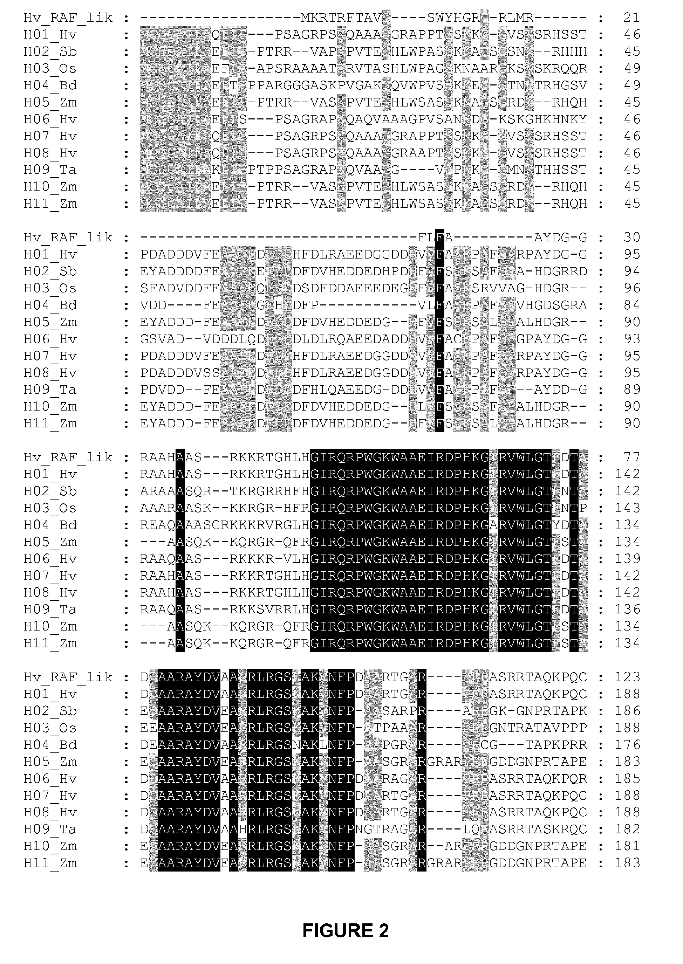

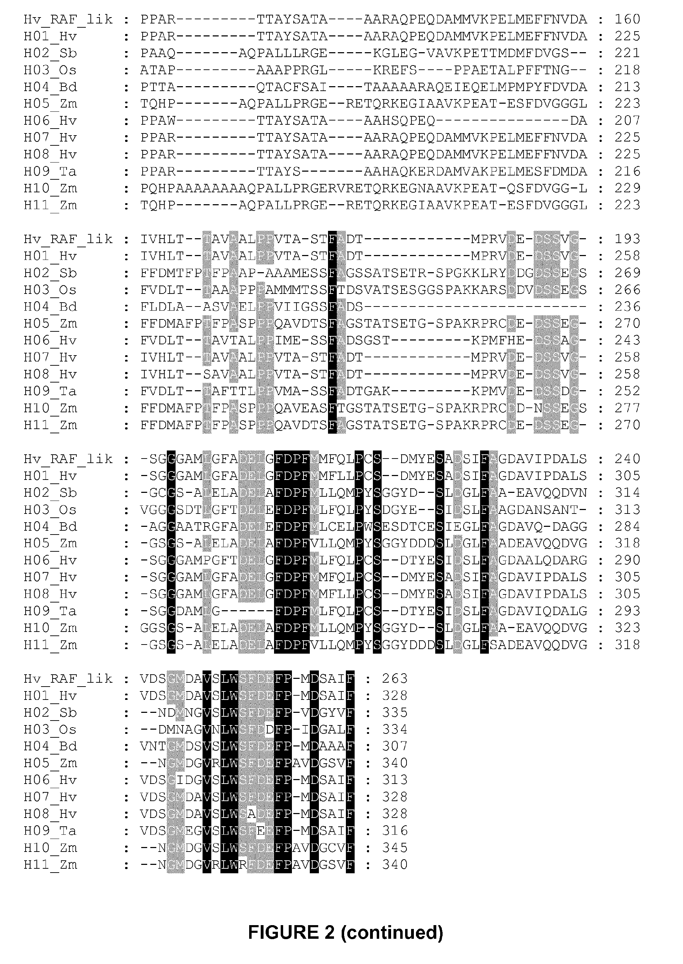

Alignment of the polypeptide sequences was performed using the ClustalW 2.0 algorithm of progressive alignment (Thompson et al. (1997) Nucleic Acids Res 25:4876-4882; Chenna et al. (2003). Nucleic Acids Res 31:3497-3500) with standard setting (slow alignment, similarity matrix: Gonnet, gap opening penalty 10, gap extension penalty: 0.2). Minor manual editing was done to further optimise the alignment. The HvRAF-like polypeptides are aligned in Figure 2.

Example 3: Calculation of global percentage identity between polypeptide sequences

Global percentages of similarity and identity between full length polypeptide sequences useful in performing the methods of the invention were determined using MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT generates similarity/identity matrices for DNA or protein sequences without needing pre-alignment of the data. The program performs a series of pair-wise alignments using the Myers and Miller global alignment algorithm, calculates similarity and identity, and then places the results in a distance matrix.

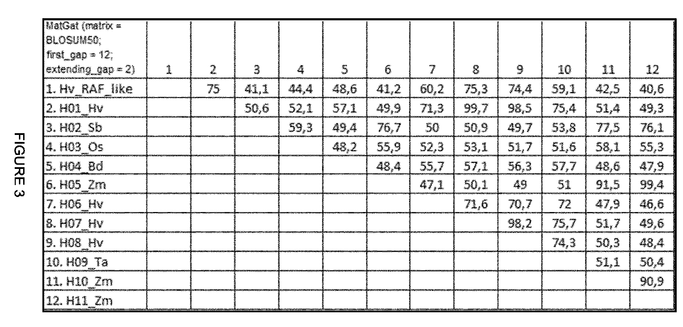

Results of the MatGAT analysis are shown in Figure 3 with global identity percentages over the full length of the polypeptide sequences. The sequence identity (in %) between the HvRAF-like polypeptide sequences useful in performing the methods of the invention is generally higher than 30% compared to SEQ ID NO: 5.

Like for full length sequences, a MATGAT table based on subsequences of a specific domain, may be generated. Based on a multiple alignment of HvRAF-like polypeptides, such as for example the one of Example 2, a skilled person may select conserved sequences and submit as input for a MaTGAT analysis. This approach is useful where overall sequence conservation among HvRAF-like proteins is rather low.

Example 4: Identification of domains comprised in polypeptide sequences useful in performing the methods of the invention

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. InterPro is hosted at the European Bioinformatics Institute in the United Kingdom.

The results of the InterPro scan (InterPro database, release 4.8) of the polypeptide sequence as represented by SEQ ID NO: 5 are presented in Table B.

**Table B:** InterPro scan results (major accession numbers) of the polypeptide sequence as represented by SEQ ID NO: 5.

| Database | Accession number | Accession name | Amino acid coordinates on SEQ ID NO 5 |
|---|---|---|---|
| HMMPfam: | PF00847 | "AP2" | 42 .. 106 |
| Gene3D: | G3DSA:3.30.730.10 | "TF_ERF" | 43 .. 106 |
| BlastProDom: | PD001423 | "TF_ERF" | 51 .. 74 |
| HMMSmart: | SM00380 | "AP2" | 44 .. 107 |
| ProfileScan: | PS51032 | "AP2_ERF" | 44 .. 101 |
| FPrintScan: | PR00367 | "ETHRSPELEMNT" | 45 .. 56; 67 .. 83 |
| superfamily: | SSF54171 | "DNA-binding domain" | 43 .. 104 |
| ScanRegExp: | PS00024 | "HEMOPEXIN" | 247 .. 262 |

In one embodiment a HvRAF-like polypeptide comprises a conserved domain (or motif) with at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to a conserved domain from amino acid 42 to 262 in SEQ ID NO:5).

Example 5: Topology prediction of the HvRAF-like polypeptide sequences

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. For the sequences predicted to contain an N-terminal presequence a potential cleavage site can also be predicted. TargetP is maintained at the server of the Technical University of Denmark.

A number of parameters must be selected before analysing a sequence, such as organism group (non-plant or plant), cutoff sets (none, predefined set of cutoffs, or user-specified set of cutoffs), and the calculation of prediction of cleavage sites (yes or no).

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

Example 6: Cloning of the HvRAF-like encoding nucleic acid sequence

The nucleic acid sequence was amplified by PCR using as template a custom-made *Arabidopsis thaliana* seedlings cDNA library. PCR was performed using a commercially available proofreading Taq DNA polymerase in standard conditions, using 200 ng of template in a 50 μl PCR mix. The primers used were prm08769 (SEQ ID NO: 28; sense, start codon in bold): 5'-GGGGACCACTTTGTACAAGAAAGCTGGGTTCAGAAAATGG CGCTGTCCATGGG-3' and prm08770 (SEQ ID NO: 29; reverse, complementary): 5'-GGGGACAAGTTTGTACAAAAAAGCAGGCTTAAACAATGTGTGGCGGCGCCATCCT-3', which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pHvRAF-like. Plasmid pDONR201 was purchased from Invitrogen, as part of the Gateway® technology.

The entry clone comprising SEQ ID NO: 1 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 35) for constitutive expression was located upstream of this Gateway cassette.

After the LR recombination step, the resulting expression vector pGOS2::HvRAF-like (Figure 5) was transformed into *Agrobacterium* strain LBA4044 according to methods well known in the art.

Example 7: Plant transformation

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 to 60 minutes, preferably 30 minutes in sodium hypochlorite solution (depending on the grade of contamination), followed by a 3 to 6 times, preferably 4 time wash with sterile distilled water. The sterile seeds were then germinated on a medium containing 2,4-D (callus induction medium). After incubation in light for 6 days scutellum-derived calli is transformed with Agrobacterium as described herein below.

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The calli were immersed in the suspension for 1 to 15 minutes. The callus tissues were then blotted dry on a filter paper and transferred to solidified, co-cultivation medium and incubated for 3 days in the dark at 25°C. After washing away the *Agrobacterium,* the calli were grown on 2,4-D-containing medium for 10 to 14 days (growth time for indica: 3 weeks) under light at 28°C - 32°C in the presence of a selection agent. During this period, rapidly growing resistant callus developed. After transfer of this material to regeneration media, the embryogenic potential was released and shoots developed in the next four to six weeks. Shoots were excised from the calli and incubated for 2 to 3 weeks on an auxin-containing medium from which they were transferred to soil. Hardened shoots were grown under high humidity and short days in a greenhouse.

Transformation of rice cultivar indica can also be done in a similar way as give above according to techniques well known to a skilled person.

35 to 90 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

Example 8: Transformation of other crops

*Corn transformation*

Transformation of maize *(Zea mays)* is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa *(Medicago sativa)* is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

*Cotton transformation*

Cotton is transformed using *Agrobacterium tumefaciens* according to the method described in US 5,159,135. Cotton seeds are surface sterilised in 3% sodium hypochlorite solution during 20 minutes and washed in distilled water with 500 μg/ml cefotaxime. The seeds are then transferred to SH-medium with 50μg/ml benomyl for germination. Hypocotyls of 4 to 6 days old seedlings are removed, cut into 0.5 cm pieces and are placed on 0.8% agar. An *Agrobacterium* suspension (approx. 108 cells per ml, diluted from an overnight culture transformed with the gene of interest and suitable selection markers) is used for inoculation of the hypocotyl explants. After 3 days at room temperature and lighting, the tissues are transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 μg/ml MgCL2, and with 50 to 100 μg/ml cefotaxime and 400-500μ g/ml carbenicillin to kill residual bacteria. Individual cell lines are isolated after two to three months (with subcultures every four to six weeks) and are further cultivated on selective medium for tissue amplification (30°C, 16 hr photoperiod). Transformed tissues are subsequently further cultivated on non-selective medium during 2 to 3 months to give rise to somatic embryos. Healthy looking embryos of at least 4 mm length are transferred to tubes with SH medium in fine vermiculite, supplemented with 0.1 mg/l indole acetic acid, 6 furfurylaminopurine and gibberellic acid. The embryos are cultivated at 30°C with a photoperiod of 16 hrs, and plantlets at the 2 to 3 leaf stage are transferred to pots with vermiculite and nutrients. The plants are hardened and subsequently moved to the greenhouse for further cultivation.

*Sugarbeet transformation*

Seeds of sugarbeet (Beta vulgaris L.) are sterilized in 70% ethanol for one minute followed by 20 min. shaking in 20% Hypochlorite bleach e.g. Clorox® regular bleach (commercially available from Clorox, 1221 Broadway, Oakland, CA 94612, USA). Seeds are rinsed with sterile water and air dried followed by plating onto germinating medium (Murashige and Skoog (MS) based medium (Murashige, T., and Skoog, ., 1962. Physiol. Plant, vol. 15, 473-497) including B5 vitamins (Gamborg et al.; Exp. Cell Res., vol. 50, 151-8.) supplemented with 10 g/l sucrose and 0,8% agar). Hypocotyl tissue is used essentially for the initiation of shoot cultures according to Hussey and Hepher (Hussey, G., and Hepher, A., 1978. Annals of Botany, 42, 477-9) and are maintained on MS based medium supplemented with 30g/l sucrose plus 0,25mg/l benzylamino purine and 0,75% agar, pH 5,8 at 23-25°C with a 16-hour photoperiod. Agrobacterium tumefaciens strain carrying a binary plasmid harbouring a selectable marker gene, for example nptII, is used in transformation experiments. One day before transformation, a liquid LB culture including antibiotics is grown on a shaker (28°C, 150rpm) until an optical density (O.D.) at 600 nm of ~1 is reached. Overnight-grown bacterial cultures are centrifuged and resuspended in inoculation medium (O.D. ~1) including Acetosyringone, pH 5,5. Shoot base tissue is cut into slices (1.0 cm x 1.0 cm x 2.0 mm approximately). Tissue is immersed for 30s in liquid bacterial inoculation medium. Excess liquid is removed by filter paper blotting. Co-cultivation occurred for 24-72 hours on MS based medium incl. 30g/l sucrose followed by a non-selective period including MS based medium, 30g/l sucrose with 1 mg/l BAP to induce shoot development and cefotaxim for eliminating the Agrobacterium. After 3-10 days explants are transferred to similar selective medium harbouring for example kanamycin or G418 (50-100 mg/l genotype dependent). Tissues are transferred to fresh medium every 2-3 weeks to maintain selection pressure. The very rapid initiation of shoots (after 3-4 days) indicates regeneration of existing meristems rather than organogenesis of newly developed transgenic meristems. Small shoots are transferred after several rounds of subculture to root induction medium containing 5 mg/l NAA and kanamycin or G418. Additional steps are taken to reduce the potential of generating transformed plants that are chimeric (partially transgenic). Tissue samples from regenerated shoots are used for DNA analysis. Other transformation methods for sugarbeet are known in the art, for example those by Linsey & Gallois (Linsey, K., and Gallois, P., 1990. Journal of Experimental Botany; vol. 41, No. 226; 529-36) or the methods published in the international application published as WO9623891A.

*Sugarcane transformation*

Spindles are isolated from 6-month-old field grown sugarcane plants (Arencibia et al., 1998. Transgenic Research, vol. 7, 213-22; Enriquez-Obregon et al., 1998. Planta, vol. 206, 20-27). Material is sterilized by immersion in a 20% Hypochlorite bleach e.g. Clorox® regular bleach (commercially available from Clorox, 1221 Broadway, Oakland, CA 94612, USA) for 20 minutes. Transverse sections around 0,5cm are placed on the medium in the top-up direction. Plant material is cultivated for 4 weeks on MS (Murashige, T., and Skoog, ., 1962. Physiol. Plant, vol. 15, 473-497) based medium incl. B5 vitamins (Gamborg, O., et al., 1968. Exp. Cell Res., vol. 50, 151-8) supplemented with 20g/l sucrose, 500 mg/l casein hydrolysate, 0,8% agar and 5mg/l 2,4-D at 23°C in the dark. Cultures are transferred after 4 weeks onto identical fresh medium. Agrobacterium tumefaciens strain carrying a binary plasmid harbouring a selectable marker gene, for example hpt, is used in transformation experiments. One day before transformation, a liquid LB culture including antibiotics is grown on a shaker (28°C, 150rpm) until an optical density (O.D.) at 600 nm of ~0,6 is reached. Overnight-grown bacterial cultures are centrifuged and resuspended in MS based inoculation medium (O.D. ~0,4) including acetosyringone, pH 5,5. Sugarcane embryogenic callus pieces (2-4 mm) are isolated based on morphological characteristics as compact structure and yellow colour and dried for 20 min. in the flow hood followed by immersion in a liquid bacterial inoculation medium for 10-20 minutes. Excess liquid is removed by filter paper blotting. Co-cultivation occurred for 3-5 days in the dark on filter paper which is placed on top of MS based medium incl. B5 vitamins containing 1 mg/l 2,4-D. After co-cultivation calli are washed with sterile water followed by a non-selective cultivation period on similar medium containing 500 mg/l cefotaxime for eliminating remaining Agrobacterium cells. After 3-10 days explants are transferred to MS based selective medium incl. B5 vitamins containing 1 mg/l 2,4-D for another 3 weeks harbouring 25 mg/l of hygromycin (genotype dependent). All treatments are made at 23°C under dark conditions. Resistant calli are further cultivated on medium lacking 2,4-D including 1 mg/l BA and 25 mg/l hygromycin under 16 h light photoperiod resulting in the development of shoot structures. Shoots are isolated and cultivated on selective rooting medium (MS based including, 20g/l sucrose, 20 mg/l hygromycin and 500 mg/l cefotaxime). Tissue samples from regenerated shoots are used for DNA analysis. Other transformation methods for sugarcane are known in the art, for example from the international application published as WO2010/151634A and the granted European patent EP1831378.

Example 9: Phenotypic evaluation procedure

9.1 Evaluation setup

35 to 90 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions were watered at regular intervals to ensure that water and nutrients were not limiting and to satisfy plant needs to complete growth and development, unless they were used in a stress screen.

From the stage of sowing until the stage of maturity the plants were passed several times through a digital imaging cabinet. At each time point digital images (2048x1536 pixels, 16 million colours) were taken of each plant from at least 6 different angles.

T1 events can be further evaluated in the T2 generation following the same evaluation procedure as for the T1 generation, e.g. with less events and/or with more individuals per event.

*Drought screen*

T1 or T2 plants are grown in potting soil under normal conditions until they approached the heading stage. They are then transferred to a "dry" section where irrigation is withheld. Soil moisture probes are inserted in randomly chosen pots to monitor the soil water content (SWC). When SWC goes below certain thresholds, the plants are automatically re-watered continuously until a normal level is reached again. The plants are then re-transferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

T1 or T2 plants are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

*Salt stress screen*

T1 or T2 plants are grown on a substrate made of coco fibers and particles of baked clay (Argex) (3 to 1 ratio). A normal nutrient solution is used during the first two weeks after transplanting the plantlets in the greenhouse. After the first two weeks, 25 mM of salt (NaCl) is added to the nutrient solution, until the plants are harvested. Growth and yield parameters are recorded as detailed for growth under normal conditions.

9.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

9.3 Parameters measured

From the stage of sowing until the stage of maturity the plants were passed several times through a digital imaging cabinet. At each time point digital images (2048x1536 pixels, 16 million colours) were taken of each plant from at least 6 different angles as described in WO2010/031780. These measurements were used to determine different parameters.

*Biomass-related parameter measurement*

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass.

Increase in root biomass is expressed as an increase in total root biomass (measured as maximum biomass of roots observed during the lifespan of a plant); or as an increase in the root/shoot index, measured as the ratio between root mass and shoot mass in the period of active growth of root and shoot. In other words, the root/shoot index is defined as the ratio of the rapidity of root growth to the rapidity of shoot growth in the period of active growth of root and shoot. Root biomass can be determined using a method as described in WO 2006/029987.

A robust indication of the height of the plant is the measurement of the location of the centre of gravity, i.e. determining the height (in mm) of the gravity centre of the leafy biomass. This avoids influence by a single erect leaf, based on the asymptote of curve fitting or, if the fit is not satisfactory, based on the absolute maximum.

*Parameters related to development time*

The early vigour is the plant aboveground area three weeks post-germination. Early vigour was determined by counting the total number of pixels from aboveground plant parts discriminated from the background. This value was averaged for the pictures taken on the same time point from different angles and was converted to a physical surface value expressed in square mm by calibration.

AreaEmer is an indication of quick early development when this value is decreased compared to control plants.

It is the ratio (expressed in %) between the time a plant needs to make 30 % of the final biomass and the time needs to make 90 % of its final biomass.

The "time to flower" or "flowering time" of the plant can be determined using the method as described in WO 2007/093444.

*Seed-related parameter measurements*

The mature primary panicles were harvested, counted, bagged, barcode-labelled and then dried for three days in an oven at 37°C. The panicles were then threshed and all the seeds were collected and counted. The seeds are usually covered by a dry outer covering, the husk. The filled husks (herein also named filled florets) were separated from the empty ones using an air-blowing device. The empty husks were discarded and the remaining fraction was counted again. The filled husks were weighed on an analytical balance.

The total number of seeds was determined by counting the number of filled husks that remained after the separation step. The total seed weight was measured by weighing all filled husks harvested from a plant.

The total number of seeds (or florets) per plant was determined by counting the number of husks (whether filled or not) harvested from a plant.

Panicles: This refers to the number of fertile panicles.

Thousand Kernel Weight (TKW) is extrapolated from the number of seeds counted and their total weight.

The Harvest Index (HI) in the present invention is defined as the ratio between the total seed weight and the above ground area ($mm^2$), multiplied by a factor $10^6$.

The number of flowers per panicle as defined in the present invention is the ratio between the total number of seeds over the number of mature primary panicles.

The "seed fill rate" or "seed filling rate" as defined in the present invention is the proportion (expressed as a %) of the number of filled seeds (i.e. florets containing seeds) over the total number of seeds (i.e. total number of florets). In other words, the seed filling rate is the percentage of florets that are filled with seed.

*T4 and T5 Field Trials*

In addition, T4 homozygous rice plants were grown and evaluated under field conditions as follows.

To evaluate yield components of transgenic plants under normal field conditions, three independent T4 (2011) and T5 (2012) homozygous lines of the GOS2::HvRAF plants, together with NT controls, were transplanted to a paddy field at Kyungpook National University, Gunwie, Korea. A randomized design was employed with two (2011) and three (2012) replicates. At 25 d after sowing, the seedlings were randomly transplanted with 15x30-cm spacing and a single seedling per hill. Fertilizer was applied at 70:40:70 (nitrogen:phosphorus:potassium) kg $ha^{-1}$ after the last paddling and 45 d after transplantation. Yield parameters were scored for 20 (2011) and 30 (2012) plants per transgenic line. Plants located at borders were excluded from data scoring.

To evaluate yield components of transgenic plants under drought field conditions, three independent T4 (2011) and T5 (2012) homozygous lines of each of the GOS2::HvRAF plants and NT controls were transplanted to a removable rain-off shelter (located at Myongji University, Yongin, Korea) with a 1-m-deep container filled with natural paddy soil.

The experimental design, transplanting spacing, use of fertilizer, drought treatments, and scoring of agronomic traits were as described (Oh et al., 2009). When the plants grown under normal and drought conditions had reached maturity and the grains had ripened, they were harvested and threshed by hand (separation of seeds from the vegetative parts of the plant). The unfilled and filled grains were then taken apart, independently counted using a Countmate MC1000H (Prince Ltd.), and weighed. The following agronomic traits were scored: flowering date, panicle length, number of tillers, number of panicles, spikelets per panicle, filling rate (%), total grain weight (g), and 1,000 grain weight (g). The results from three independent lines were separately analyzed by one-way ANOVA and compared with those of the NT controls.

The ANOVA was used to reject the null hypothesis of equal means of transgenic lines and NT controls (P, 0.05). SPSS version 16.0 was used to perform these statistical analyses.

<u>Example 10: Results of the phenotypic evaluation of the transgenic plants</u>

The results of the evaluation of transgenic rice plants in the T4 generation and expressing a nucleic acid encoding the HvRAF-like polypeptide of SEQ ID NO: 5 under non-stress field conditions are presented below in Tables D and E. When grown under non-stress field conditions, an increase of at least 5 % was observed for panicle length, number of panicles, and for seed yield (including total weight of seeds, number of seeds,and total seed weight, g/1000 seeds (TKW).

Table D: Data summary for transgenic rice plants; for each parameter, the overall percent increase is shown for T4 generation plants, for each parameter the p-value is <0.05.

| Parameter | Overall increase |
|---|---|
| Panicle length | 5.7 |
| Panicles | 12.6 |
| Nrtotalseed | 13.1 |
| totalwgseeds | 22.5 |
| nrfilledseed | 14.3 |
| TKW | 8.7 |

**Table E:** Data summary for transgenic rice plants; for each parameter, the overall percent increase is shown for T5 generation plants, for each parameter the p-value is <0.05.

| Parameter | Overall increase |
|---|---|
| Panicle length | 4.7 |
| Panicles | 15.6 |
| Nrtotalseed | 9.4 |
| totalwgseeds | 10.5 |
| nrfilledseed | 7.9 |
| TKW | 1.9 |

Example 11: Sugarcane phenotypic evaluation procedure

11.1 The transgenic sugarcane plants generated are grown for 10 to 15 months, either in the greenhouse or the field. Standard conditions for growth of the plants are used.

12.2 Sugar extraction method
Stalks of sugarcane plants which are 10 to 15 months old and have more than 10 internodes are harvested. After all of the leaves have been removed, the internodes of the stalk are numbered from top (= 1) to bottom (for example = 36). A stalk disc approximately 1-2 g in weight is excised from the middle of each internode. The stalk discs of 3 internodes are then combined to give one sample and frozen in liquid nitrogen.
For the sugar extraction, the stalk discs are first comminuted in a Waring blender (from Waring, New Hartford, Connecticut, USA). The sugars are extracted by shaking for one hour at 95°C in 10 mM sodium phosphate buffer pH 7.0. Thereafter, the solids are removed by filtration through a 30 µm sieve. The resulting solution is subsequently employed for the sugar determination (see herein below).

11.3 Fresh weight and biomass
The transgenic sugarcane plants expressing the HvRAF-like polypeptide are grown for 10 to 15 months. In each case a sugarcane stalk of the transgenic line and a wild-type sugarcane plant is defoliated, the stalk is divided into segments of 3 internodes, and these internode segments are frozen in liquid nitrogen in a sealed 50 ml plastic container. The fresh weight of the samples is determined. The extraction for the purposes of the sugar determination is done as described below.
The stem biomass is increased in the transgenic plant.

11.4 Sugar determination (glucose, fructose and sucrose)
The glucose, fructose and sucrose contents in the extract obtained in accordance with the sugar extraction method described above is determined photometrically in an enzyme assay via the conversion of NAD+ (nicotinamide adenine dinucleotide) into NADH (reduced nicotinamide adenine dinucleotide). During the reduction, the aromatic character at the nicotinamide ring is lost, and the absorption spectrum thus changes. This change in the absorption spectrum can be detected photometrically. The glucose and fructose present in the extract is converted into glucose-6-phosphate and fructose-6-phosphate by means of the enzyme hexokinase and adenosin triphosphate (ATP). The glucose- 6-phosphate is subsequently oxidized by the enzyme glucose-6-phosphate dehydrogenase to give 6-phosphogluconate. In this reaction, NAD+ is reduced to give NADH, and the amount of NADH formed is determined photometrically. The ratio between the NADH formed and the glucose present in the extract is 1:1, so that the glucose content can be calculated from the NADH content using the molar absorption coefficient of NADH (6.3 1 per mmol and per cm lightpath). Following the complete oxidation of glucose-6-phosphate, fructose-6-phosphate, which has likewise formed in the solution, is converted by the enzyme phosphoglucoisomerase to give glucose- 6-phosphate which, in turn, is oxidized to give 6-phosphogluconate. Again, the ratio between fructose and the amount of NADH formed is 1 :1. Thereafter, the sucrose present in the extract is cleaved by the enzyme sucrase (Megazyme) to give glucose and fructose. The glucose and fructose molecules liberated are then converted with the abovementioned enzymes in the NAD+-dependent reaction to give 6- phosphogluconate. The conversion of one sucrose molecule into 6-phosphogluconate results in two NADH molecules. The amount of NADH formed is likewise determined photometrically and used for calculating the sucrose content, using the molar absorption coefficient of NADH. The sugarcane stalks are divided into segments of in each case three internodes, as specified above. The internodes are numbered from top to bottom (top = internode 1, bottom = internode 21). In the sugarcane wild-type plant, the sucrose contents rises from internode 1-3 up to internode 10-12. The sucrose contents of all subsequent internodes are similarly high.

## Claims

1. A method for the production of a transgenic plant having enhanced yield-related traits relative to a control plant, comprising the steps of:

   (i) introducing and expressing in a plant cell or plant a nucleic acid encoding a HvRAF-like polypeptide, wherein said HvRAF-like polypeptide comprises the polypeptide represented by SEQ ID NO: 5 or a homologue thereof which has at least 30% overall sequence identity to SEQ ID NO : 5, and
   (ii) cultivating said plant cell or plant under conditions promoting plant growth and development.

2. Method according to claim 1, wherein said HvRAF-like polypeptide comprises

   a. all of the following motifs:

      (i) Motif 1 represented by SEQ ID NO: 31,
      (ii) Motif 2 represented by SEQ ID NO: 32,
      (iii) Motif 3 represented by SEQ ID NO: 33,
      (iv) Motif 4 represented by SEQ ID NO: 34,

   or
   b. any 3 or 2 of the motifs 1 to 4 as defined under "a" above; or
   c. Motif 1 or Motif 2 or Motif 3 or Motif 4 as defined under "a" above.

3. Method according to any one of claims 1 or 2, wherein said nucleic acid encoding a HvRAF-like polypeptide encodes any one of the polypeptides listed in Table A or is a portion of such a nucleic acid, or a nucleic acid capable of hybridising with such a nucleic acid.

4. Method according to any one of claims 1 to 3, wherein said one or more enhanced yield-related traits comprises increased yield and/or early vigour relative to control plants, and preferably comprises increased biomass and/or increased seed yield relative to control plants.

5. Method according to claim 4, wherein said one or more enhanced yield-related traits comprises an increase of at least 5% relative to control plants for at least one of said parameters.

**6.** Method according to any one of claims 1 to 5, wherein said nucleic acid is operably linked to a constitutive promoter of plant origin, preferably to a medium strength constitutive promoter of plant origin, more preferably to a GOS2 promoter, most preferably to a GOS2 promoter from rice.

**7.** An isolated nucleic acid molecule selected from the group consisting of:

(i) a nucleic acid represented by SEQ ID NO: 1 or SEQ ID NO: 4;
(ii) the complement of a nucleic acid represented by SEQ ID NO: 1 or SEQ ID NO: 4;
(iii) a nucleic acid encoding a HvRAF-like polypeptide having at least 30% sequence identity to the amino acid sequence represented by SEQ ID NO: 5; and
(iv) a nucleic acid molecule which hybridizes with a nucleic acid molecule of (i) to (iii) under high stringency hybridization conditions.

**8.** An isolated polypeptide selected from the group consisting of:

(i) an amino acid sequence represented by SEQ ID NO: 5;
(ii) an amino acid sequence having at least 30%, sequence identity to the amino acid sequence represented by SEQ ID NO: 5; and
(iii) derivatives of any of the amino acid sequences given in (i) or (ii) above.

**9.** Construct comprising:

(i) a nucleic acid encoding a HvRAF-like polypeptide as defined in any of claims 1 to 3 and 8;
(ii) one or more control sequences capable of driving expression of the nucleic acid sequence of (i); and optionally
(iii) a transcription termination sequence.

**10.** Construct according to claim 9, wherein one of said control sequences is a constitutive promoter of plant origin, preferably to a medium strength constitutive promoter of plant origin, more preferably to a GOS2 promoter, most preferably to a GOS2 promoter from rice.

**11.** Transgenic plant having one or more enhanced yield-related traits as defined in either one of claims 4 or 5, resulting from the introduction and expression of an isolated nucleic acid encoding a HvRAF-like polypeptide as defined in any of claims 1 to 3 and 8 in said plant, or resulting from the introduction and expression of an isolated nucleic acid as defined in claim 7 in said plant; or a transgenic plant cell derived from said transgenic plant.

**12.** Use of an isolated nucleic acid encoding a HvRAF-like polypeptide as defined in any of claims 1 to 3 and 8, an isolated nucleic acid as defined in claim 7, or a construct as defined in either one of claims 9 or 10, for enhancing one or more yield-related traits in a plant as defined in either one of claims 4 or 5.

**13.** Plant, or part thereof, or plant cell transformed with a construct according to either one of claims 9 or 10.

**14.** Harvestable parts of a plant according to claim 11 or 13, wherein said harvestable parts are preferably shoot biomass and/or root biomass and/or seeds.

**15.** A product derived from a plant according to any one of claims 11 or 13, or from harvestable parts of a plant according to claim 14.

MKRTRFTAVGSWYHGRGRLMRFLFAAYDGGRAAHAASRK

KRT **[GHLHGIRQRPWGKWAAEIRDPHKGTRVWLGTFDTA**
**Motif 1**

**DDAARAYDVAARRLRGSKAKVNFPD]** AARTGARPRRASR
**Motif 1**

RTAQKPQCPPARTTAYSATAAARAQPEQDAMMVKPELME

FFNVDAIVHLTTAVAALPPVTASTFADTMPRVDEDSSV

GS **[GGGAMLGFADELGFDPFMMFQLPCSDMYE] [SADSI**
**Motif 2** **Motif**

**FAG]** DAVIPD **[ALSVDSGMDAVSLWSFDEFP]** MDSAIF
**3** **Motif 4**

**FIGURE 1**

```
Hv_RAF_lik : ------------------MKRTRFTAVG----SWYHGRG-RLMR------ :  21
H01_Hv     : MCGGAILAQLIP---PSAGRPSKQAAAGGRAPPTSSKKG-GVSKSRHSST :  46
H02_Sb     : MCGGAILAELIP-PTRR--VAPKPVTEGHLWPASSKKAGSGSNK--RHHH :  45
H03_Os     : MCGGAILAEFIP-APSRAAAATKRVTASHLWPAGSKNAARGKSKSKRQQR :  49
H04_Bd     : MCGGAILAELTPPPARGGGASKPVGAKGQVWPVSSKKEG-GTNKTRHGSV :  49
H05_Zm     : MCGGAILAELIP-PTRR--VASKPVTEGHLWSASSKKAGSGRDK--RHQH :  45
H06_Hv     : MCGGAILAELIS---PSAGRAPKQAQVAAAGPVSANKDG-KSKGHKHNKY :  46
H07_Hv     : MCGGAILAQLIP---PSAGRPSKQAAAGGRAPPTSSKKG-GVSKSRHSST :  46
H08_Hv     : MCGGAILAQLIP---PSAGRPSKQAAAGGRAAPTSSKKG-GVSKSRHSST :  46
H09_Ta     : MCGGAILAKLIPPTPPSAGRAPKQVAAGG----VSPKKG-GMNKTHHSST :  45
H10_Zm     : MCGGAILAELIP-PTRR--VASKPVTEGHLWSASSKKAGSGRDK--RHQH :  45
H11_Zm     : MCGGAILAELIP-PTRR--VASKPVTEGHLWSASSKKAGSGRDK--RHQH :  45

Hv_RAF_lik : -------------------------------FLFA---------AYDG-G :  30
H01_Hv     : PDADDDVFEAAFEDFDDHFDLRAEEDGGDDHVVFASKPAFSPRPAYDG-G :  95
H02_Sb     : EYADDDDFEAAFEEFDDDFDVHEDDEDHPDHFVFSSKSAFSPA-HDGRRD :  94
H03_Os     : SFADVDDFEAAFEQFDDDSDFDDAEEEDEGHFVFASKSRVVAG-HDGR-- :  96
H04_Bd     : VDD----FEAAFEGFHDDFP-----------VLFASKPAFSPVHGDSGRA :  84
H05_Zm     : EYADDD-FEAAFEDFDDDFDVHEDDEDG--HFVFSSKSALSPALHDGR-- :  90
H06_Hv     : GSVAD--VDDDLQDFDDDLDLRQAEEDADDHVVFACKPAFSPGPAYDG-G :  93
H07_Hv     : PDADDDVFEAAFEDFDDHFDLRAEEDGGDDHVVFASKPAFSPRPAYDG-G :  95
H08_Hv     : PDADDDVSSAAFEDFDDHFDLRAEEDGGDDHVVFASKPAFSPRPAYDG-G :  95
H09_Ta     : PDVDD--FEAAFEDFDDDFHLQAEEDG-DDHVVFASKPAFSP--AYDD-G :  89
H10_Zm     : EYADDD-FEAAFEDFDDDFDVHEDDEDG--HLVFSSKSAFSPALHDGR-- :  90
H11_Zm     : EYADDD-FEAAFEDFDDDFDVHEDDEDG--HFVFSSKSALSPALHDGR-- :  90

Hv_RAF_lik : RAAHAAS---RKKRTGHLHGIRQRPWGKWAAEIRDPHKGTRVWLGTFDTA :  77
H01_Hv     : RAAHAAS---RKKRTGHLHGIRQRPWGKWAAEIRDPHKGTRVWLGTFDTA : 142
H02_Sb     : ARAAASQR--TKRGRRHFHGIRQRPWGKWAAEIRDPHKGTRVWLGTFNTA : 142
H03_Os     : AAARAASK--KKRGR-HFRGIRQRPWGKWAAEIRDPHKGTRVWLGTFNTP : 143
H04_Bd     : REAQAAASCRKKKRVRGLHGIRQRPWGKWAAEIRDPHKGARVWLGTYDTA : 134
H05_Zm     : ---AASQK--KQRGR-QFRGIRQRPWGKWAAEIRDPHKGTRVWLGTFSTA : 134
H06_Hv     : RAAQAAS---RKKKR-VLHGIRQRPWGKWAAEIRDPHKGTRVWLGTFDTA : 139
H07_Hv     : RAAHAAS---RKKRTGHLHGIRQRPWGKWAAEIRDPHKGTRVWLGTFDTA : 142
H08_Hv     : RAAHAAS---RKKRTGHLHGIRQRPWGKWAAEIRDPHKGTRVWLGTFDTA : 142
H09_Ta     : RAAQAAS---RKKSVRRLHGIRQRPWGKWAAEIRDPHKGTRVWLGTFDTA : 136
H10_Zm     : ---AASQK--KQRGR-QFRGIRQRPWGKWAAEIRDPHKGTRVWLGTFSTA : 134
H11_Zm     : ---AASQK--KQRGR-QFRGIRQRPWGKWAAEIRDPHKGTRVWLGTFSTA : 134

Hv_RAF_lik : DDAARAYDVAARRLRGSKAKVNFPDAARTGAR----PRRASRRTAQKPQC : 123
H01_Hv     : DDAARAYDVAARRLRGSKAKVNFPDAARTGAR----PRRASRRTAQKPQC : 188
H02_Sb     : EDAARAYDVEARRLRGSKAKVNFP-AASARPR----ARRGK-GNPRTAPK : 186
H03_Os     : EEAARAYDVEARRLRGSKAKVNFP-ATPAAAR----PRRGNTRATAVPPP : 188
H04_Bd     : DEAARAYDVAARRLRGSNAKLNFP-AAPGRAR----PRCG---TAPKPRR : 176
H05_Zm     : EDAARAYDVEARRLRGSKAKVNFP-AASGRARGRARPRRGDDGNPRTAPE : 183
H06_Hv     : DDAARAYDVAARRLRGSKAKVNFPDAARAGAR----PRRASRRTAQKPQR : 185
H07_Hv     : DDAARAYDVAARRLRGSKAKVNFPDAARTGAR----PRRASRRTAQKPQC : 188
H08_Hv     : DDAARAYDVAARRLRGSKAKVNFPDAARTGAR----PRRASRRTAQKPQC : 188
H09_Ta     : DDAARAYDVAAHRLRGSKAKVNFPNGTRAGAR----LQRASRRTASKRQC : 182
H10_Zm     : EDAARAYDVEARRLRGSKAKVNFP-AASGRAR--ARPRRGDDGNPRTAPE : 181
H11_Zm     : EDAARAYDVEARRLRGSKAKVNFP-AASGRARGRARPRRGDDGNPRTAPE : 183
```

FIGURE 2

```
Hv_RAF_lik : PPAR---------TTAYSATA----AARAQPEQDAMMVKPELMEFFNVDA : 160
H01_Hv     : PPAR---------TTAYSATA----AARAQPEQDAMMVKPELMEFFNVDA : 225
H02_Sb     : PAAQ-------AQPALLLRGE-----KGLEG-VAVKPETTMDMFDVGS-- : 221
H03_Os     : ATAP---------AAAPPRGL-----KREFS----PPAETALPFFTNG-- : 218
H04_Bd     : PTTA---------QTACFSAI----TAAAAARAQEIEQELMPMPYFDVDA : 213
H05_Zm     : TQHP-------AQPALLPRGE--RETQRKEGIAAVKPEAT-ESFDVGGGL : 223
H06_Hv     : PPAW---------TTAYSATA----AAHSQPEQ---------------DA : 207
H07_Hv     : PPAR---------TTAYSATA----AARAQPEQDAMMVKPELMEFFNVDA : 225
H08_Hv     : PPAR---------TTAYSATA----AARAQPEQDAMMVKPELMEFFNVDA : 225
H09_Ta     : PPAR---------TTAYS-------AAHAQKERDAMVAKPELMESFDMDA : 216
H10_Zm     : PQHPAAAAAAAAQPALLPRGERVRETQRKEGNAAVKPEAT-QSFDVGG-L : 229
H11_Zm     : TQHP-------AQPALLPRGE--RETQRKEGIAAVKPEAT-ESFDVGGGL : 223

Hv_RAF_lik : IVHLT--TAVAALPPVTA-STFADT------------MPRVDE-DSSVG- : 193
H01_Hv     : IVHLT--TAVAALPPVTA-STFADT------------MPRVDE-DSSVG- : 258
H02_Sb     : FFDMTFPTFPAAP-AAAMESSFAGSSATSETR-SPGKKLRYDDGDSSEGS : 269
H03_Os     : FVDLT--TAAAPPPAMMMTSSFTDSVATSESGGSPAKKARSDDVDSSEGS : 266
H04_Bd     : FLDLA--ASVAELPPVIIGSSFADS------------------------- : 236
H05_Zm     : FFDMAFPTFPASPPPQAVDTSFAGSTATSETG-SPAKRPRCDE-DSSEG- : 270
H06_Hv     : FVDLT--TAVTALPPIME-SSFADSGST---------KPMFHE-DSSAG- : 243
H07_Hv     : IVHLT--TAVAALPPVTA-STFADT------------MPRVDE-DSSVG- : 258
H08_Hv     : IVHLT--SAVAALPPVTA-STFADT------------MPRVDE-DSSVG- : 258
H09_Ta     : FVDLT--TAFTTLPPVMA-SSFADTGAK---------KPMVDE-DSSDG- : 252
H10_Zm     : FFDMAFPTFPASPPPQAVEASFTGSTATSETG-SPAKRPRCDD-NSSEGS : 277
H11_Zm     : FFDMAFPTFPASPPPQAVDTSFAGSTATSETG-SPAKRPRCDE-DSSEG- : 270

Hv_RAF_lik : -SGGGAMLGFADELGFDPFYMFQLPCS--DMYESADSIFAGDAVIPDALS : 240
H01_Hv     : -SGGGAMLGFADELGFDPFYMFLLPCS--DMYESADSIFAGDAVIPDALS : 305
H02_Sb     : -GCGS-ALELADELAFDPFYLLQMPYSGGYD--SLDGLFAA-EAVQQDVN : 314
H03_Os     : VGGGSDTLGFTDELEFDPFYLFQLPYSDGYE--SIDSLFAAGDANSANT- : 313
H04_Bd     : -AGGAATRGFADELEFDPFYLCELPWSESDTCESIEGLFAGDAVQ-DAGG : 284
H05_Zm     : -GSGS-ALELADELAFDPFVLLQMPYSGGYDDDSLDGLFAADEAVQQDVG : 318
H06_Hv     : -SGGGAMPGFTDELGFDPFYLFQLPCS--DTYESIDSLFAGDAALQDARG : 290
H07_Hv     : -SGGGAMLGFADELGFDPFYMFQLPCS--DMYESADSIFAGDAVIPDALS : 305
H08_Hv     : -SGGGAMLGFADELGFDPFYMFLLPCS--DMYESADSIFAGDAVIPDALS : 305
H09_Ta     : -SGGDAMLG------FDPFYLFQLPCS--DTYESIDSLFAGDAVIQDALG : 293
H10_Zm     : GGSGS-ALELADELAFDPFYLLQMPYSGGYD--SLDGLFAA-EAVQQDVG : 323
H11_Zm     : -GSGS-ALELADELAFDPFVLLQMPYSGGYDDDSLDGLFSADEAVQQDVG : 318

Hv_RAF_lik : VDSGMDAVSLWSFDEFP-MDSAIF : 263
H01_Hv     : VDSGMDAVSLWSFDEFP-MDSAIF : 328
H02_Sb     : --NDMNGVSLWSFDEFP-VDGYVF : 335
H03_Os     : --DMNAGVNLWSFDDFP-IDGALF : 334
H04_Bd     : VNTGMDSVSLWSFDEFP-MDAAAF : 307
H05_Zm     : --NGMDGVRLWSFDEFPAVDGSVF : 340
H06_Hv     : VDSGIDGVSLWSFDEFP-MDSAIF : 313
H07_Hv     : VDSGMDAVSLWSFDEFP-MDSAIF : 328
H08_Hv     : VDSGMDAVSLWSADEFP-MDSAIF : 328
H09_Ta     : VDSGMEGVSLWSFEEFP-MDSAIF : 316
H10_Zm     : --NGMDGVSLWSFDEFPAVDGCVF : 345
H11_Zm     : --NGMDGVRLWRFDEFPAVDGSVF : 340
```

FIGURE 2 (continued)

FIGURE 3

| MatGat (matrix = BLOSUM50; first_gap = 12; extending_gap = 2) | 1 | 2 | 3 | 4 | 5 | 6 | 7 | 8 | 9 | 10 | 11 | 12 |
|---|---|---|---|---|---|---|---|---|---|---|---|---|
| 1. Hv_RAF_like | | 75 | 41,1 | 44,4 | 48,6 | 41,2 | 60,2 | 75,3 | 74,4 | 59,1 | 42,5 | 40,6 |
| 2. H01_Hv | | | 50,6 | 52,1 | 57,1 | 49,9 | 71,3 | 99,7 | 98,5 | 75,4 | 51,4 | 49,3 |
| 3. H02_Sb | | | | 59,3 | 49,4 | 76,7 | 50 | 50,9 | 49,7 | 53,8 | 77,5 | 76,1 |
| 4. H03_Os | | | | | 48,2 | 55,9 | 52,3 | 53,1 | 51,7 | 51,6 | 58,1 | 55,3 |
| 5. H04_Bd | | | | | | 48,4 | 55,7 | 57,1 | 56,3 | 57,7 | 48,6 | 47,9 |
| 6. H05_Zm | | | | | | | 47,1 | 50,1 | 49 | 51 | 91,5 | 99,4 |
| 7. H06_Hv | | | | | | | | 71,6 | 70,7 | 72 | 47,9 | 46,6 |
| 8. H07_Hv | | | | | | | | | 98,2 | 75,7 | 51,7 | 49,6 |
| 9. H08_Hv | | | | | | | | | | 74,3 | 50,3 | 48,4 |
| 10. H09_Ta | | | | | | | | | | | 51,1 | 50,4 |
| 11. H10_Zm | | | | | | | | | | | | 90,9 |
| 12. H11_Zm | | | | | | | | | | | | |

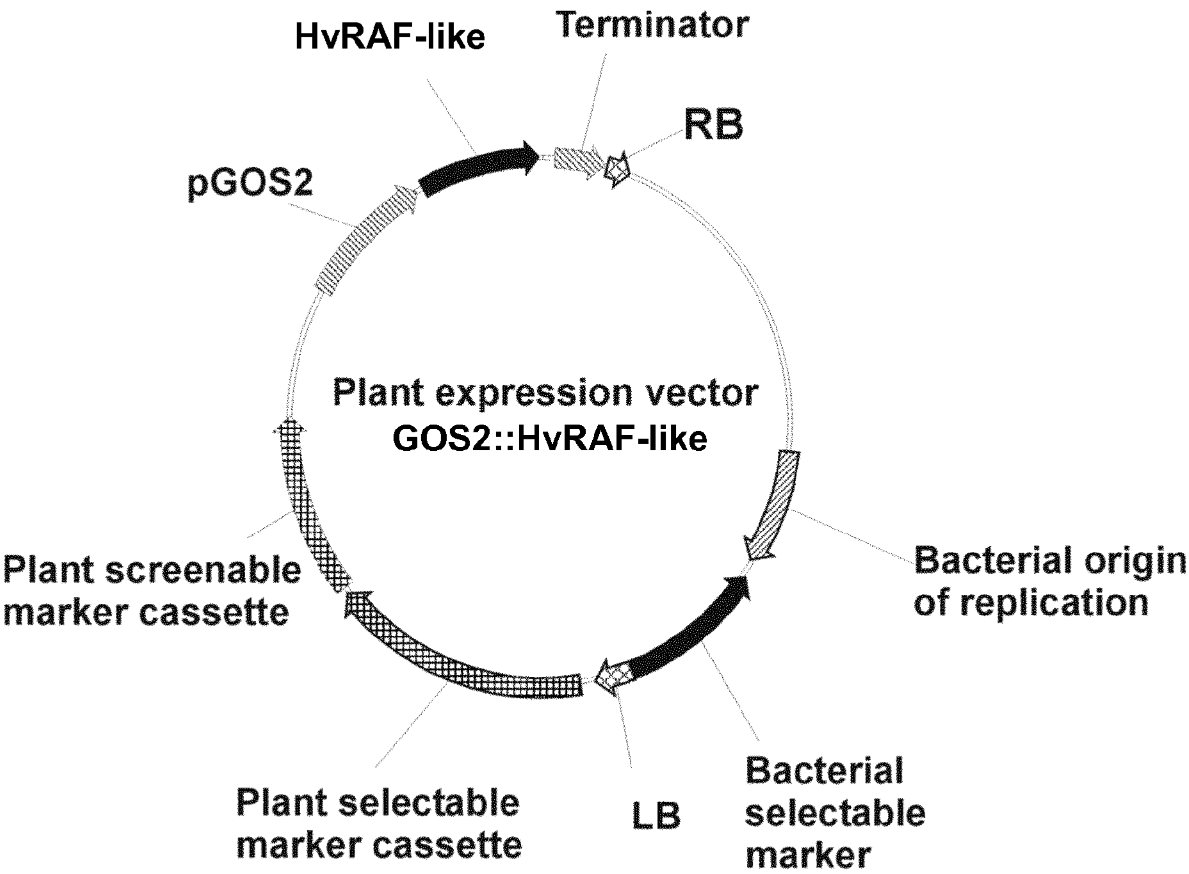
HvRAF-like
Terminator
RB
pGOS2
Plant expression vector
GOS2::HvRAF-like
Bacterial origin
of replication
Plant screenable
marker cassette
Plant selectable
marker cassette
LB
Bacterial
selectable
marker

FIGURE 4

Europäisches Patentamt
European Patent Office
Office européen des brevets

# EUROPEAN SEARCH REPORT

Application Number
EP 14 15 1610

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | DATABASE UniProt [Online]<br><br>3 April 2013 (2013-04-03), "SubName: Full=Uncharacterized protein;", XP002725145, retrieved from EBI accession no. UNIPROT:M0UR09 Database accession no. M0UR09<br>* sequence *<br>----- | 8 | INV.<br>C12N15/82 |
| X | JINWOOK JUNG ET AL: "The barley ERF-type transcription factor HvRAF confers enhanced pathogen resistance and salt tolerance in Arabidopsis", PLANTA ; AN INTERNATIONAL JOURNAL OF PLANT BIOLOGY, SPRINGER, BERLIN, DE, vol. 225, no. 3, 26 August 2006 (2006-08-26), pages 575-588, XP019491705, ISSN: 1432-2048<br>* abstract; figures 1, 8, 9 *<br>----- | 1-15 | |
| | | | TECHNICAL FIELDS SEARCHED (IPC) |
| X | WO 2006/135151 A1 (SEOUL NAT UNIV IND FOUNDATION [KR]; KIM MIN-KYUN [KR]; JUNG JIN-WOOK [) 21 December 2006 (2006-12-21)<br>* claims 3,7,8; examples 8, 9; sequences 3, 4,23 *<br>----- | 1-15 | C12N |
| X | ZHAO-SHI XU ET AL: "Functions and Application of the AP2/ERF Transcription Factor Family in Crop ImprovementF", JOURNAL OF INTEGRATIVE PLANT BIOLOGY, vol. 53, no. 7, 7 July 2011 (2011-07-07), pages 570-585, XP55120951, ISSN: 1672-9072, DOI: 10.1111/j.1744-7909.2011.01062.x<br>* abstract; table 3 *<br>-----<br>-/-- | 1-15 | |

1

The present search report has been drawn up for all claims

| Place of search | Date of completion of the search | Examiner |
|---|---|---|
| Munich | 2 June 2014 | Krüger, Julia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 15 1610

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProt [Online]<br><br>29 May 2013 (2013-05-29), "SubName: Full=Ethylene-responsive transcription factor 1; SubName: Full=Uncharacterized protein;", XP002725186, retrieved from EBI accession no. UNIPROT:M7YLX0<br>Database accession no. M7YLX0<br>* sequence *<br>& LING HONG-QING ET AL: "Draft genome of the wheat A-genome progenitor Triticum urartu", NATURE (LONDON), vol. 496, no. 7443, April 2013 (2013-04), pages 87-90, ISSN: 0028-0836(print)<br>----- | 7-10 | |
| X | WO 2009/014665 A2 (MONSANTO TECHNOLOGY LLC [US]; ABAD MARK [US]; ALIBHAI MURTAZA [US]; AU) 29 January 2009 (2009-01-29)<br>* claim 2; example 6; sequence 225 *<br>----- | 1-15 | TECHNICAL FIELDS SEARCHED (IPC) |

The present search report has been drawn up for all claims

1

| Place of search | Date of completion of the search | Examiner |
|---|---|---|
| Munich | 2 June 2014 | Krüger, Julia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 14 15 1610

This annex lists the patent family members relating to the patent documents cited in the above-mentioned European search report. The members are as contained in the European Patent Office EDP file on
The European Patent Office is in no way liable for these particulars which are merely given for the purpose of information.

02-06-2014

| Patent document cited in search report | Publication date | Patent family member(s) | Publication date |
|---|---|---|---|
| WO 2006135151 A1 | 21-12-2006 | KR 20060132442 A | 21-12-2006 |
| | | US 2010186112 A1 | 22-07-2010 |
| | | WO 2006135151 A1 | 21-12-2006 |
| WO 2009014665 A2 | 29-01-2009 | US 2009044288 A1 | 12-02-2009 |
| | | WO 2009014665 A2 | 29-01-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5811238 A
- US 6395547 A
- WO 2004070039 A
- WO 2004065596 A
- US 4962028 A
- WO 0114572 A
- WO 9514098 A
- WO 9412015 A
- US 5401836 A
- US 20050044585 A
- EP 99106056 A
- US 5565350 A
- WO 0015815 A
- WO 9322443 A, Zarling
- WO 9853083 A, Grierson
- WO 9953050 A, Waterhouse
- US 4987071 A, Cech
- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- EP 1198985 A1
- WO 2007093444 A
- WO 2011023537 A
- WO 2011023539 A
- EP 11172672 A
- EP 11172825 A
- EP 11181420 A
- US 5164310 A
- US 5159135 A
- WO 9623891 A
- WO 2010151634 A
- EP 1831378 A
- WO 2010031780 A
- WO 2006029987 A


### Non-patent literature cited in the description


- **WANG et al.** *Planta,* 2003, vol. 218, 1-14
- Creighton (1984) Proteins. 1984
- Current Protocols in Molecular Biology. John Wiley & Sons, 1989
- **TERPE.** *Appl. Microbiol. Biotechnol.,* 2003, vol. 60, 523-533
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* 2003, vol. 31, 315-318
- A generalized profile syntax for biomolecular sequences motifs and its function in automatic sequence interpretation. **BUCHER ; BAIROCH.** In) ISMB-94; Proceedings 2nd International Conference on Intelligent Systems for Molecular Biology. AAAI Press, 1994, 53-61
- **HULO et al.** *Nucl. Acids. Res.,* 2004, vol. 32, D134-D137
- **BATEMAN et al.** *Nucleic Acids Research,* 2002, vol. 30 (1), 276-280
- **R.D. FINN ; J. MISTRY ; J. TATE ; P. COGGILL ; A. HEGER ; J.E. POLLINGTON ; O.L. GAVIN ; P. GUNESEKARAN ; G. CERIC ; K. FORSLUND.** *Nucleic Acids Research,* 2010, vol. 38, 211-222
- **GASTEIGER et al.** ExPASy: the proteomics server for in-depth protein knowledge and analysis. *Nucleic Acids Res.,* 2003, vol. 31, 3784-3788
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** *BMC Bioinformatics,* 10 July 2003, vol. 4, 29
- **SMITH TF ; WATERMAN MS.** *J. Mol. Biol,* 1981, vol. 147 (1), 195-7
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- **CASTLE et al.** *Science,* 2004, vol. 304 (5674), 1151-4
- **HEID et al.** *Genome Methods,* 1996, vol. 6, 986-994

- **MANIATIS T ; FRITSCH EF ; SAMBROOK J.** Molecular Cloning: A Laboratory Manual. Cold Spring Harbor Laboratory, 1989
- **SILHAVY et al.** Experiments with Gene Fusions. Cold Spring Harbor Laboratory, 1984
- **AUSUBEL et al.** Current Protocols in Molecular Biology. Greene Publishing Assoc. and Wiley Interscience, 1987
- Plant Molecular Biology Manual. Kluwer Academic Publisher, 1990
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- *Plant Mol Biol.,* January 1995, vol. 27 (2), 237-48
- **KOYAMA et al.** *J Biosci Bioeng.,* January 2005, vol. 99 (1), 38-42
- **MUDGE et al.** *Plant J.,* 2002, vol. 31, 341
- **XIAO et al.** *Plant Biol (Stuttg).,* July 2006, vol. 8 (4), 439-49
- **NITZ et al.** *Plant Sci,* 2001, vol. 161 (2), 337-346
- **TINGEY et al.** *EMBO J.,* 1987, vol. 6, 1
- **VAN DER ZAAL et al.** *Plant Mol. Biol.,* 1991, vol. 16, 983
- **OPPENHEIMER et al.** *Gene,* 1988, vol. 63, 87
- **CONKLING et al.** *Plant Physiol.,* 1990, vol. 93, 1203
- **SUZUKI et al.** *Plant Mol. Biol.,* 1993, vol. 21, 109-119
- **BAUMBERGER et al.** *Genes & Dev.,* 2001, vol. 15, 1128
- **LAUTER et al.** *PNAS,* 1996, vol. 3, 8139
- **LIU et al.** *Plant Mol. Biol.,* vol. 17 (6), 1139-1154
- **DOWNEY et al.** *J. Biol. Chem.,* 2000, vol. 275, 39420
- **W SONG.** *PhD Thesis, North Carolina State University, Raleigh, NC USA,* 1997
- **WANG et al.** *Plant Sci.,* 2002, vol. 163, 273
- **DIENER et al.** *Plant Cell,* 2001, vol. 13, 1625
- **QUESADA et al.** *Plant Mol. Biol.,* 1997, vol. 34, 265
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **WU et al.** *Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876

- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* 1986, vol. 208, 15-22
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **FUKAVAMA et al.** *Plant Physiol.,* November 2001, vol. 127, 1136-46
- **KAUSCH et al.** *Plant Mol Biol.,* January 2001, vol. 45 (1), 1-15
- **LIN et al.** *DNA Seq.,* August 2004, vol. 15 (4), 269-76
- **NOMURA et al.** *Plant Mol Biol.,* September 2000, vol. 44 (1), 99-106
- **PANGULURI et al.** *Indian J Exp Biol.,* April 2005, vol. 43 (4), 369-72
- **WAGNER ; KOHORN.** *Plant Cell,* 2001, vol. 13 (2), 303-318
- **TRIBBLE et al.** *J. Biol. Chem.,* 2000, vol. 275, 22255-22267
- **VELMURUGAN et al.** *J. Cell Biol.,* 2000, vol. 149, 553-566
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- **B. JENES et al.** Techniques for Gene Transfer, in: Transgenic Plants, Vol. 1, Engineering and Utilization. Academic Press, 1993, vol. 1, 128-143
- *Potrykus Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- **F.F. WHITE.** Vectors for Gene Transfer in Higher Plants; in Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 1-9

- **FELDMANN K.** Methods in Arabidopsis Research. Word Scientific, Singapore, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** Methods in Arabidopsis Research. Singapore, World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2000, vol. 20 (10), 1030-4
- **LIDA ; TERADA.** *Curr Opin Biotech,* 2004, vol. 15 (2), 132-8
- **MILLER et al.** *Nature Biotechnol.,* 2007, vol. 25, 778-785
- **RABBANI et al.** *Plant Physiol,* 2003, vol. 133, 1755-1767
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* 1989, vol. 17, 6795-6807
- **BAILEY ; ELKAN.** Proceedings of the Second International Conference on Intelligent Systems for Molecular Biology. AAAI Press, 1994, 28-36
- Current Protocols in Molecular Biology
- **SAMBROOK.** Molecular Cloning: a laboratory manual. Cold Spring Harbor Laboratory Press, 2001
- Current Protocols in Molecular Biology. Current Protocols, 1994, vol. 1, 2
- **R.D.D. CROY.** Plant Molecular Biology Labfax. BIOS Scientific Publications Ltd (UK) and Blackwell Scientific Publications (UK, 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **THOMPSON et al.** *Nucleic Acids Res,* 1997, vol. 25, 4876-4882
- **CHENNA et al.** *Nucleic Acids Res,* 2003, vol. 31, 3497-3500
- **CAMPANELLA JJ ; BITINCKA L ; SMALLEY J.** MatGAT: an application that generates similarity/identity matrices using protein or DNA sequences. *BMC Bioinformatics,* 2003, vol. 4, 29
- **PARK ; KANEHISA.** *Bioinformatics,* 2003, vol. 19, 1656-1663
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *1978 Am J Bot,* 1978, vol. 65, 654-659
- **GAMBORG et al.** *Exp. Cell Res.,* 1968, vol. 50, 151-158
- **MURASHIGE, T. ; SKOOG.** *Physiol. Plant,* 1962, vol. 15, 473-497
- **GAMBORG et al.** *Exp. Cell Res.,* vol. 50, 151-8
- **HUSSEY, G. ; HEPHER, A.** *Annals of Botany,* 1978, vol. 42, 477-9
- **LINSEY, K. ; GALLOIS, P.** *Journal of Experimental Botany,* 1990, vol. 41 (226), 529-36
- **ARENCIBIA et al.** *Transgenic Research,* 1998, vol. 7, 213-22
- **ENRIQUEZ-OBREGON et al.** *Planta,* 1998, vol. 206, 20-27
- **MURASHIGE, T. ; SKOOG, .** *Physiol. Plant,* 1962, vol. 15, 473-497

- **GAMBORG, O. et al.** *Exp. Cell Res.,* 1968, vol. 50, 151-8